(19) Europäisches Patentamt
European Patent Office
Office européen des brevets

(11) EP 0 716 076 A2

(12) **EUROPÄISCHE PATENTANMELDUNG**

(43) Veröffentlichungstag:
**12.06.1996 Patentblatt 1996/24**

(21) Anmeldenummer: **95810737.7**

(22) Anmeldetag: **27.11.1995**

(51) Int Cl.6: **C07C 229/14**, C07C 229/12, C07D 295/15, C07C 229/16, C09K 15/00, C08K 5/16, C07D 211/58, C07D 211/76, C07C 239/20, C07D 211/46, C07C 69/708

(84) Benannte Vertragsstaaten:
**BE DE ES FR GB IT NL**

(30) Priorität: **05.12.1994 CH 3684/94**

(71) Anmelder: **CIBA-GEIGY AG**
**CH-4002 Basel (CH)**

(72) Erfinder:
- **Gilg, Bernard, Dr.**
  **F-68300 St. Louis-la-Chaussée (FR)**
- **Pitteloud, Rita, Dr.**
  **CH-1724 Praroman (CH)**

(54) **Bisphenolesterderivate**

(57) Stabilisatoren der Formel I,

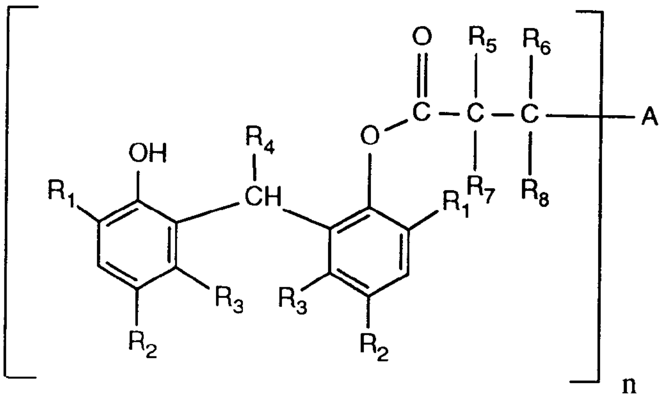

(I)

worin $R_1$ und $R_2$ z.B. $C_1$-$C_5$-Alkyl sind, die Reste $R_3$ bis $R_8$ z.B. Wasserstoff bedeuten, die Variable n z.B. 1 oder 2 ist und, wenn n 1 ist, A z.B. einen Alkoxyrest darstellt, und wenn n 2 ist, A z.B. ein Diaminrest bedeutet.

EP 0 716 076 A2

## Beschreibung

Die vorliegende Erfindung betrifft neue Bisphenolesterderivate, ihre Verwendung und das mit ihrer Hilfe gegen oxidativen, thermischen und lichtinduzierten Abbau stabilisierte organische Material.

Die Verwendung von einigen Bisphenolesterderivaten als Stabilisatoren wird zum Beispiel in JP-A-Hei 4-308 581, DE-A-3 718 751, EP-A-479 560 und US-A-4 414 408 beschrieben.

Ein Gegenstand der vorliegenden Erfindung sind Verbindungen der Formel I,

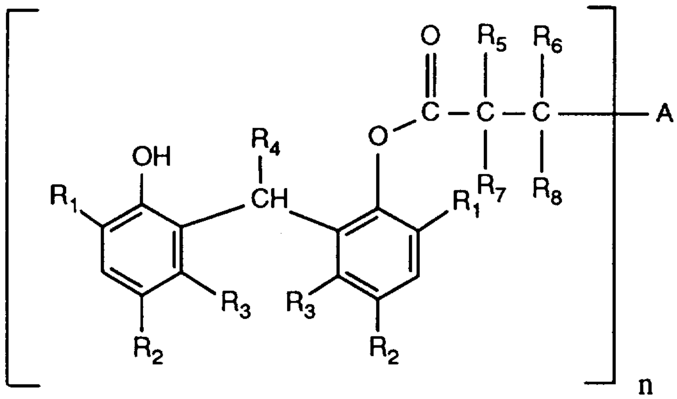

(I)

worin

die Reste $R_1$ unabhängig voneinander $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl oder $-CH_2$-S-$X_1$ bedeuten,

die Reste $R_2$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl, $-CH_2$-S-$X_1$, $-(CH_2)_p$COO-$X_2$ oder $-(CH_2)_q$O-$X_3$ sind,

die Reste $R_3$ unabhängig voneinander Wasserstoff oder $C_1$-$C_4$-Alkyl darstellen,

$R_4$ Wasserstoff oder $C_1$-$C_8$-Alkyl ist,

$R_5$ Wasserstoff, $C_1$-$C_{10}$-Alkyl, Phenyl, $-CH_2$-COO-$X_4$ oder CN bedeutet,

$R_6$ Wasserstoff, $C_1$-$C_4$-Alkyl, Phenyl, -COO-$X_5$, -CN oder -CON($X_6$)($X_7$) ist,

$R_7$ Wasserstoff oder $C_1$-$C_{10}$-Alkyl bedeutet,

$R_8$ Wasserstoff, $C_1$-$C_4$-Alkyl oder Phenyl ist,

$X_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder $-(CH_2)_r$COO-$Y_1$ darstellt,

$X_2$, $X_4$ und $X_5$ unabhängig voneinander $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl bedeuten,

$X_3$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$X_6$ und $X_7$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl oder $C_7$-$C_9$-Phenylalkyl sind,

$Y_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

$Y_2$ Wasserstoff oder $C_1$-$C_8$-Alkyl bedeutet,

p 0, 1 oder 2 ist,

q eine ganze Zahl von 0 bis 8 darstellt,

r 1 oder 2 bedeutet,

n eine ganze Zahl von 1 bis 4 ist und,

wenn n 1 ist, A eine Gruppe -O-$Z_1$, -N($Z_2$)($Z_3$), -NH(O$Z_4$), -O-N=C($Z_5$)($Z_6$), -S(O)$_m$$Z_7$, -NH-$Z_8$ oder -S-$Z_8$ bedeutet, oder A ferner einen unsubstituierten oder durch $C_1$-$C_4$-Alkyl substituierten heterocyclischen Rest darstellt, der die freie Valenz an einem Stickstoffatom besitzt,

$Z_1$ Wasserstoff, $C_1$-$C_{25}$-Alkyl, durch Sauerstoff, Schwefel oder >N-$Y_2$ unterbrochenes $C_3$-$C_{25}$-Alkyl, $C_3$-$C_{24}$-Alkenyl, einen monocyclischen, gesättigten Kohlenwasserstoffrest mit 5 bis 20 Kohlenstoffatomen, einen bicyclischen, gesättigten Kohlenwasserstoffrest mit 7 bis 20 Kohlenstoffatomen, einen tricyclischen, gesättigten Kohlenwasserstoffrest mit 10 bis 20 Kohlenstoffatomen, $C_5$-$C_{12}$-Cycloalkenyl, durch $C_1$-$C_4$-Alkyl substituiertes $C_5$-$C_{12}$-Cycloalkenyl, $C_7$-$C_9$-Phenylalkyl, am Phenylring durch $C_1$-$C_4$-Alkyl substituiertes $C_7$-$C_9$-Phenylalkyl, Tetrahydrofurfuryl, Tetrahydroabietyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl, Furoyl oder eine Gruppe der Formel IIa oder IIb bedeutet,

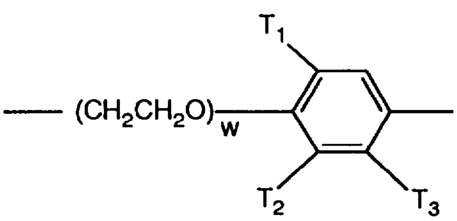 (IIa), 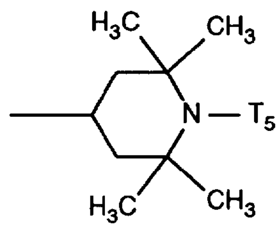 (IIb)

$Z_2$ Wasserstoff, $C_1$-$C_{25}$-Alkyl, durch OH substituiertes $C_2$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl, Furoyl, -$(CH_2)_p$COO-$X_2$ oder einen Rest der Formel IIb bedeutet,

$Z_3$ Wasserstoff, $C_1$-$C_{25}$-Alkyl, durch OH substituiertes $C_2$-$C_{25}$-Alkyl, $C_3$-$C_{24}$-Alkenyl,

$C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb ist oder

$Z_2$ und $Z_3$ zusammen $C_3$-$C_6$-Alkylen, $C_3$-$C_6$-Oxoalkylen oder durch Sauerstoff, Schwefel oder >N-$T_6$ unterbrochenes $C_3$-$C_6$-Alkylen bilden,

$Z_4$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

$Z_5$ und $Z_6$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl oder $C_7$-$C_9$-Phenylalkyl bedeuten oder

die Reste $Z_5$ und $Z_6$ zusammen mit dem Kohlenstoffatom, an das sie gebunden sind, einen unsubstituierten oder durch $C_1$-$C_4$-Alkyl substituierten $C_5$-$C_{12}$-Cycloalkylidenring bilden,

$Z_7$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$ und $T_2$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl oder $-CH_2-S-X_1$ bedeuten,

$T_3$ Wasserstoff oder $C_1$-$C_4$-Alkyl ist,

$T_4$ Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl, $-CH_2-S-X_1$, $-(CH_2)_pCOO-X_2$ oder $-(CH_2)_qO-X_3$ ist,

$T_5$ Wasserstoff, $C_1$-$C_8$-Alkyl, durch -OH substituiertes $C_2$-$C_4$-Alkyl, $O^{\cdot}$, -OH, -NO, $-CH_2CN$, $C_1$-$C_{18}$-Alkyloxy, $C_5$-$C_{12}$-Cycloalkyloxy, $C_3$-$C_6$-Alkenyl, $C_7$-$C_9$-Phenylalkyl, am Phenylring durch $C_1$-$C_4$-Alkyl substituiertes $C_7$-$C_9$-Phenylalkyl, $C_1$-$C_8$-Alkanoyl, $C_3$-$C_8$-Alkenoyl oder Benzoyl bedeutet,

$T_6$ $C_1$-$C_{12}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

m 1 oder 2 bedeutet,

w 0 oder 1 bedeutet,

wenn n 2 ist, A eine Gruppe der Formel IIIa, IIIb, IIIc, IIId, IIIe oder IIIf ist,

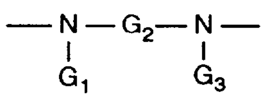 (IIIa), 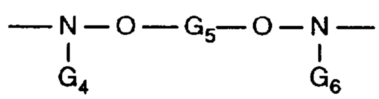 (IIIb),

$-O-G_7-O-$ (IIIc), 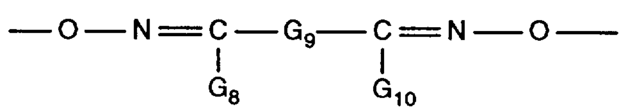 (IIId),

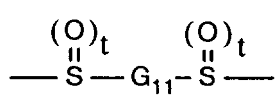 (IIIe), $>N-G_{12}$ (IIIf)

$G_1$ und $G_3$ unabhängig voneinander Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $-(CH_2)_pCOO-X_2$ oder einen Rest der Formel IIb bedeuten,

$G_2$ $C_2$-$C_{12}$-Alkylen, durch Sauerstoff, Schwefel oder $>N-Y_2$ unterbrochenes $C_4$-$C_{20}$-Alkylen, $C_4$-$C_{20}$-Alkenylen, $C_4$-$C_{20}$-Alkinylen,
($C_1$ -$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen, Naphthylen, $C_2$-$C_{20}$-Alkandioyl, $C_4$-$C_{20}$-Alkendioyl oder Carboxybenzoyl bedeutet,

$G_4$ und $G_6$ unabhängig voneinander Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_5$ $C_2$-$C_{12}$-Alkylen, durch Sauerstoff, Schwefel oder $>N-Y_2$ unterbrochenes $C_4$-$C_{20}$-Alkylen, $C_4$-$C_{20}$-Alkenylen, $C_4$-

$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen oder Naphthylen bedeutet,

$G_7$ $C_2$-$C_{20}$-Alkylen, durch Sauerstoff, Schwefel oder >N-$Y_2$ unterbrochenes $C_4$-$C_{20}$-Alkylen, $C_4$-$C_{20}$-Alkenylen, $C_4$-$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen, Naphthylen, $C_2$-$C_{20}$-Alkandioyl, $C_4$-$C_{20}$-Alkendioyl, Carboxybenzoyl oder eine Gruppe der Formel IVa, IVb oder IVc

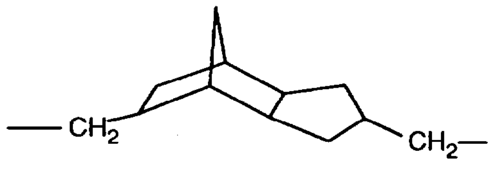

(IVa)

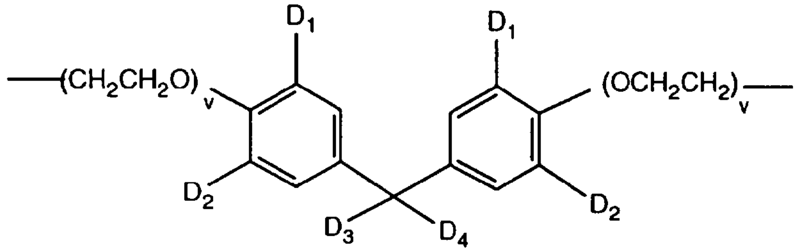

(IVb)

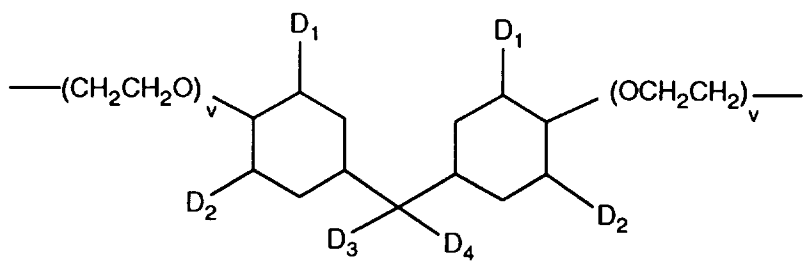

(IVc)

darstellt,

$G_8$ und $G_{10}$ unabhängig voneinander Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_9$ und $G_{11}$ $C_2$-$C_{12}$-Alkylen, durch Sauerstoff, Schwefel oder >N-$Y_2$ unterbrochenes $C_4$-$C_{20}$-Alkylen, $C_4$-$C_{20}$-Alkenylen, $C_4$-$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen oder Naphthylen ist,

$G_{12}$ Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb ist,

die Reste $D_1$ unabhängig voneinander Wasserstoff, $C_1$-$C_8$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl bedeuten,

die Reste $D_2$ unabhängig voneinander Wasserstoff, $C_1$-$C_8$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl sind,

$D_3$ und $D_4$ unabhängig voneinander Wasserstoff, $CF_3$, $C_1$-$C_{12}$-Alkyl oder Phenyl bedeuten oder

die Reste $D_3$ und $D_4$ zusammen mit dem Kohlenstoffatom, an das sie gebunden sind, einen unsubstituierten oder durch $C_1$-$C_4$-Alkyl substituierten $C_5$-$C_{12}$-Cycloalkylidenring bilden,

t 1 oder 2 bedeutet,

v 0 oder 1 bedeutet,

wenn n 3 ist, A eine Gruppe der Formel Va, Vb oder Vc bedeutet,

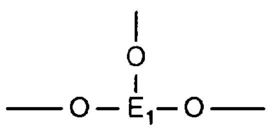 (Va), 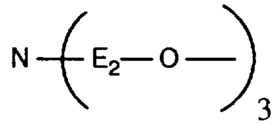 (Vb), 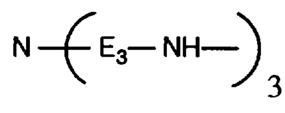 (Vc)

$E_1$ $C_3$-$C_7$-Alkantriyl darstellt,

$E_2$ und $E_3$ $C_2$-$C_8$-Alkylen sind,

wenn n 4 ist, A eine Gruppe der Formel VI bedeutet

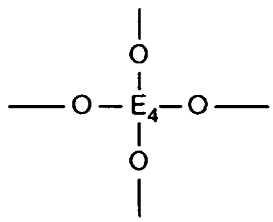 (VI)

und $E_4$ $C_4$-$C_{10}$-Alkantetrayl oder durch Sauerstoff unterbrochenes $C_4$-$C_{10}$-Alkantetrayl ist.

Alkyl mit bis zu 25 Kohlenstoffatomen, bevorzugt mit bis zu 18 Kohlenstoffatomen, insbesondere mit bis zu 10 Kohlenstoffatomen, bedeutet beispielsweise Methyl, Ethyl, Propyl, Isopropyl, n-Butyl, sec-Butyl, Isobutyl, tert-Butyl, 2-Ethylbutyl, n-Pentyl, tert-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl.

Eine der bevorzugten Bedeutungen von $R_1$ und $R_2$ ist verzweigtes $C_1$-$C_{10}$-Alkyl, insbesondere $C_1$-$C_5$-Alkyl, z.B. Methyl, tert-Butyl und tert-Pentyl.

Eine der bevorzugten Bedeutungen von $R_4$ ist $C_1$-$C_4$-Alkyl, insbesondere Methyl.

Eine der bevorzugten Bedeutungen von $Y_2$ is $C_1$-$C_4$-Alkyl, insbesondere Methyl.

Eine der bevorzugten Bedeutungen von $Z_1$ und $Z_2$ ist $C_1$-$C_{18}$-Alkyl.

Eine der bevorzugten Bedeutungen von $Z_7$ ist $C_1$-$C_{18}$-Alkyl, insbesondere $C_1$-$C_{10}$-Alkyl.

Eine der bevorzugten Bedeutungen von $T_1$, $T_2$ und $T_4$ ist $C_1$-$C_4$-Alkyl, insbesondere Methyl und tert-Butyl.

Eine der bevorzugten Bedeutungen von $T_5$ ist $C_1$-$C_4$-Alkyl, insbesondere Methyl.

Eine der bevorzugten Bedeutungen von $T_6$ ist $C_1$-$C_4$-Alkyl.

Beispiele für durch Sauerstoff, Schwefel oder >N-$Y_2$unterbrochenes $C_3$-$C_{25}$-Alkyl sind $CH_3$-O-$CH_2CH_2$-, $CH_3$-$CH_2$-O-$CH_2$-$CH_2$-$CH_2$-O-, $(CH_3)_2$CH-O-$CH_2$-$CH_2$-, $CH_3$-S-$CH_2CH_2$-, $CH_3$-NH-$CH_2CH_2$-, $CH_3$-N($CH_3$)-$CH_2CH_2$-, $CH_3$-O-$CH_2CH_2$-O-$CH_2CH_2$-, $CH_3$-(O-$CH_2CH_2$-$)_2$O-$CH_2CH_2$-, $CH_3$-(O-$CH_2CH_2$-$)_3$O-$CH_2CH_2$- und $CH_3$-(O-$CH_2CH_2$-$)_4$O-$CH_2CH_2$-. Durch Sauerstoff oder >N-$Y_2$ unterbrochenes $C_3$-$C_{25}$-Alkyl, insbesondere durch Sauerstoff unterbrochenes $C_3$-$C_{10}$-Alkyl, ist bevorzugt. Die Reste ($C_1$-$C_5$-Alkyl)-($OCH_2CH_2)_{1-10}$- und ($C_1$-$C_5$-Alkyl)-($OCH_2CH_2)_{1-2}$- sind besonders bevorzugt.

Durch -OH substituiertes $C_2$-$C_{25}$-Alkyl, insbesondere durch -OH substituiertes $C_2$-$C_4$-Alkyl, bedeutet zum Beispiel 2-Hydroxyethyl, 2-Hydroxypropyl, 2-Hydroxybutyl oder 4-Hydroxybutyl.

Beispiele für $C_1$-$C_{18}$-Alkyloxy sind Methoxy, Ethoxy, Propoxy, Isopropoxy, Butoxy, Isobutoxy, Pentoxy, Isopentoxy, Hexoxy, Heptoxy, Octoxy, Decyloxy, Dodecyloxy, Tetradecyloxy, Hexadecyloxy und Octadecyloxy. Bevorzugt ist $C_6$-

$C_{12}$-Alkyloxy,

insbesondere Heptoxy und Octoxy.

Alkenyl mit bis zu 24 Kohlenstoffatomen, insbesondere mit bis zu 18 Kohlenstoffatomen, bedeutet beispielsweise Vinyl, Propenyl, Isopropenyl, 2-Butenyl, 3-Butenyl, Isobutenyl, 3-Methyl-but-2-enyl, n-Oct-2-enyl, n-Dodec-2-enyl, iso-Dodecenyl, n-Octadec-2-enyl oder n-Octadec-4-enyl. Alkenylreste, in denen das Kohlenstoffatom in der Stellung 1 gesättigt ist, sind bevorzugt. $C_3$-$C_{18}$-Alkenyl ist besonders bevorzugt.

Unsubstituiertes oder durch $C_1$-$C_4$-Alkyl substituiertes $C_5$-$C_{12}$-Cycloalkyl bedeutet zum Beispiel Cyclopentyl, Cyclohexyl, Cycloheptyl, Cyclooctyl, Cyclododecyl, 2- oder 4-Methylcyclohexyl, Dimethylcyclohexyl, Trimethylcyclohexyl oder tert-Butylcyclohexyl. Unsubstituiertes oder durch $C_1$-$C_4$-Alkyl substituiertes $C_5$-$C_8$-Cycloalkyl, insbesondere Cyclohexyl, ist bevorzugt.

Eine der bevorzugten Bedeutungen von $R_1$ ist Cyclohexyl.

Beispiele für $C_5$-$C_{12}$-Cycloalkyloxy sind Cyclopentoxy, Cyclohexoxy, Cycloheptoxy, Cyclooctoxy, Cyclodecyloxy und Cyclododecyloxy. Cyclopentoxy und Cyclohexoxy sind bevorzugt.

Ein monocyclischer, gesättigter Kohlenwasserstoffrest mit 5 bis 20 Kohlenstoffatomen bedeutet zum Beispiel unsubstituiertes oder durch $C_1$-$C_4$-Alkyl substituiertes $C_5$-$C_{12}$-Cycloalkyl oder unsubstituiertes oder am Cycloalkylrest durch $C_1$-$C_4$-Alkyl substituiertes ($C_5$-$C_{12}$-Cycloalkyl)-($C_1$-$C_4$-alkyl) wie Cyclohexylmethyl, Methylcyclohexylmethyl oder Dimethylcyclohexylmethyl.

Ein bicyclischer, gesättigter Kohlenwasserstoffrest mit 7 bis 20 Kohlenstoffatomen bedeutet zum Beispiel

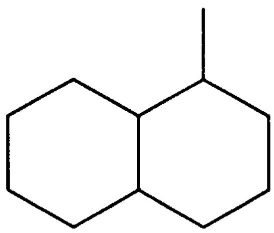 , 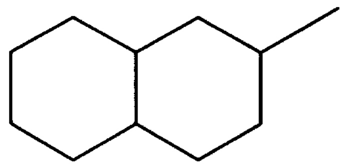

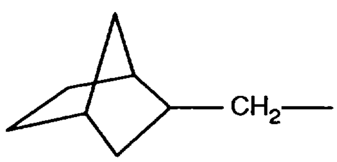 , 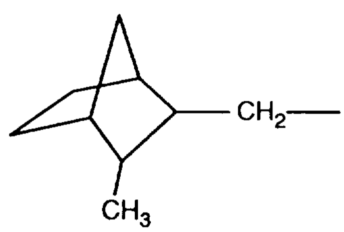 ,

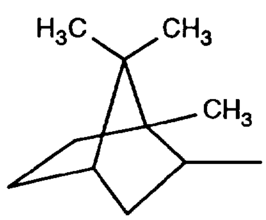 oder 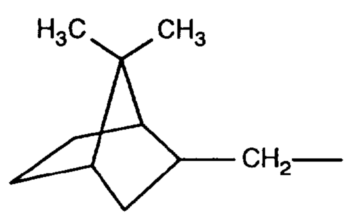 .

Ein tricyclischer, gesättigter Kohlenwasserstoffrest mit 10 bis 20 Kohlenstoffatomen bedeutet zum Beispiel

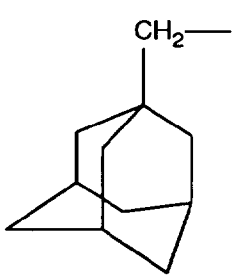 oder 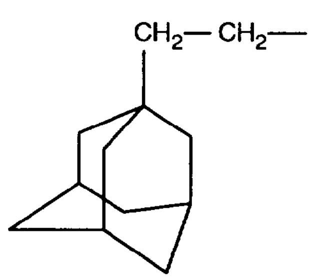 .

Beispiele für unsubstituiertes oder durch $C_1$-$C_4$-Alkyl substituiertes $C_5$-$C_{12}$-Cycloalkenyl, bevorzugt $C_5$-$C_8$-Cycloalkenyl, sind Cyclohex-2-enyl, Cyclohept-3-enyl und 4-tert-Butylcyclohex-2-enyl. Bevorzugt ist Cyclohexenyl.

Durch $C_1$-$C_4$-Alkyl substituiertes Phenyl ist beispielsweise Methylphenyl, Dimethylphenyl, Trimethylphenyl, Ethylphenyl, Diethylphenyl, Isopropylphenyl, tert-Butylphenyl, Di-tert-butylphenyl oder Methyl-di-t-butylphenyl.

Beispiele für einen unsubstituierten oder durch $C_1$-$C_4$-Alkyl substituierten $C_5$-$C_{12}$-Cycloalkylidenring sind

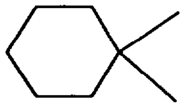 und 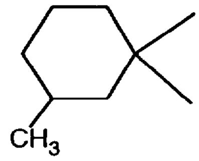 .

Ein $C_5$-$C_8$-Cycloalkylidenring ist bevorzugt.

Beispiele für $C_7$-$C_9$-Phenylalkyl, welches gegebenenfalls am Phenylring durch $C_1$-$C_4$-Alkyl substituiert ist, sind Benzyl, Phenethyl, 3-Phenylpropyl, α-Methylbenzyl, α,α-Dimethylbenzyl, Methylbenzyl, Dimethylbenzyl, Trimethylbenzyl und tert-Butylbenzyl.

Alkanoyl mit bis zu 25 Kohlenstoffatomen bedeutet beispielsweise Methanoyl, Ethanoyl, Propanoyl, Butanoyl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Octadecanoyl, Nonadecanoyl oder Eicosanoyl. $C_1$-$C_{18}$-Alkanoyl ist eine bevorzugte Bedeutung.

Beispiele für durch Sauerstoff, Schwefel oder >N-$Y_2$ unterbrochenes $C_3$-$C_{25}$-Alkanoyl sind -CO-$CH_2CH_2$-S-($C_1$-$C_{10}$-Alkyl), -CO-$CH_2CH_2$-O-($C_1$-$C_{10}$-Alkyl) und -CO-$CH_2CH_2$-N($Y_2$)-($C_1$-$C_{10}$-Alkyl). Durch Sauerstoff oder >N-$Y_2$ unterbrochenes $C_3$-$C_{25}$-Alkanoyl ist bevorzugt.

Beispiele für $C_3$-$C_{25}$-Alkenoyl sind Acryloyl, Methacryloyl, Crotonoyl, Isocrotonoyl und Oleolyl. $C_3$-$C_{18}$-Alkenoyl ist bevorzugt.

$C_6$-$C_9$-Cycloalkylcarbonyl bedeutet beispielsweise Cyclopentylcarbonyl, Cyclohexylcarbonyl, Cycloheptylcarbonyl oder Cyclooctylcarbonyl.

Durch $C_1$-$C_4$-Alkyl substituiertes Benzoyl bedeutet zum Beispiel Methylbenzoyl oder tert-Butylbenzoyl.

Beispiele für einen unsubstituierten oder durch $C_1$-$C_4$-Alkyl substituierten heterocyclischen Rest, der die freie Valenz an einem Stickstoffatom besitzt, sind:

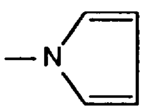 , 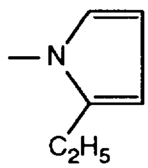 , 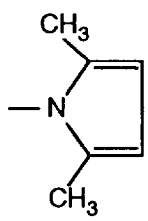 , 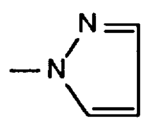 , 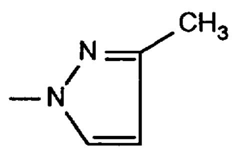 ,

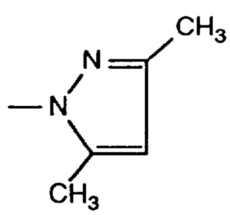 , 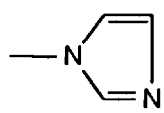 , 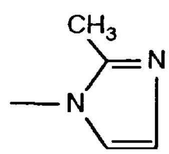 ,

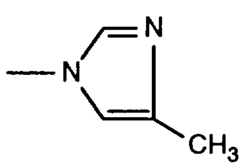 , 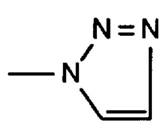 , 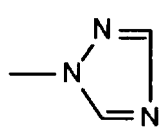 , 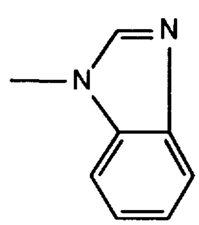 , 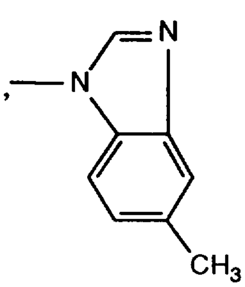 ,

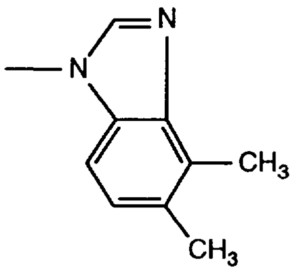 , 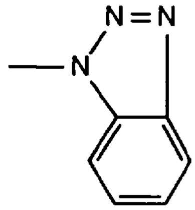 und 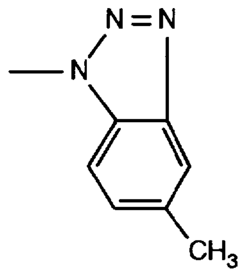 .

Bevorzugt ist ein 5-7 gliedriger heteromonocyclischer Rest und ein 9-10 gliedriger heterobicyclischer Rest. Als Heteroatom ist Stickstoff besonders bevorzugt. Bevorzugt ist auch unsubstituiertes oder durch $C_1$-$C_4$-Alkyl substituiertes 1-Pyrrolyl, unsubstituiertes oder durch $C_1$-$C_4$-Alkyl substituiertes 1-Pyrazolyl, unsubstituiertes oder durch $C_1$-$C_4$-Alkyl substituiertes 1-Imidazolyl, 1-Triazolyl, unsubstituiertes oder durch $C_1$-$C_4$-Alkyl substituiertes 1-Benzimidazolyl oder 1-Benzotriazolyl.

$Z_8$ bedeutet zum Beispiel eine Gruppe

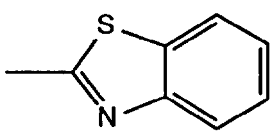 , 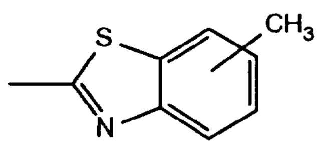 , 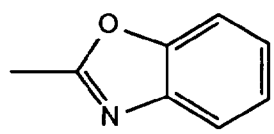

oder

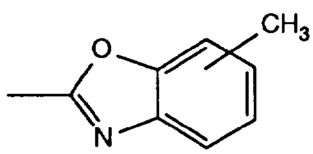 .

Alkylen mit bis zu 20 Kohlenstoffatomen, insbesondere mit bis zu 12 oder bis zu 6 Kohlenstoffatomen, bedeutet zum Beispiel Ethylen, Propylen, Trimethylen, Tetramethylen, Pentamethylen, 2,2-Dimethyltrimethylen, Hexamethylen, Trimethylhexamethylen, Octamethylen, Decamethylen, Undecamethylen oder Dodecamethylen.

Eine der bevorzugten Bedeutungen von $G_2$, $G_5$, $G_7$, $G_9$ und $G_{11}$ ist $C_2$-$C_8$-Alkylen.

Durch Sauerstoff, Schwefel oder >N-$Y_2$ unterbrochenes $C_4$-$C_{20}$-Alkylen bedeutet zum Beispiel $-CH_2CH_2-O-CH_2CH_2-$, $-CH_2CH_2-S-CH_2CH_2-$, $-CH_2CH_2-NH-CH_2CH_2-$, $-CH_2CH_2CH_2-NH-CH_2CH_2CH_2-$, $-CH_2CH_2CH_2-NH-CH_2CH_2-$, $-CH_2CH_2CH_2CH_2-NH-CH_2CH_2CH_2-$, $-(CH_2)_6-NH-(CH_2)_6-$, $-CH_2CH_2-N(CH_3)-CH_2CH_2-$, $-CH_2CH_2CH_2-N(CH_3)-CH_2CH_2CH_2-$, $-CH_2CH_2-(O-CH_2CH_2-)_2O-CH_2CH_2-$, $-CH_2CH_2-(O-CH_2CH_2-)_3O-CH_2CH_2-$, $-CH_2CH_2-(O-CH_2CH_2-)_4O-CH_2CH_2-$, $-CH_2CH_2CH_2CH_2-O-CH_2CH_2CH_2CH_2-O-CH_2CH_2CH_2CH_2-$, $-CH_2CH_2-NH-CH_2CH_2-NH-CH_2CH_2-$, $-CH_2CH_2CH_2-NH-CH_2CH_2-NH-CH_2CH_2CH_2-$ oder $-CH_2CH_2CH_2-NH-CH_2CH_2CH_2CH_2-NH-CH_2CH_2CH_2-$. Durch Sauerstoff oder >N-$Y_2$ unterbrochenes $C_4$-$C_{20}$-Alkylen ist bevorzugt. Die Reste $-CH_2CH_2-O-CH_2CH_2-$ und $-CH_2CH_2CH_2CH_2-O-CH_2CH_2CH_2CH_2-O-CH_2CH_2CH_2CH_2-$ sind besonders bevorzugt.

Eine der bevorzugten Bedeutungen für den Rest $-N(Z_2)(Z_3)$ ist Morpholino.

Durch >N-$T_6$ unterbrochenes $C_3$-$C_6$-Alkylen bedeutet zum Beispiel $-CH_2CH_2-N(CH_3)-CH_2CH_2-$, $-CH_2CH_2CH_2-N(CH_3)-CH_2CH_2CH_2-$.

$C_3$-$C_6$-Oxoalkylen bedeutet zum Beispiel $-CO-CH_2CH_2CH_2CH_2-$.

$C_4$-$C_{20}$-Alkenylen bedeutet zum Beispiel 2-Butenylen-1,4, 3-Pentenylen-1,5 oder 2-Hexenylen-1,6.

$C_4$-$C_{20}$-Alkinylen bedeutet zum Beispiel 2-Butinylen ($-CH_2-C\equiv C-CH_2-$), 2-Pentinylen, 2-Hexinylen, 3-Hexinylen, 3-Heptinylen, 2-Decinylen, 4-Decinylen oder 8-Octadecinylen.

($C_1$-$C_4$-Alkylen)-phenylen-($C_1$-$C_4$-alkylen) bedeutet zum Beispiel eine Gruppe

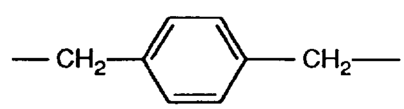 .

Beispiele für einen monocyclischen, gesättigten Kohlenwasserstoffrest mit zwei freien Valenzen und 5 bis 12 Kohlenstoffatomen sind eine Gruppe

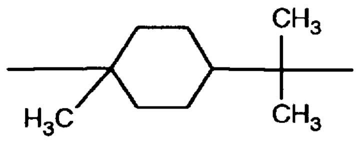

und $C_5$-$C_{12}$-Cycloalkylen wie Cyclopentylen, Cyclohexylen, Cycloheptylen oder Cyclooctylen.

Beispiele für einen bicyclischen, gesättigten Kohlenwasserstoffrest mit zwei freien Valenzen und 7 bis 30 Kohlenstoffatomen sind

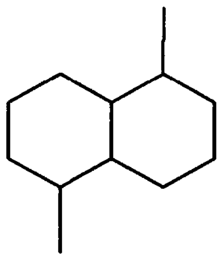 und 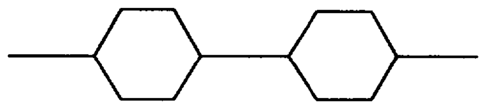 .

Durch $C_1$-$C_4$-Alkyl substituiertes Phenylen bedeutet zum Beispiel Methylphenylen oder tert-Butylphenylen.

$C_2$-$C_{20}$-Alkandioyl bedeutet zum Beispiel Ethandioyl, Propandioyl, Butandioyl, Pentandioyl, Hexandioyl, Heptandioyl, Octandioyl, Nonandioyl oder Decandioyl. $C_2$-$C_{10}$-Alkandioyl ist bevorzugt.

$C_4$-$C_{20}$-Alkendioyl bedeutet zum Beispiel Maleoyl, Fumaroyl, Citraconoyl oder Mesaconoyl.

Beispiele für $C_3$-$C_7$-Alkantriyl sind

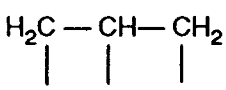 , 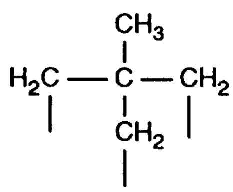 , 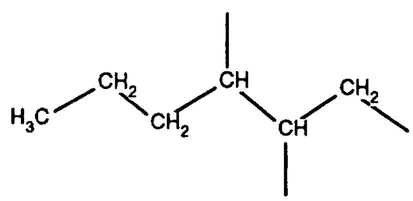 ,

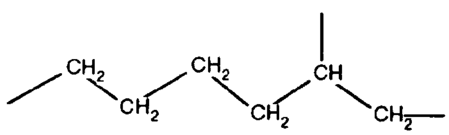 und 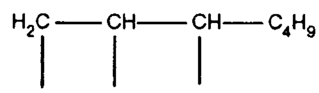 .

Beispiele für $C_4$-$C_{10}$-Alkantetrayl oder durch Sauerstoff unterbrochenes $C_4$-$C_{10}$-Alkantetrayl sind

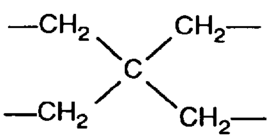 , 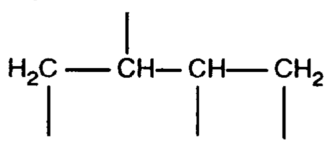 und 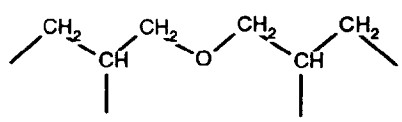 .

Die Reste $R_5$, $R_6$, $R_7$ und $R_8$ bedeuten bevorzugt Wasserstoff.

Die Variable n ist bevorzugt 1 oder 2.

Bevorzugt sind Verbindungen der Formel I, worin

die Reste $R_1$ unabhängig voneinander $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl oder $-CH_2$-S-$X_1$ bedeuten,

die Reste $R_2$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl, $-CH_2$-S-$X_1$, $-(CH_2)_pCOO$-$X_2$ oder $-(CH_2)_qO$-$X_3$ sind,

die Reste $R_3$ unabhängig voneinander Wasserstoff oder $C_1$-$C_4$-Alkyl darstellen,

$R_4$ Wasserstoff oder $C_1$-$C_8$-Alkyl ist,

$R_5$ Wasserstoff, $C_1$-$C_{10}$-Alkyl oder Phenyl bedeutet,

$R_6$ Wasserstoff, $C_1$-$C_4$-Alkyl oder Phenyl ist,

$R_7$ Wasserstoff oder $C_1$-$C_{10}$-Alkyl bedeutet,

$R_8$ Wasserstoff, $C_1$-$C_4$-Alkyl oder Phenyl ist,

$X_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$X_2$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl bedeutet,

$X_3$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $C_1$-$C_{18}$-Alkanoyl, $C_3$-$C_{18}$-Alkenoyl, durch Sauerstoff oder $>N-Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$Y_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist,

$Y_2$ Wasserstoff oder $C_1$-$C_8$-Alkyl bedeutet,

p 0, 1 oder 2 ist,

q eine ganze Zahl von 0 bis 8 darstellt,

r 1 oder 2 bedeutet,

n eine ganze Zahl von 1 bis 4 ist und,

wenn n 1 ist, A eine Gruppe $-O-Z_1$, $-N(Z_2)(Z_3)$, $-NH(OZ_4)$, $-O-N=C(Z_5)(Z_6)$, $-S(O)_mZ_7$, $-NH-Z_8$ oder $-S-Z_8$ bedeutet, oder A ferner ein unsubstituierter oder durch $C_1$-$C_4$-Alkyl substituierter heterocyclischer Rest ist, der die freie Valenz an einem Stickstoffatom besitzt,

$Z_1$ Wasserstoff, $C_1$-$C_{18}$-Alkyl, durch Sauerstoff oder $>N-Y_2$ unterbrochenes $C_3$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, $C_7$-$C_9$-Phenylalkyl, am Phenylring durch $C_1$-$C_4$-Alkyl substituiertes $C_7$-$C_9$-Phenylalkyl, Tetrahydrofurfuryl, Tetrahydroabietyl, $C_1$-$C_{18}$-Alkanoyl, $C_3$-$C_{18}$-Alkenoyl, durch Sauerstoff oder $>N-Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl, Furoyl oder eine Gruppe der Formel IIa oder IIb bedeutet,

$Z_2$ Wasserstoff, $C_1$-$C_{18}$-Alkyl, durch OH substituiertes $C_2$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $C_1$-$C_{18}$-Alkanoyl, $C_3$-$C_{18}$-Alkenoyl, durch Sauerstoff oder $>N-Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl, Furoyl, $-(CH_2)_pCOO-X_2$ oder einen Rest der Formel IIb bedeutet,

$Z_3$ Wasserstoff, $C_1$-$C_{18}$-Alkyl, durch OH substituiertes $C_2$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb ist oder

$Z_2$ und $Z_3$ zusammen $C_3$-$C_6$-Alkylen, $C_3$-$C_6$-Oxoalkylen oder durch Sauerstoff oder $>N-T_6$ unterbrochenes $C_3$-$C_6$-Alkylen bilden,

$Z_4$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist,

$Z_5$ und $Z_6$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl oder $C_7$-$C_9$-Phenylalkyl bedeuten oder

die Reste $Z_5$ und $Z_6$ zusammen mit dem Kohlenstoffatom, an das sie gebunden sind, einen unsubstituierten oder durch $C_1$-$C_4$-Alkyl substituierten $C_5$-$C_8$-Cycloalkylidenring bilden,

$Z_7$ $C_1$-$C_{18}$-Alkyl, $C_5$-$C_8$-Cycloalkyl, durch $C_1$-$C_4$-Alkyl substituiertes $C_5$-$C_8$-Cycloalkyl,

Phenyl, durch $C_1$-$C_4$-Alkyl substituiertes Phenyl, $C_7$-$C_9$-Phenylalkyl oder $-(CH_2)_rCOO-Y_1$ darstellt,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$ und $T_2$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl oder $-CH_2-S-X_1$ bedeuten,

$T_3$ Wasserstoff oder $C_1$-$C_4$-Alkyl ist,

$T_4$ Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl, $-CH_2-S-X_1$, $-(CH_2)_pCOO-X_2$ oder $-(CH_2)_qO-X_3$ ist,

$T_5$ Wasserstoff, $C_1$-$C_4$-Alkyl, -OH, $C_6$-$C_{12}$-Alkyloxy, $C_5$-$C_8$-Cycloalkyloxy, Allyl, Benzyl oder Acetyl bedeutet,

$T_6$ $C_1$-$C_{12}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist,

m 1 oder 2 bedeutet,

w 0 oder 1 bedeutet,

wenn n 2 ist, A eine Gruppe der Formel IIIa, IIIb, IIIc, IIId, IIIe oder IIIf ist,

$G_1$ und $G_3$ unabhängig voneinander Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $-(CH_2)_pCOO-X_2$ oder einen Rest der Formel IIb bedeuten,

$G_2$ $C_2$-$C_{12}$-Alkylen, durch Sauerstoff oder >N-$Y_2$ unterbrochenes $C_4$-$C_{12}$-Alkylen,

$C_4$-$C_{12}$-Alkenylen, $C_4$-$C_{12}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen, Naphthylen, $C_2$-$C_{18}$-Alkandioyl, $C_4$-$C_{18}$-Alkendioyl oder Carboxybenzoyl bedeutet,

$G_4$ und $G_6$ unabhängig voneinander Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_5$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen oder Naphthylen bedeutet,

$G_7$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen, Naphthylen, $C_2$-$C_{18}$-Alkandioyl, $C_4$-$C_{18}$-Alkendioyl oder Carboxybenzoyl ist,

$G_8$ und $G_{10}$ unabhängig voneinander Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_9$ und $G_{11}$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen oder Naphthylen ist,

$G_{12}$ Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb ist und

t 1 oder 2 bedeutet.

Ebenfalls bevorzugt sind Verbindungen der Formel I, worin

die Reste $R_1$ unabhängig voneinander $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl oder -$CH_2$-S-$X_1$ bedeuten,

die Reste $R_2$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl, -$CH_2$-S-$X_1$, -$(CH_2)_p$COO-$X_2$ oder -$(CH_2)_q$O-$X_3$ sind,

die Reste $R_3$ Wasserstoff darstellen,

$R_4$ Wasserstoff oder $C_1$-$C_4$-Alkyl ist,

$R_5$ Wasserstoff, $C_1$-$C_4$-Alkyl oder Phenyl bedeutet,

$R_6$ Wasserstoff, $C_1$-$C_4$-Alkyl oder Phenyl ist,

$R_7$ Wasserstoff oder $C_1$-$C_4$-Alkyl bedeutet,

$R_8$ Wasserstoff, $C_1$-$C_4$-Alkyl oder Phenyl ist,

$X_1$ $C_1$-$C_{10}$-Alkyl, $C_5$-$C_8$-Cycloalkyl, Phenyl, $C_7$-$C_9$-Phenylalkyl oder -$(CH_2)_r$COO-$Y_1$ darstellt,

$X_2$ $C_1$-$C_{10}$-Alkyl, $C_5$-$C_8$-Cycloalkyl, Phenyl oder $C_7$-$C_9$-Phenylalkyl bedeutet,

$X_3$ $C_1$-$C_{10}$-Alkyl, $C_5$-$C_8$-Cycloalkyl, Phenyl, $C_7$-$C_9$-Phenylalkyl, $C_1$-$C_{10}$-Alkanoyl, $C_3$-$C_{18}$-Alkenoyl, durch Sauerstoff unterbrochenes $C_3$-$C_{18}$-Alkanoyl oder Benzoyl ist,

$Y_1$ $C_1$-$C_{10}$-Alkyl, $C_5$-$C_8$-Cycloalkyl, Phenyl oder $C_7$-$C_9$-Phenylalkyl ist,

p 0, 1 oder 2 ist,

q eine ganze Zahl von 0 bis 8 darstellt,

r 1 oder 2 bedeutet,

n eine ganze Zahl von 1 bis 4 ist und,

wenn n 1 ist, A eine Gruppe -O-$Z_1$, -N($Z_2$)($Z_3$), -NH(O$Z_4$), -O-N=C($Z_5$)($Z_6$), -S(O)$_m$$Z_7$, -NH-$Z_8$ oder -S-$Z_8$ bedeutet, oder A ferner ein unsubstituierter oder durch $C_1$-$C_4$-Alkyl substituierter heterocyclischer Rest ist, der die freie Valenz an einem Stickstoffatom besitzt,

$Z_1$ Wasserstoff, $C_1$-$C_{18}$-Alkyl, durch Sauerstoff unterbrochenes $C_3$-$C_{18}$-Alkyl, $C_3$-$C_{18}$-Alkenyl, $C_5$-$C_8$-Cycloalkyl, $C_5$-$C_8$-Cycloalkenyl, $C_7$-$C_9$-Phenylalkyl, Tetrahydrofurfuryl, $C_1$-$C_{10}$-Alkanoyl, $C_3$-$C_{18}$-Alkenoyl, durch Sauerstoff

unterbrochenes $C_3$-$C_{18}$-Alkanoyl, Benzoyl oder eine Gruppe der Formel IIa oder IIb bedeutet,

$Z_2$ Wasserstoff, $C_1$-$C_{18}$-Alkyl, durch OH substituiertes $C_2$-$C_{10}$-Alkyl, $C_3$-$C_{18}$-Alkenyl, $C_5$-$C_8$-Cycloalkyl, Phenyl, $C_7$-$C_9$-Phenylalkyl, $C_1$-$C_{10}$-Alkanoyl, $C_3$-$C_{18}$-Alkenoyl, durch Sauerstoff unterbrochenes $C_3$-$C_{18}$-Alkanoyl, Benzoyl, -$(CH_2)_pCOO$-$X_2$ oder einen Rest der Formel IIb bedeutet,

$Z_3$ Wasserstoff, $C_1$-$C_{18}$-Alkyl, durch OH substituiertes $C_2$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb ist oder

$Z_2$ und $Z_3$ zusammen $C_3$-$C_6$-Alkylen, $C_3$-$C_6$-Oxoalkylen oder durch Sauerstoff unterbrochenes $C_3$-$C_6$-Alkylen bilden,

$Z_4$ $C_1$-$C_{10}$-Alkyl, $C_5$-$C_8$-Cycloalkyl, Phenyl oder $C_7$-$C_9$-Phenylalkyl ist,

$Z_5$ und $Z_6$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl oder $C_7$-$C_9$-Phenylalkyl bedeuten oder

die Reste $Z_5$ und $Z_6$ zusammen mit dem Kohlenstoffatom, an das sie gebunden sind, einen $C_5$-$C_8$-Cycloalkylidenring bilden,

$Z_7$ $C_1$-$C_{10}$-Alkyl, $C_5$-$C_8$-Cycloalkyl, Phenyl, $C_7$-$C_9$-Phenylalkyl oder -$(CH_2)_rCOO$-$Y_1$ darstellt,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$ und $T_2$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl oder -$CH_2$-S-$X_1$ bedeuten,

$T_3$ Wasserstoff oder $C_1$-$C_4$-Alkyl ist,

$T_4$ Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl, -$CH_2$-S-$X_1$, -$(CH_2)_pCOO$-$X_2$ oder -$(CH_2)_qO$-$X_3$ ist,

$T_5$ Wasserstoff, $C_1$-$C_4$-Alkyl, -OH, $C_6$-$C_{12}$-Alkyloxy, $C_5$-$C_8$-Cycloalkyloxy, Allyl, Benzyl oder Acetyl bedeutet,

m 1 oder 2 bedeutet,

w 0 oder 1 bedeutet,

wenn n 2 ist, A eine Gruppe der Formel IIIa, IIIb, IIIc, IIId, IIIe oder IIIf ist,

$G_1$ und $G_3$ unabhängig voneinander Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl, -$(CH_2)_pCOO$-$X_2$ oder einen Rest der Formel IIb bedeuten,

$G_2$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen, Phenylen, $C_2$-$C_{10}$-Alkandioyl, $C_4$-$C_{10}$-Alkendioyl oder Carboxybenzoyl bedeutet,

$G_4$ und $G_6$ unabhängig voneinander Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb sind,

$G_5$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen oder Phenylen bedeutet,

$G_7$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien

Valenzen und 5 bis 10 Kohlenstoffatomen, Phenylen, $C_2$-$C_{10}$-Alkandioyl, $C_4$-$C_{10}$-Alkendioyl oder Carboxybenzoyl ist,

$G_8$ und $G_{10}$ unabhängig voneinander Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb sind,

$G_9$ und $G_{11}$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen oder Phenylen ist,

$G_{12}$ Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb ist und

t 1 oder 2 bedeutet.

Bevorzugt sind auch Verbindungen der Formel I, worin

n 1 ist,

A eine Gruppe der Formel -N($Z_2$)($Z_3$) bedeutet,

$Z_2$ Wasserstoff ist und

$Z_3$ Wasserstoff, $C_1$-$C_{25}$-Alkyl, durch OH substituiertes $C_2$-$C_{25}$-Alkyl, $C_3$-$C_{24}$-Alkenyl,

$C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb ist.

Ferner bevorzugt sind Verbindungen der Formel I, worin

n 1 ist,

A eine Gruppe der Formel -O-$Z_1$ darstellt und

$Z_1$ durch Sauerstoff unterbrochenes $C_1$-$C_{25}$-Alkyl oder eine Gruppe der Formel IIa bedeutet und

w 1 ist.

Eine weitere bevorzugte Ausführungsform der Erfindung sind Verbindungen der Formel I, worin

die Reste $R_1$ gleich sind und $C_1$-$C_5$-Alkyl oder $C_5$-$C_8$-Cycloalkyl bedeuten,

die Reste $R_2$ gleich sind und $C_1$-$C_5$-Alkyl darstellen,

die Reste $R_3$ Wasserstoff bedeuten und

$R_4$ Wasserstoff oder $C_1$-$C_4$-Alkyl ist.

Besonders bevorzugt sind Verbindungen der Formel I, worin

die Reste $R_1$ gleich sind und $C_1$-$C_5$-Alkyl oder $C_5$-$C_8$-Cycloalkyl bedeuten,

die Reste $R_2$ gleich sind und $C_1$-$C_5$-Alkyl darstellen,

die Reste $R_3$ Wasserstoff bedeuten,

$R_4$ Wasserstoff oder $C_1$-$C_4$-Alkyl ist,

$R_5$, $R_6$, $R_7$ und $R_8$ Wasserstoff sind,

n 1 oder 2 ist und,

wenn n 1 ist, A eine Gruppe $-O-Z_1$, $-N(Z_2)(Z_3)$, $-NH(OZ_4)$, $-O-N=C(Z_5)(Z_6)$, $-S(O)_mZ_7$, $-NH-Z_8$ oder $-S-Z_8$ bedeutet, oder A ferner ein unsubstituierter oder durch $C_1$-$C_4$-Alkyl substituierter heterocyclischer Rest ist, der die freie Valenz an einem Stickstoffatom besitzt,

$Z_1$ $C_1$-$C_{18}$-Alkyl, durch Sauerstoff unterbrochenes $C_3$-$C_{10}$-Alkyl, $C_5$-$C_8$-Cycloalkyl, Tetrahydrofurfuryl oder eine Gruppe der Formel IIa oder IIb ist,

$Z_2$ $C_1$-$C_{18}$-Alkyl, durch -OH substituiertes $C_2$-$C_4$-Alkyl, $C_7$-$C_9$-Phenylalkyl, $-(CH_2)_pCOO-X_2$ oder einen Rest der Formel IIb darstellt,

$Z_3$ Wasserstoff, $C_1$-$C_{18}$-Alkyl, durch -OH substituiertes $C_2$-$C_4$-Alkyl, $C_7$-$C_9$-Phenylalkyl oder einen Rest der Formel IIb bedeutet oder

$Z_2$ und $Z_3$ zusammen $C_3$-$C_6$-Oxoalkylen oder durch Sauerstoff unterbrochenes $C_3$-$C_6$-Alkylen bilden,

$Z_4$ $C_7$-$C_9$-Phenylalkyl bedeutet,

die Reste $Z_5$ und $Z_6$ zusammen mit dem Kohlenstoffatom, an das sie gebunden sind, einen $C_5$-$C_8$-Cycloalkylidenring bilden,

$Z_7$ $C_1$-$C_{10}$-Alkyl bedeutet,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$, $T_2$, $T_3$ und $T_4$ unabhängig voneinander Wasserstoff oder $C_1$-$C_4$-Alkyl sind,

$T_5$ Wasserstoff oder $C_1$-$C_4$-Alkyl ist,

$X_2$ $C_1$-$C_{10}$-Alkyl darstellt,

m 1 oder 2 bdeutet,

p 1 ist,

w 0 oder 1 darstellt und

wenn n 2 ist, A eine Gruppe der Formel IIIa, IIIc oder IIIf bedeutet,

$G_1$ und $G_3$ unabhängig voneinander Wasserstoff, $C_7$-$C_9$-Phenylalkyl oder einen Rest der Formel IIb bedeuten,

$G_2$ $C_2$-$C_8$-Alkylen oder einen monocyclischen, gesättigten Kohlenwasserstoffrest mit zwei freien Valenzen und 10 Kohlenstoffatomen ist,

$G_7$ $C_2$-$C_8$-Alkylen oder durch Sauerstoff unterbrochenes $C_4$-$C_{12}$-Alkylen darstellt und

$G_{12}$ $C_7$-$C_9$-Phenylalkyl oder eine Gruppe der Formel IIb ist.

Die Herstellung von Verbindungen der Formel I kann beispielsweise nach folgenden, an sich bekannten Verfahren durchgeführt werden:

Verfahren A: Umsetzung einer Verbindung der Formel a

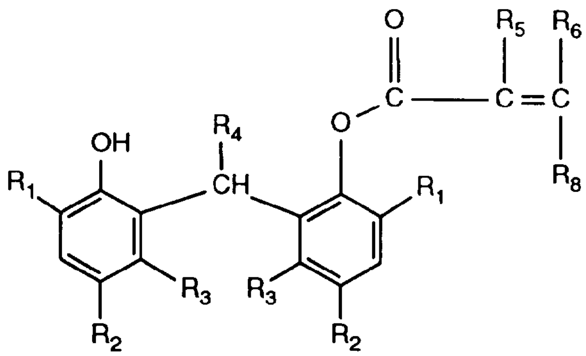

(a)

mit einer geeigneten Verbindung der Formel b (Michael Addition),

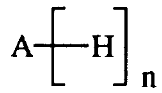

(b)

wobei die Reste $R_1$ bis $R_8$ und A sowie der Index n die für Formel I angegebenen Bedeutungen besitzen.

Die Reaktion kann durch Mischen der beiden Reaktionspartner mit oder ohne Lösungsmittel erfolgen. Mögliche Lösungsmittel sind übliche Kohlenwasserstoffe (wie z.B. Toluol, Hexan und Cyclohexan), halogenierte Kohlenwasserstoffe (wie z.B. Dichlormethan, Dichlorethan und Chlorbenzol), Ether (wie z.B Diethylether, Dibutylether,Tetrahydrofuran und Dimethoxyethan), Alkohole (wie z.B. Methanol und Ethanol) und ferner Acetonitril, Butylacetat und Dimethylformamid.

Die Umsetzung wird bevorzugt bei einer Temperatur von 5°C bis Siedepunkt der Reaktionsmischung durchgeführt

Bei der Addition eines Alkohols (oder eines Mercaptans) an die Verbindung der Formel a werden der Reaktionsmischung vorzugsweise katalytische Mengen (0,5-30 Mol%) einer Base zugesetzt. Geeignete Basen sind z.B. Alkalihydroxide, $C_1$-$C_5$-Alkalialkoxide, Amine (z.B. Triethylamin, N,N-Dimethylanilin, N-Methylanilin und Pyridin), Tetraalkylammoniumhydroxide oder Benzyltrimethylammoniumhydroxid oder auch organometallische Verbindungen (z.B. Bu-Li).

Verbindungen der Formel I, worin A eine Gruppe $-S(O)_mZ_7$ ist, können z.B. durch Umsetzung eines Bisphenolmonoacrylats der Formel a mit einem Mercaptan der Formel b (A = $SZ_7$) und anschliessender Oxidation des erhaltenen Thioethers nach üblichen Methoden (siehe z.B. Comprehensive Organic Chemistry, Vol 3, p. 124-126, 174, Ed. D. Neville Jones, Pergamon Press, 1979) erhalten werden.

Verfahren B: Veresterung oder Umesterung von organischen Carbonsäurederivaten der Formel c

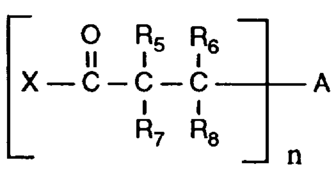

(c)

mit Bisphenolen der Formel d,

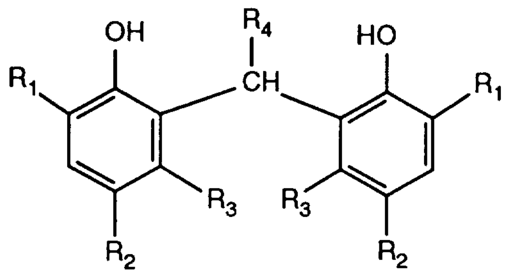

(d)

wobei die Reste $R_1$ bis $R_8$ und A sowie der Index n die für Formel I angegebenen Bedeutungen besitzen und X zum Beispiel OH, Cl,

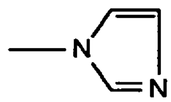

u.s.w. ist.

Die Reaktion kann in an sich bekannter Weise durchgeführt werden; zum Beispiel durch Zufügen eines der beiden Edukte zu dem zweiten Edukt und Durchmischen der beiden Reaktionspartner, bevorzugt unter Ausschluss von Sauerstoff. Die Umsetzung kann mit oder ohne Lösungsmittel (z.B. Toluol) durchgeführt werden. Die Temperatur kann z. B. zwischen dem Schmelzpunkt und dem Siedepunkt der Reaktionsmischung liegen, beispielsweise zwischen -50 und 150°C, vorzugsweise zwischen 0 und 150°C. Die Reinigung des erhaltenen Produktes kann ebenfalls nach bekannten Methoden erfolgen, beispielsweise durch Waschen mit Wasser/HCl, Extraktion mit einem organischen Lösungsmittel, Kristallisation und/oder Chromatographie. Als Lösungsmittel für die Extraktion sowie für den chromatographischen Reinigungsschritt werden Hexan, Essigester oder deren Gemische bevorzugt.

Wird in der Reaktion als Derivat der Carbonsäure der Formel c ein Säurechlorid (X = Cl) eingesetzt, so kann der Reaktionsmischung auch ein Säureakzeptor hinzugefügt werden. Geeignete Säureakzeptoren sind beispielsweise Amine wie Pyridin oder Triethylamin. Bevorzugt ist die Menge des Säureakzeptors mindestens äquivalent der Menge des Säurechlorids. Sie beträgt beispielsweise 1 bis 2 Äquivalente, insbesondere 1,2 bis 1,7 Äquivalente bezogen auf das Säurechlorid.

Das Säurechlorid kann auch "in situ" hergestellt werden. In diesem Fall werden die Carbonsäure der Formel c (X = OH), das Bisphenol der Formel d und ein Säureakzeptor (z.B. Triethylamin) vorgelegt und anschliessend Phosphoroxychlorid in Analogie zu dem beispielsweise in US-A-5,128,398 beschriebenen Verfahren zugegeben.

Werden die Carbonsäuren der Formel c und das Bisphenol der Formel d direkt als Edukte eingesetzt, wird die Umsetzung zweckmässigerweise unter Verwendung von Reagentien durchgeführt, die freigesetztes Wasser aufnehmen wie z.B. Dicyclohexylcarbodiimid.

<u>Verfahren C:</u> Umsetzung eines Chlorids der Formel e

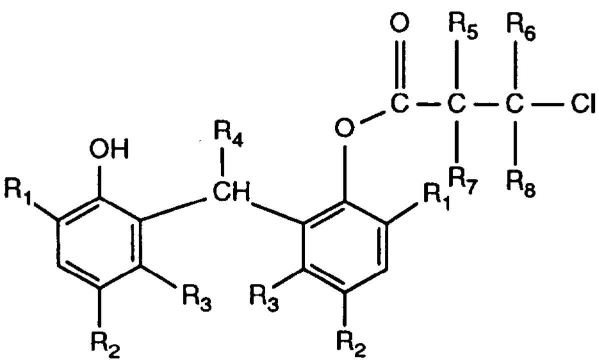

(e)

mit einer Verbindung der Formel b, vorzugsweise in Gegenwart einer Base als Säureakzeptor. Geeignete Säureakzeptoren sind Amine (wie z.B. Triethylamin und Pyridin), Hydride (wie z.B. Lithium-, Natrium- und Kaliumhydrid), Alkoholate (wie z.B. Natriummethanolat und Kalium-tert-butylat) und organometallische Basen (wie z.B. BuLi).

Werden Hydride, Alkalimetalle, Alkalihydroxide, Alkalialkoholate oder Alkyllithiumverbindungen als Basen verwendet, kann zuerst das entsprechende Anion der Verbindung der Formel b gebildet werden.

Beispiele für Verbindungen der Formel I sind in der folgenden Tabelle 1 aufgeführt.

Tabelle 1:

| Verbindung | Struktur | Elementaranalyse<br>Massenspektrum<br>Schmelzpunkt |
|---|---|---|
| 101 | 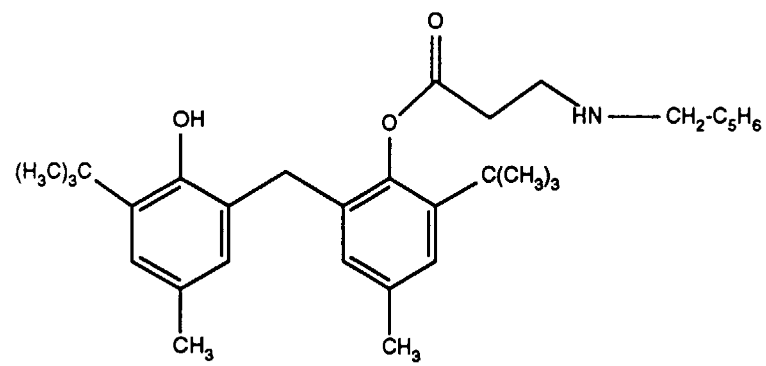 | **Elementaranalyse:**<br>C% H% N%<br>ber.: 79,00 8,64 2,79<br>gef.: 79,03 8,61 2,74<br>**Schmelzpunkt:** 124-126°C |
| 102 | 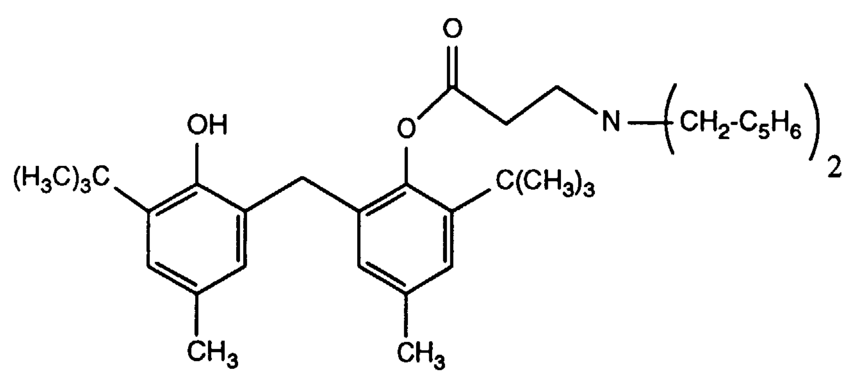 | **Elementaranalyse:**<br>C% H% N%<br>ber.: 81,18 8,35 2,37<br>gef.: 80,34 8,59 2,85<br>**Massenspektrum:** (CI): 592(MH⁺) |

Tabelle 1 (Fortsetzung):

| Verbindung | Struktur | Elementaranalyse<br>Massenspektrum<br>Schmelzpunkt |
|---|---|---|
| 103 | 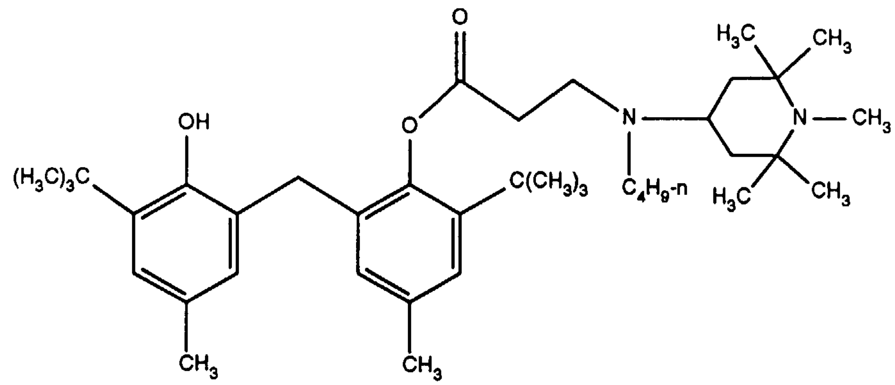 | Massenspektrum: (DEI): 620($M^+$)<br><br>Schmelzpunkt: 38-43°C |
| 104 | 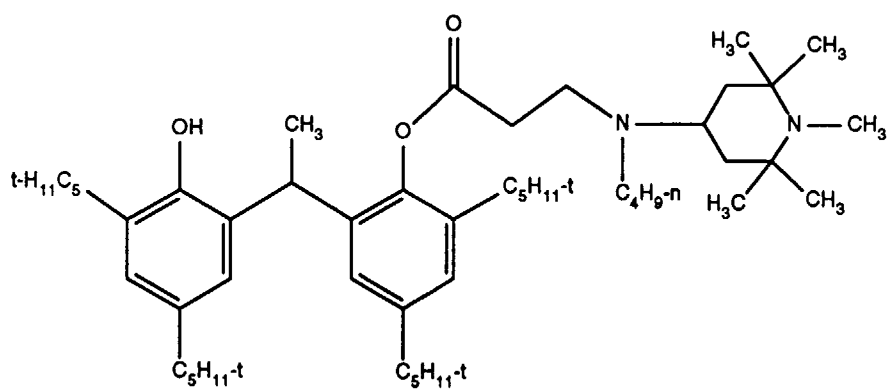 | Elementaranalyse:<br><br>C% H% N%<br>ber.: 79,01 11,18 3,61<br>gef.: 78,57 11,36 3,37 |

Tabelle 1 (Fortsetzung):

| Verbindung | Struktur | Elementaranalyse Massenspektrum Schmelzpunkt |
|---|---|---|
| 105 | 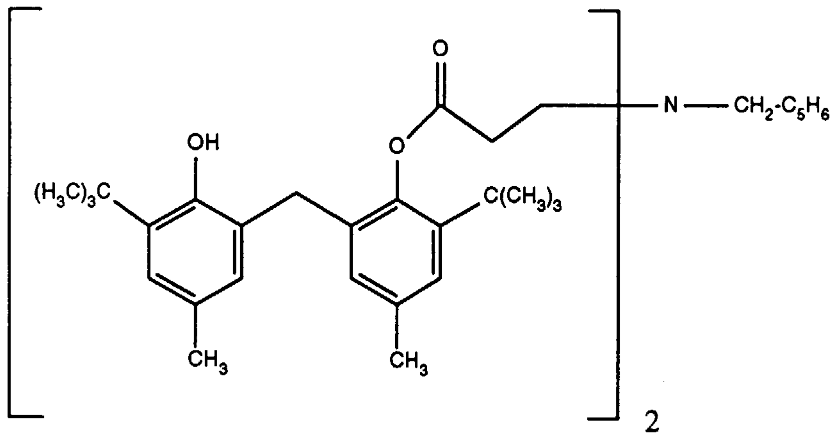 | **Elementaranalyse:**<br>C% H% N%<br>ber.: 79,07 8,66 1,56<br>gef.: 78,70 8,75 1,35<br>**Schmelzpunkt:** 70-76°C |
| 106 | 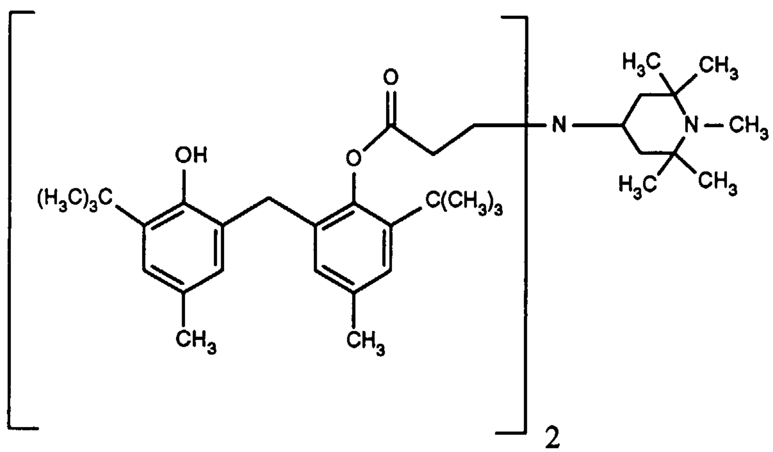 | **Elementaranalyse:**<br>C% H% N%<br>ber.: 77,62 9,46 2,92<br>gef.: 77,09 9,45 2,76<br>**Schmelzpunkt:** 89-94°C |

Tabelle 1 (Fortsetzung):

| Verbindung | Struktur | Elementaranalyse Massenspektrum Schmelzpunkt |
|---|---|---|
| 107 | 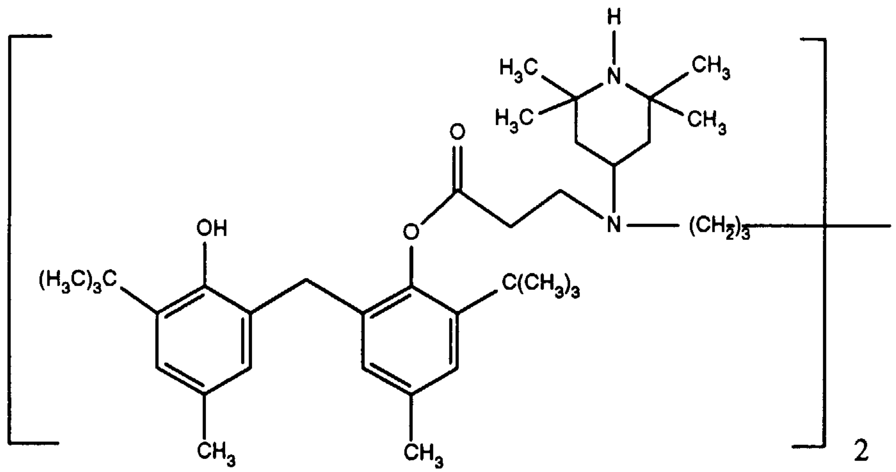 | Elementaranalyse:<br>C% H% N%<br>ber.: 77,11 10,05 4,73<br>gef.: 76,52 9,99 4,47<br>Schmelzpunkt: 83-86°C |
| 108 | 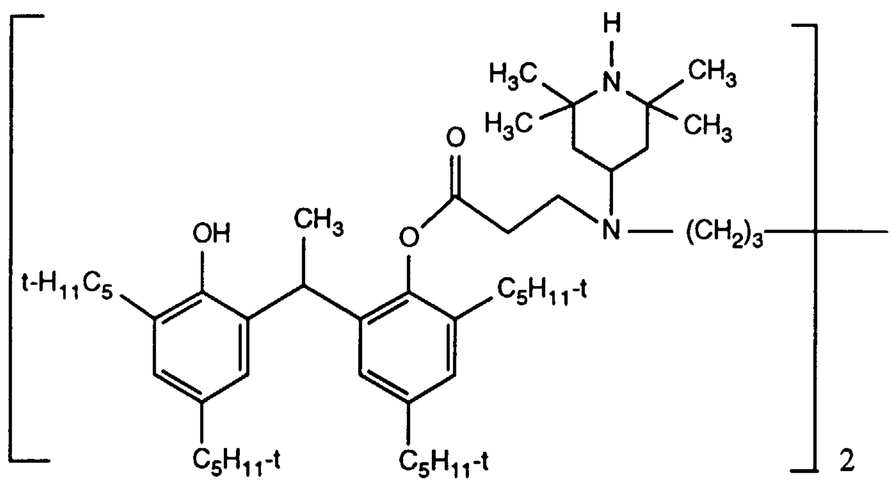 | Elementaranalyse:<br>C% H% N%<br>ber.: 78,87 10,94 3,75<br>gef.: 78,82 10,88 3,66<br>Schmelzpunkt: 66-67°C |

Tabelle 1 (Fortsetzung):

| Verbindung | Struktur | Elementaranalyse<br>Massenspektrum<br>Schmelzpunkt |
|---|---|---|
| 109 | 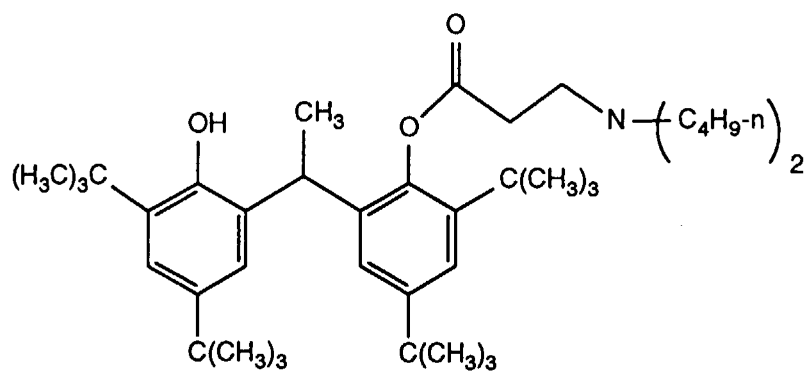 | **Elementaranalyse:**<br>C% H% N%<br>ber.: 79,17 10,86 2,25<br>gef.: 78,96 10,96 2,19<br>**Schmelzpunkt:** 89-90°C |
| 110 | 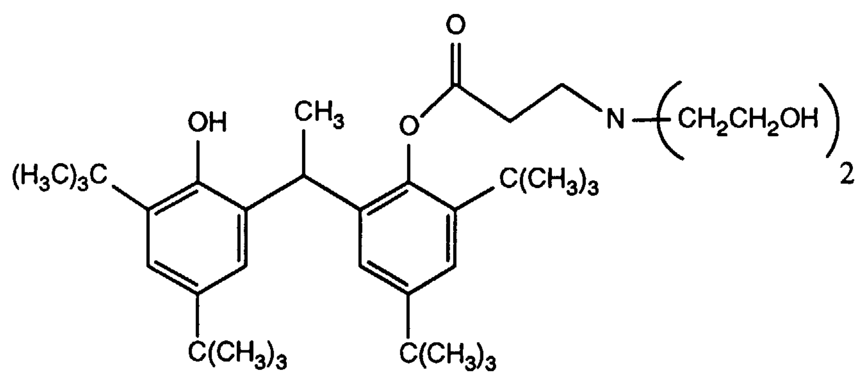 | **Elementaranalyse:**<br>C% H% N%<br>ber.: 74,33 9,95 2,34<br>gef.: 74,50 10,11 2,24<br>**Schmelzpunkt:** 136-138°C |

Tabelle 1 (Fortsetzung):

| Verbindung | Struktur | Elementaranalyse<br>Massenspektrum<br>Schmelzpunkt |
|---|---|---|
| 111 | 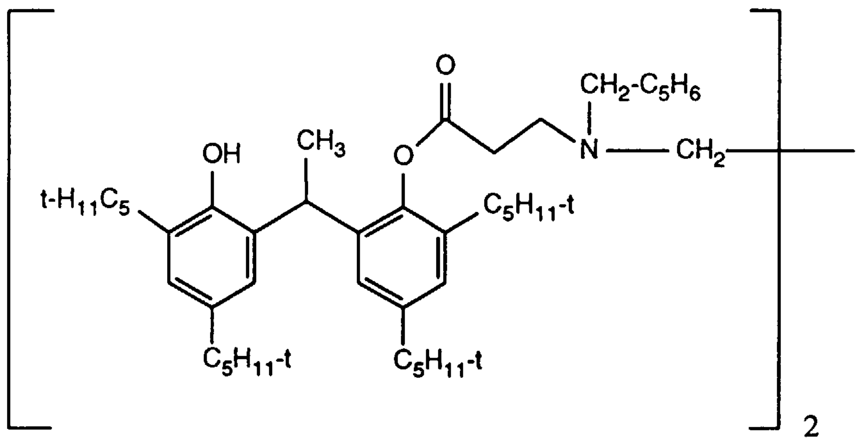 | Elementaranalyse:<br>C% H% N%<br>ber.: 80,91 9,81 2,10<br>gef.: 80,72 9,92 2,04<br>Schmelzpunkt: 129-134°C |
| 112 | 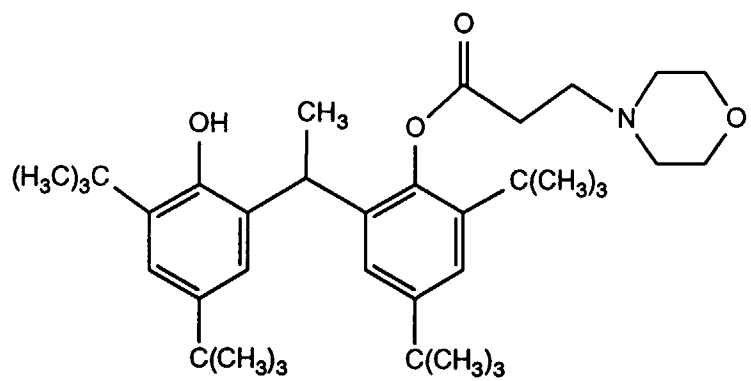 | Elementaranalyse:<br>C% H% N%<br>ber.: 76,64 9,91 2,42<br>gef.: 76,65 9,91 2,37<br>Schmelzpunkt: 150-152°C |

Tabelle 1 (Fortsetzung):

| Verbindung | Struktur | Elementaranalyse Massenspektrum Schmelzpunkt |
|---|---|---|
| 113 | 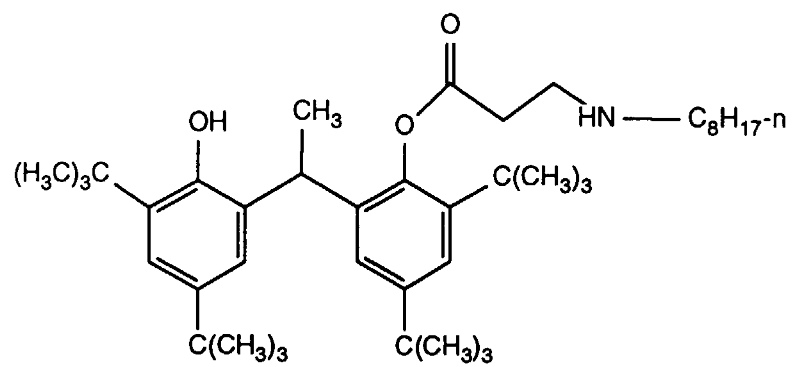 | **Elementaranalyse:**<br>C% H% N%<br>ber.: 79,17 10,86 2,25<br>gef.: 79,02 10,74 2,12<br>**Schmelzpunkt:** 102-104°C |
| 114 | 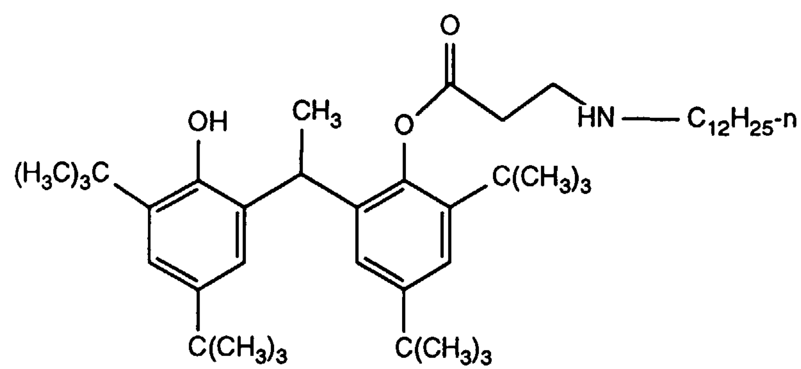 | **Elementaranalyse:**<br>C% H% N%<br>ber.: 79,71 11,15 2,07<br>gef.: 79,45 10,96 1,92<br>**Schmelzpunkt:** 85-86°C |

Tabelle 1 (Fortsetzung):

| Verbindung | Struktur | Elementaranalyse Massenspektrum Schmelzpunkt |
| --- | --- | --- |
| 115 | 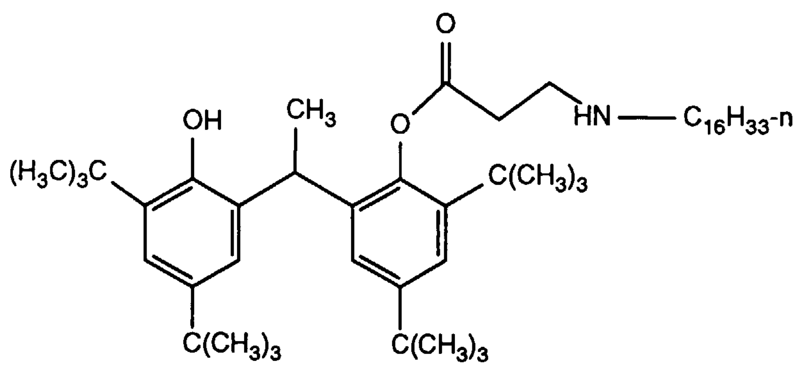 | Elementaranalyse:<br>C% H% N%<br>ber.: 80,16 11,40 1,91<br>gef.: 80,10 11,39 1,70<br>Schmelzpunkt: 62-64°C |
| 116 | 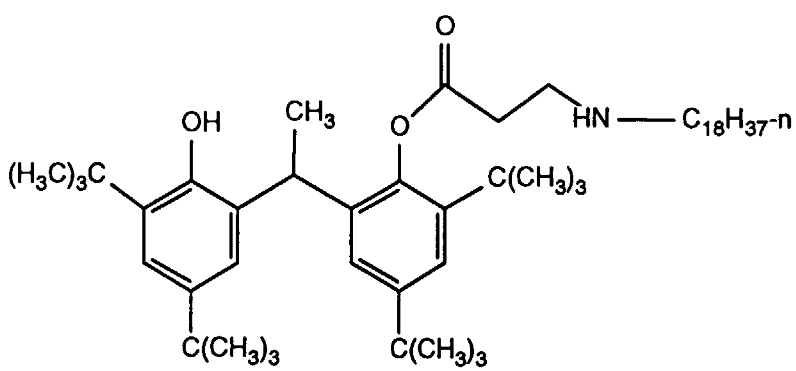 | Elementaranalyse:<br>C% H% N%<br>ber.: 80,36 11,50 1,84<br>gef.: 80,37 11,55 1,79<br>Schmelzpunkt: 64-69°C |

Tabelle 1 (Fortsetzung):

| Verbindung | Struktur | Elementaranalyse<br>Massenspektrum<br>Schmelzpunkt |
|---|---|---|
| 117 | 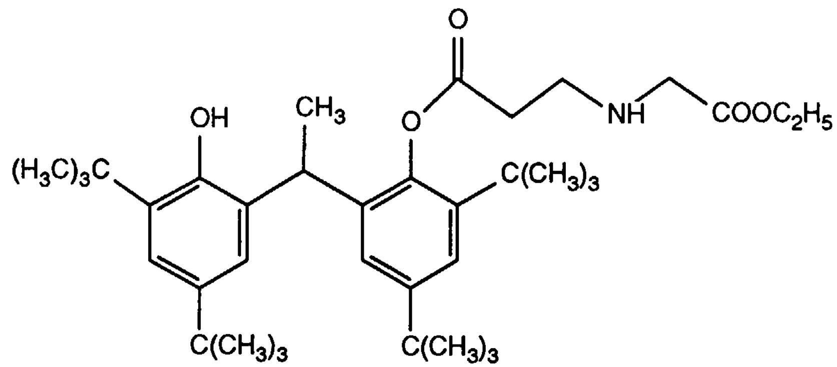 | Elementaranalyse:<br>C% H% N%<br>ber.: 74,58 9,64 2,35<br>gef.: 74,61 9,90 2,37<br>Schmelzpunkt: 121-122°C |
| 118 | 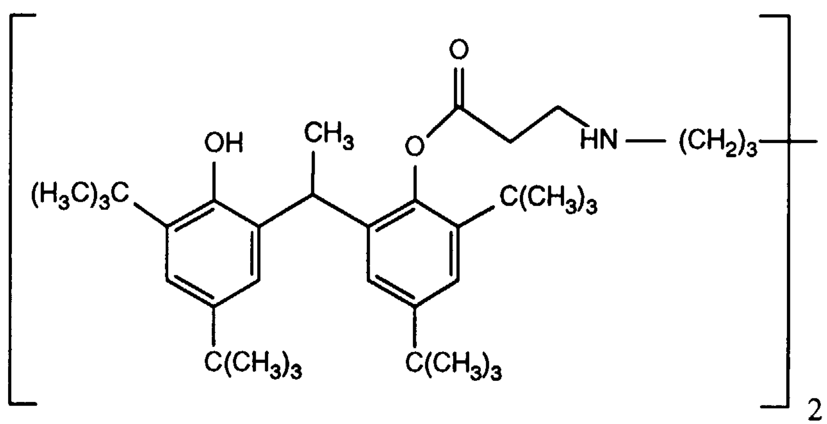 | Elementaranalyse:<br>C% H% N%<br>ber.: 78,50 10,25 2,54<br>gef.: 78,43 10,20 2,58<br>Schmelzpunkt: 148°C |

Tabelle 1 (Fortsetzung):

| Verbindung | Struktur | Elementaranalyse Massenspektrum Schmelzpunkt |
|---|---|---|
| 119 | 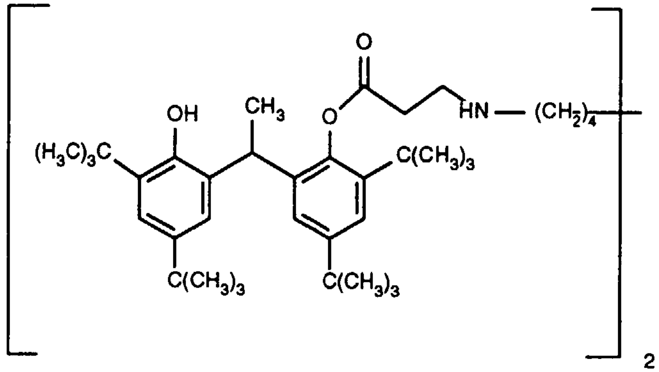 | Elementaranalyse:<br>C% H% N%<br>ber.: 79,12 10,13 2,43<br>gef.: 78,58 10,52 2,40<br>Schmelzpunkt: 72-75°C (amorph) |
| 120 | 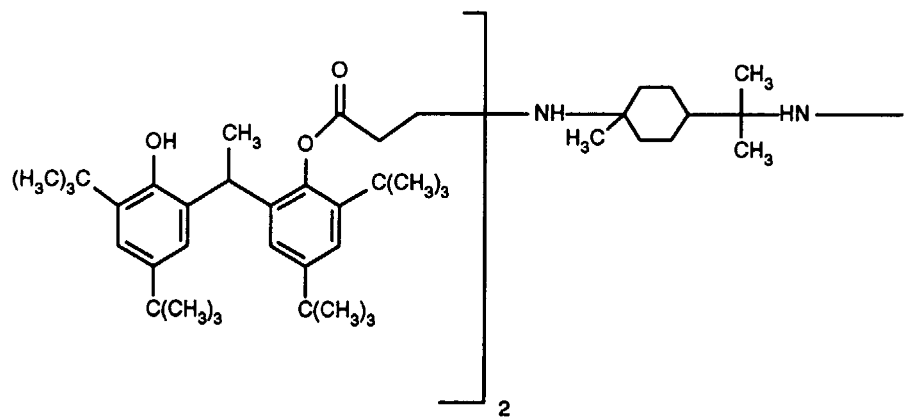 | Elementaranalyse:<br>C% H% N%<br>ber.: 78,98 10,29 2,42<br>gef.: 78,26 10,43 2,42<br>Schmelzpunkt: 102-108°C (amorph) |

Tabelle 1 (Fortsetzung):

| Verbindung | Struktur | Elementaranalyse<br>Massenspektrum<br>Schmelzpunkt |
|---|---|---|
| 121 | 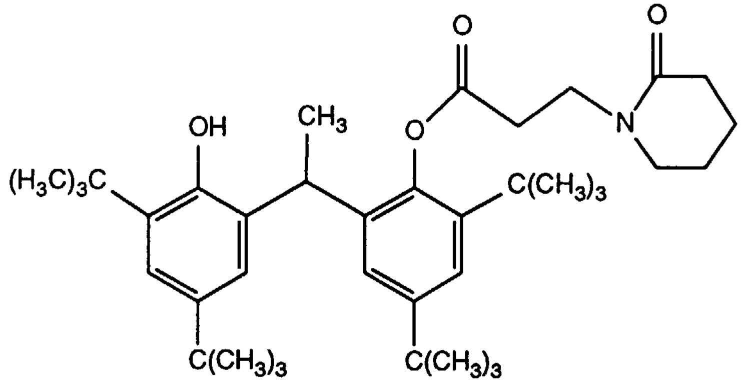 | MS (DCI): MH$^+$ 592<br>IR (KBr): 1740,7 $cm^{-1}$ 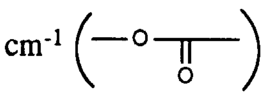<br>1629,4 $cm^{-1}$ 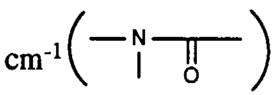<br>Schmelzpunkt: 217-223°C |
| 122 | 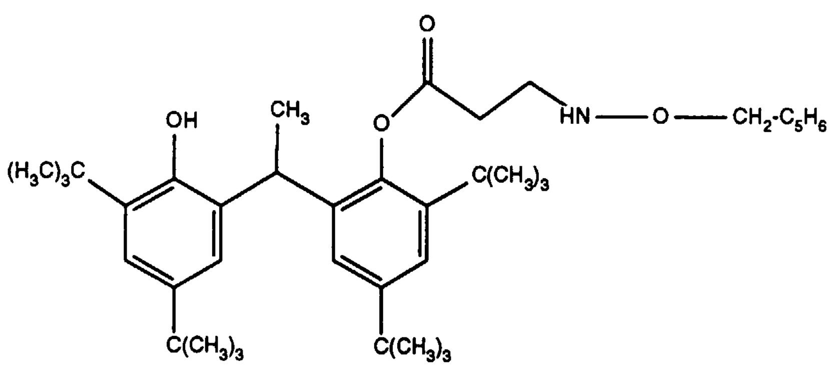 | Elementaranalyse:<br>C% H% N%<br>ber.: 78,01 9,33 2,27<br>gef.: 77,74 9,33 2,27<br>Schmelzpunkt: 123°C |

Tabelle 1 (Fortsetzung):

| Verbindung | Struktur | Elementaranalyse Massenspektrum Schmelzpunkt |
|---|---|---|
| 201 | 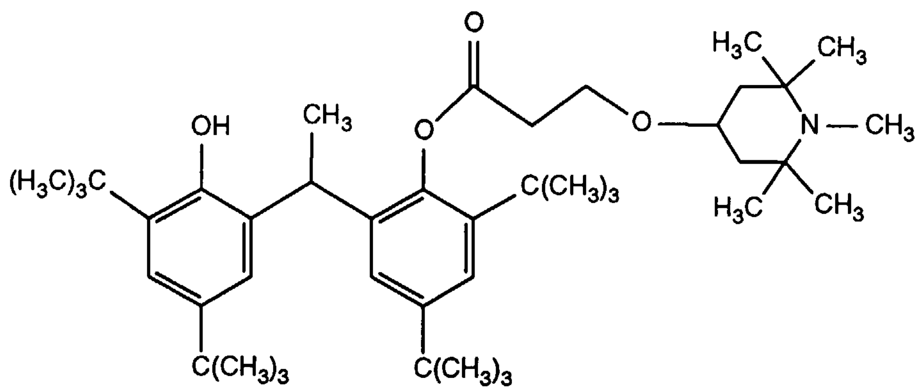 | Elementaranalyse:<br>C% H% N%<br>ber.: 76,42 9,80 2,48<br>gef.: 76,06 9,72 2,40<br>Schmelzpunkt: 104-105°C |
| 202 | 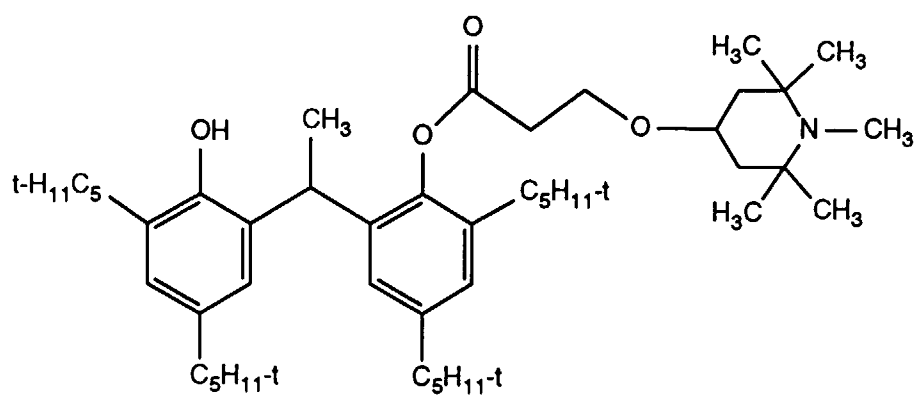 | Elementaranalyse:<br>C% H% N%<br>ber.: 78,02 10,96 1,98<br>gef.: 78,00 10,71 1,88<br>Schmelzpunkt: 46-47°C |

Tabelle 1 (Fortsetzung):

| Verbindung | Struktur | Elementaranalyse Massenspektrum Schmelzpunkt |
|---|---|---|
| 203 | 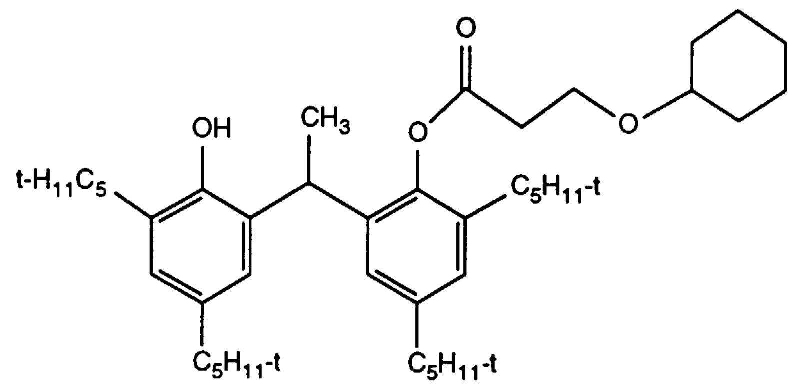 | Elementaranalyse:<br>C% H%<br>ber.: 79,83 10,56<br>gef.: 79,58 10,53<br>Schmelzpunkt: 101-105°C |
| 204 | 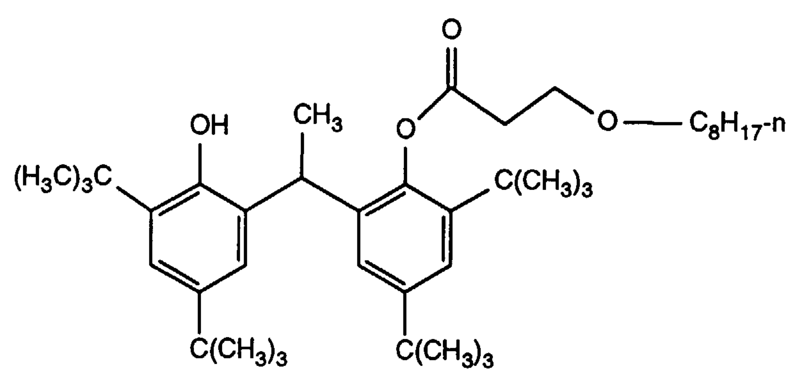 | Elementaranalyse:<br>C% H%<br>ber.: 79,05 10,68<br>gef.: 79,04 11,08<br>Schmelzpunkt: 114-115°C |

Tabelle 1 (Fortsetzung):

| Verbindung | Struktur | Elementaranalyse<br>Massenspektrum<br>Schmelzpunkt |
|---|---|---|
| 205 | 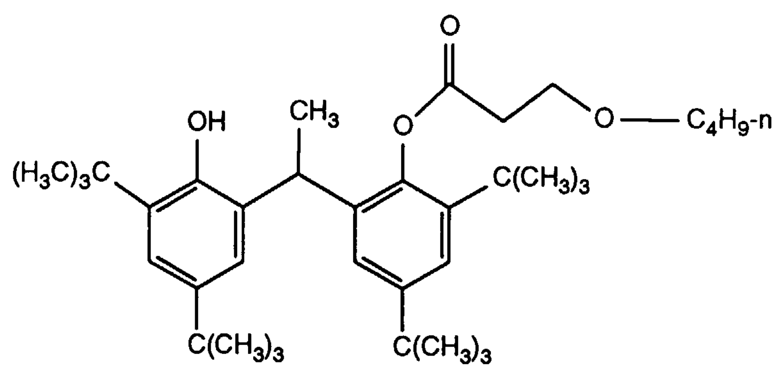 | Elementaranalyse:<br>C% H%<br>ber.: 78,40 10,31<br>gef.: 78,45 10,32<br>Schmelzpunkt: 85-87°C |
| 206 | 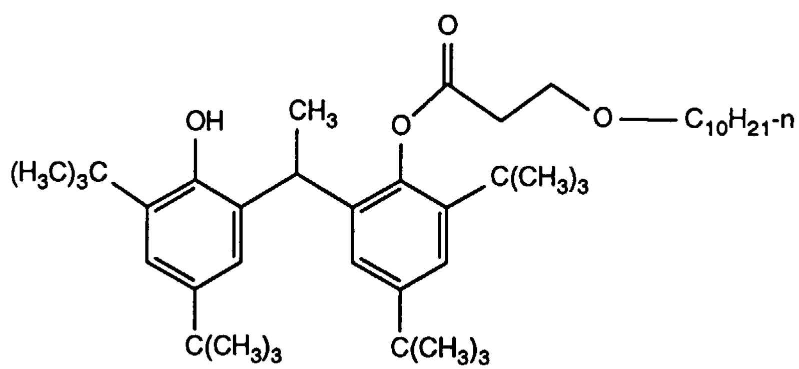 | Elementaranalyse:<br>C% H%<br>ber.: 79,33 10,84<br>gef.: 79,34 10,98<br>Schmelzpunkt: 69-71°C |

Tabelle 1 (Fortsetzung):

| Verbindung | Struktur | Elementaranalyse<br>Massenspektrum<br>Schmelzpunkt |
|---|---|---|
| 207 | 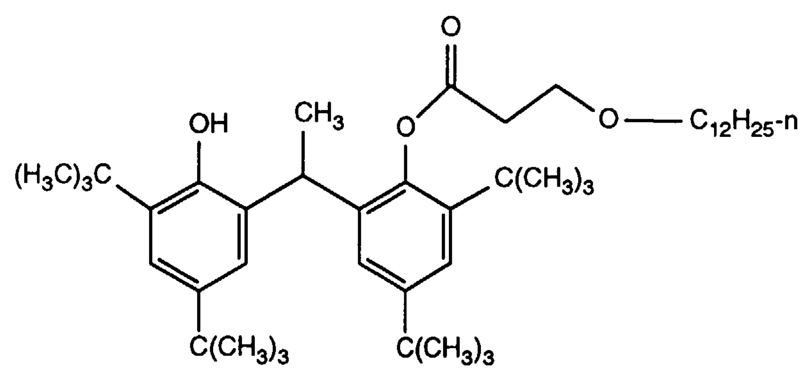 | Elementaranalyse:<br>C% H%<br>ber.: 79,59 10,98<br>gef.: 79,30 11,26<br>Schmelzpunkt: 71-73°C |
| 208 | 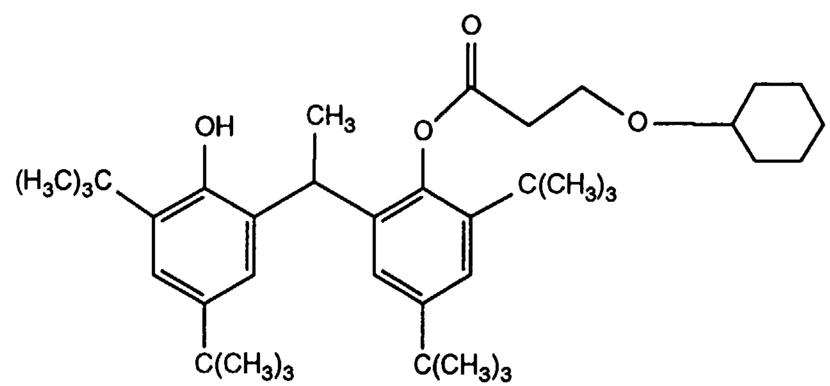 | Elementaranalyse:<br>C% H%<br>ber.: 79,01 10,20<br>gef.: 78,54 10,27<br>Schmelzpunkt: 87-91°C |

Tabelle 1 (Fortsetzung):

| Verbindung | Struktur | Elementaranalyse Massenspektrum Schmelzpunkt |
|---|---|---|
| 209 | 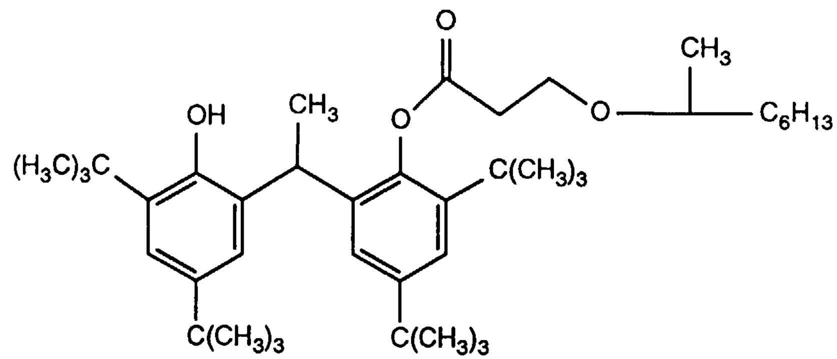 | **Elementaranalyse:**<br>C% H%<br>ber.: 79,05 10,68<br>gef.: 79,10 10,69<br>**Schmelzpunkt:** 64-74°C |
| 210 | 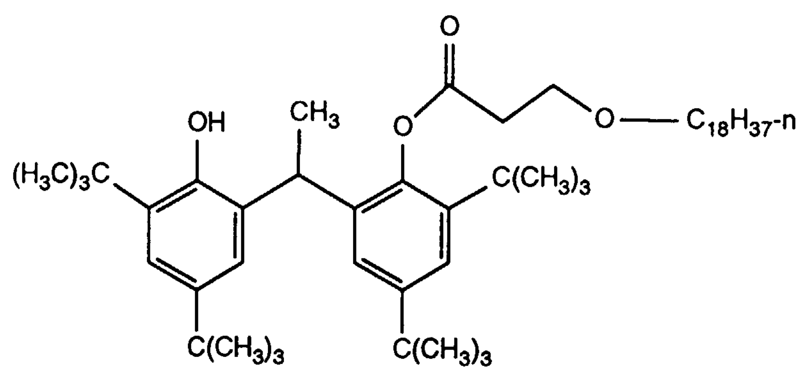 | **Elementaranalyse:**<br>C% H%<br>ber.: 80,26 11,36<br>gef.: 80,29 11,33<br>**Schmelzpunkt:** 65-67°C |

Tabelle 1 (Fortsetzung):

| Verbindung | Struktur | Elementaranalyse<br>Massenspektrum<br>Schmelzpunkt |
|---|---|---|
| 211 | 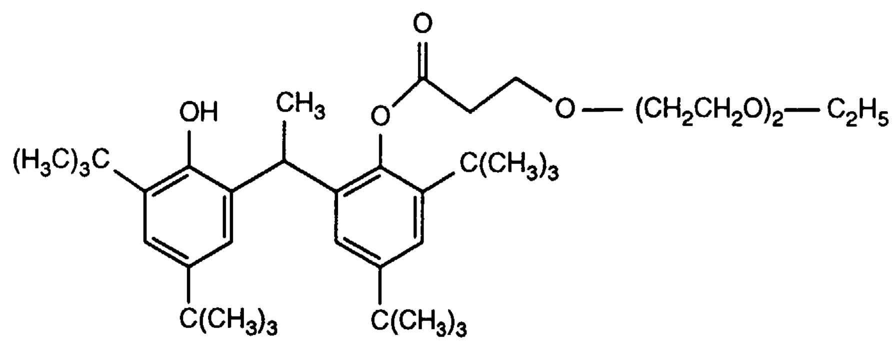 | Elementaranalyse:<br>C% H%<br>ber.: 74,72 9,97<br>gef.: 74,53 10,02<br>Schmelzpunkt: 74-78°C (amorph) |
| 212 | 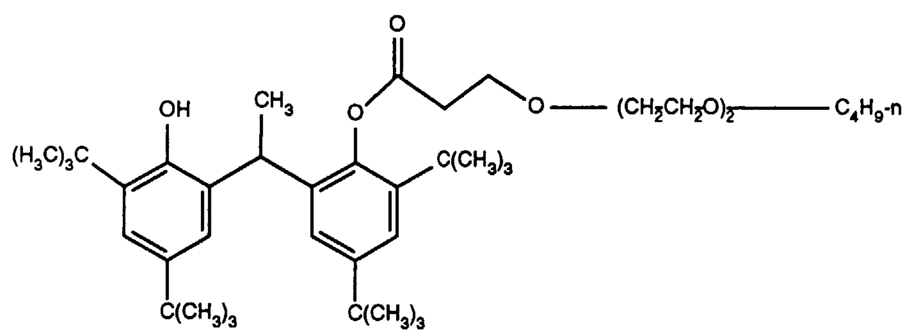 | Elementaranalyse:<br>C% H%<br>ber.: 75,19 10,16<br>gef.: 75,12 10,19 |

Tabelle 1 (Fortsetzung):

| Verbindung | Struktur | Elementaranalyse<br>Massenspektrum<br>Schmelzpunkt |
|---|---|---|
| 213 | 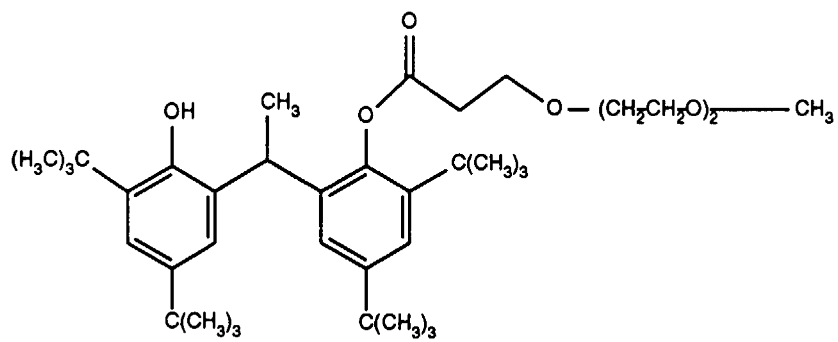 | Elementaranalyse:<br>C% H%<br>ber.: 74,08 9,91<br>gef.: 74,10 9,91<br>Schmelzpunkt: 65-66°C |
| 214 | 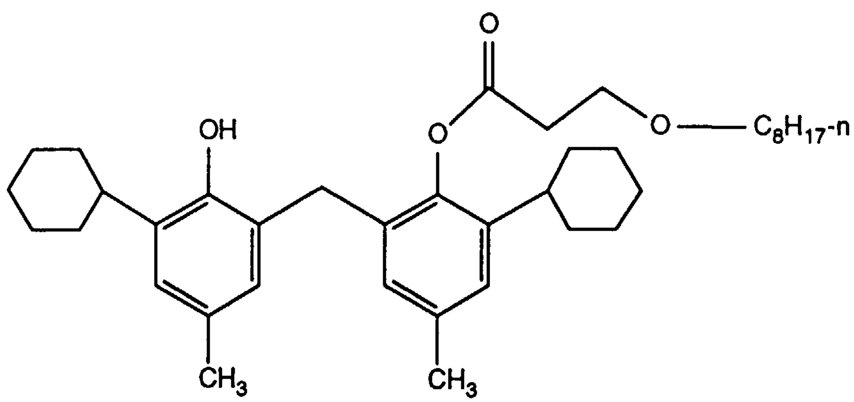 | Elementaranalyse:<br>C% H%<br>ber.: 79,12 9,79<br>gef.: 79,27 9,99 |

Tabelle 1 (Fortsetzung):

| Verbindung | Struktur | Elementaranalyse Massenspektrum Schmelzpunkt |
|---|---|---|
| 214 bis | 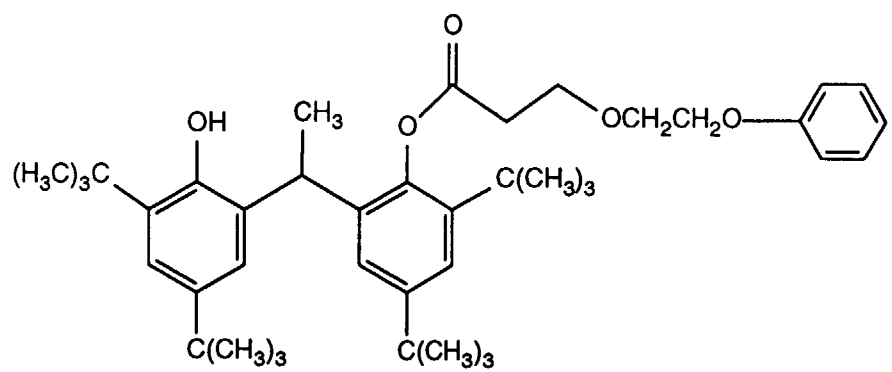 | Elementaranalyse:<br>C% H%<br>ber.: 78,05 9,27<br>gef.: 77,96 9,25<br>Schmelzpunkt: 103-105°C |
| 215 | 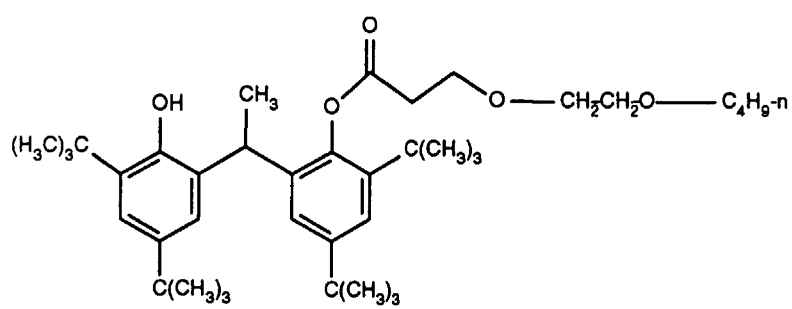 | Elementaranalyse:<br>C% H%<br>ber.: 76,68 10,23<br>gef.: 76,56 10,56<br>Schmelzpunkt: 88-91°C |

Tabelle 1 (Fortsetzung):

| Verbindung | Struktur | Elementaranalyse Massenspektrum Schmelzpunkt |
|---|---|---|
| 216 | 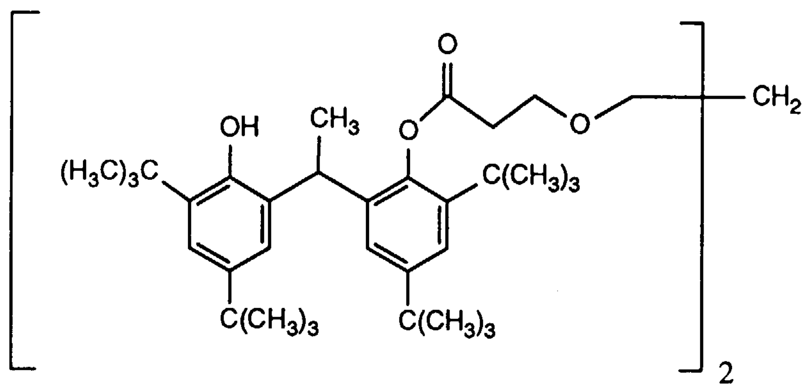 | Elementaranalyse:<br>C% H%<br>ber.: 78,07 9,88<br>gef.: 78,04 9,84<br>Schmelzpunkt: 94°C |
| 217 | 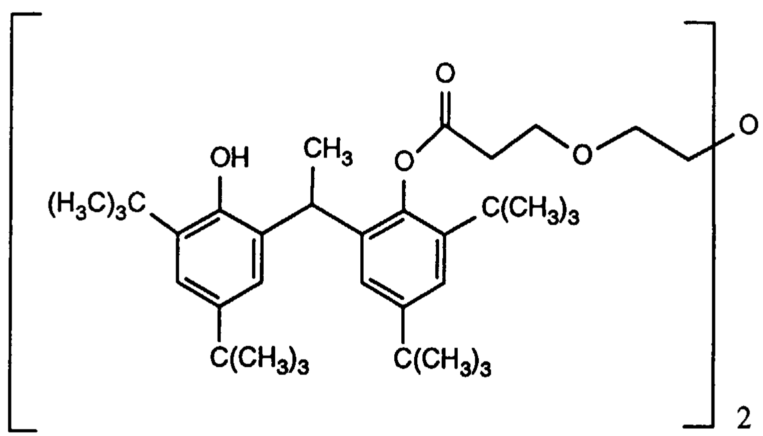 | Elementaranalyse:<br>C% H%<br>ber.: 77,02 9,79<br>gef.: 76,92 9,67<br>Schmelzpunkt: 72-73°C |

Tabelle 1 (Fortsetzung):

| Verbindung | Struktur | Elementaranalyse Massenspektrum Schmelzpunkt |
|---|---|---|
| 218 | 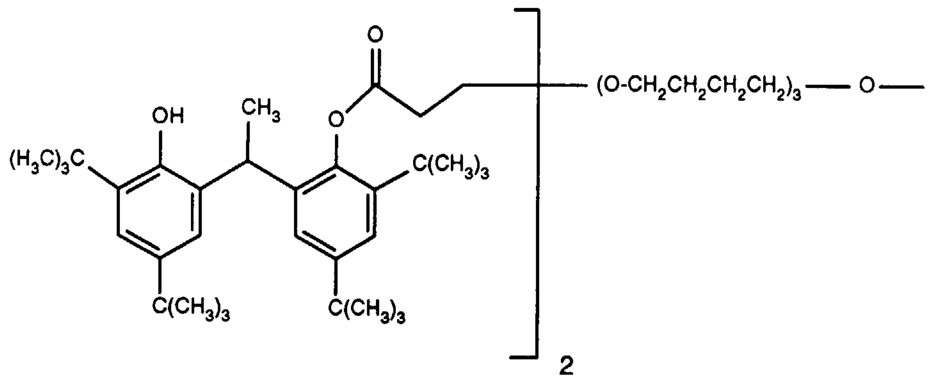 | Elementaranalyse:<br>C% H%<br>ber.: 76,80 10,08<br>gef.: 75,54 10,29<br>Schmelzpunkt: 51-53°C |
| 219 | 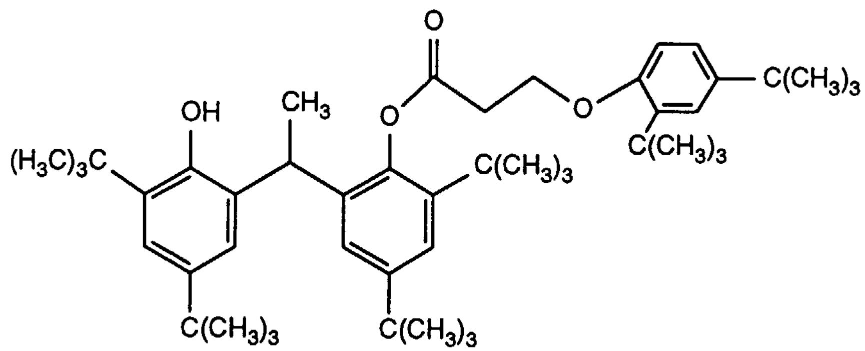 | Elementaranalyse:<br>C% H%<br>ber.: 80,75 10,09<br>gef.: 80,62 10,12<br>Schmelzpunkt: 185°C |

Tabelle 1 (Fortsetzung):

| Verbindung | Struktur | Elementaranalyse Massenspektrum Schmelzpunkt |
|---|---|---|
| 220 | 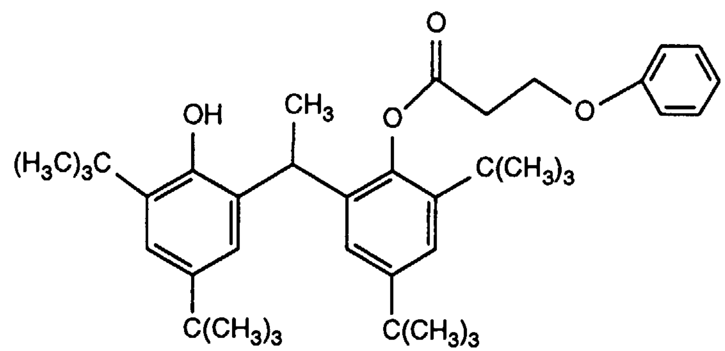 | **Elementaranalyse:**<br>C% H%<br>ber.: 79,82 9,28<br>gef.: 79,70 9,33<br>**Schmelzpunkt:** 113-116°C |
| 221 | 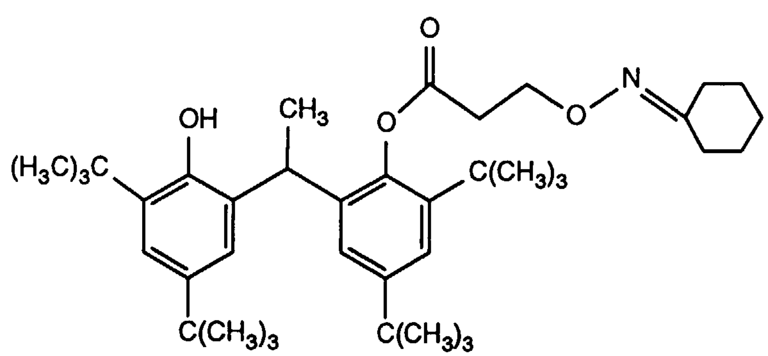 | **Elementaranalyse:**<br>C% H% N%<br>ber.: 77,31 9,82 2,31<br>gef.: 77,07 9,57 2,22<br>**Schmelzpunkt:** 118-121°C |

Tabelle 1 (Fortsetzung):

| Verbindung | Struktur | Elementaranalyse<br>Massenspektrum<br>Schmelzpunkt |
|---|---|---|
| 222 | 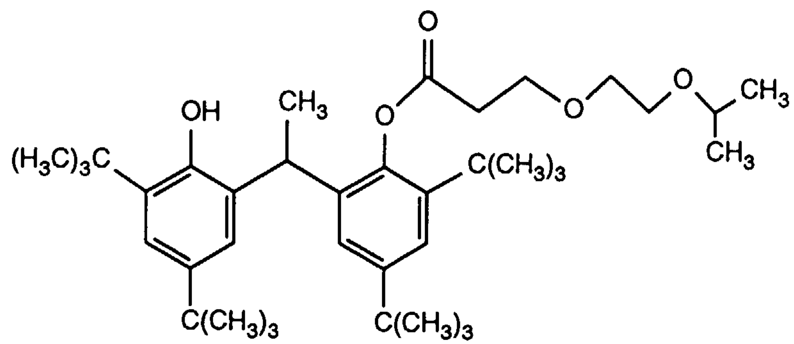 | Elementaranalyse:<br>C% H%<br>ber.: 76,47 10,13<br>gef.: 76,37 10,39<br>Schmelzpunkt: 98°C |
| 223 | 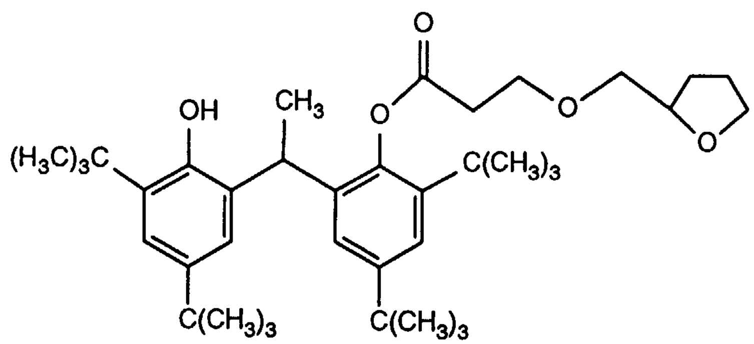 | Elementaranalyse:<br>C% H%<br>ber.: 76,72 9,83<br>gef.: 75,99 9,68<br>Schmelzpunkt: 91-93°C |

Tabelle 1 (Fortsetzung):

| Verbindung | Struktur | Elementaranalyse Massenspektrum Schmelzpunkt |
|---|---|---|
| 224 | 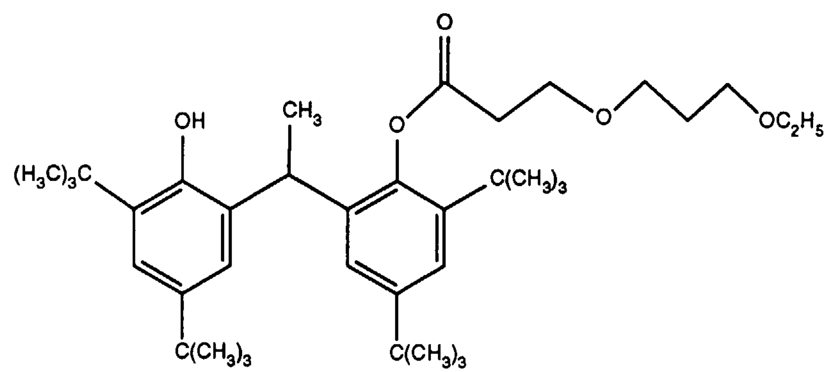 | Elementaranalyse:<br>C% H%<br>ber.: 76,47 10,13<br>gef.: 76,35 10,25<br>Schmelzpunkt: 83-85°C |
| 225 | 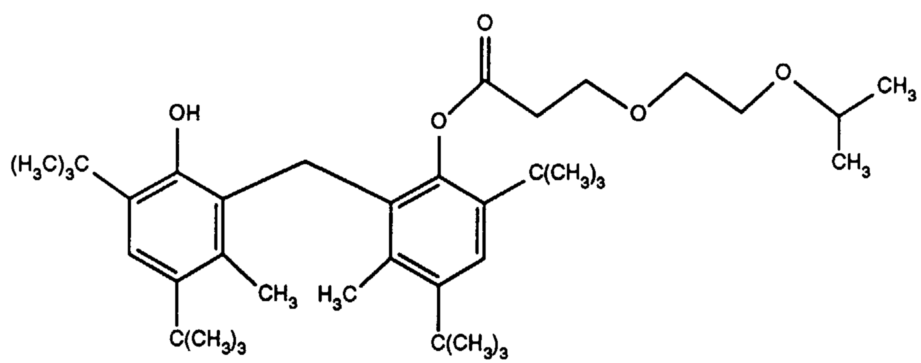 | Elementaranalyse:<br>C% H%<br>ber.: 76,68 10,23<br>gef.: 76,45 10,33<br>Schmelzpunkt: 117-120°C |

Tabelle 1 (Fortsetzung):

| Verbindung | Struktur | Elementaranalyse<br>Massenspektrum<br>Schmelzpunkt |
|---|---|---|
| 301 | 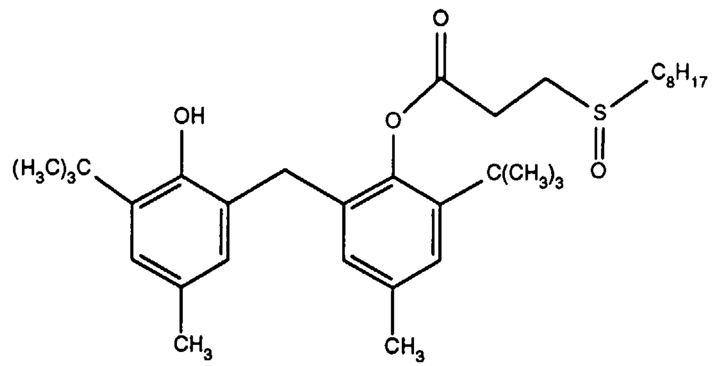 | Elementaranalyse:<br>C% H% S%<br>ber.: 73,34 9,41 5,76<br>gef.: 72,83 9,41 5,30 |
| 302 | 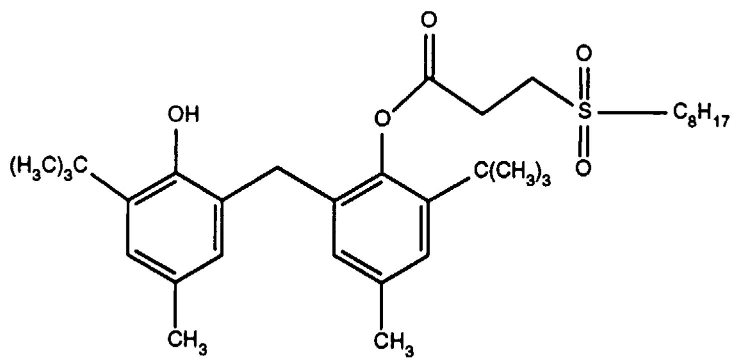 | Elementaranalyse:<br>C% H% N%<br>ber.: 71,29 9,15 5,60<br>gef.: 71,36 9,23 5,65<br>Schmelzpunkt: 108-113°C |

Tabelle 1 (Fortsetzung):

| Verbindung | Struktur | Elementaranalyse<br>Massenspektrum<br>Schmelzpunkt |
|---|---|---|
| 401 | 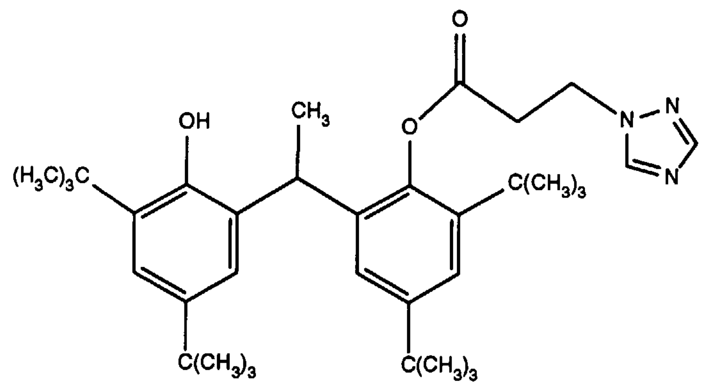 | **Elementaranalyse:**<br>C% H% N%<br>ber.: 74,83 9,15 7,48<br>gef.: 74,77 9,22 7,03<br>**Schmelzpunkt:** 160-162°C |
| 402 | 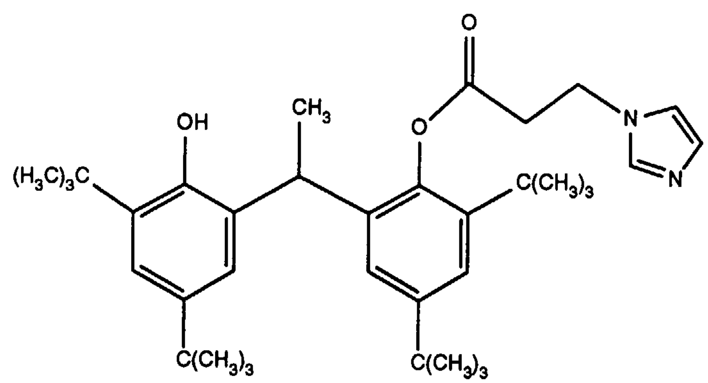 | **Elementaranalyse:**<br>C% H% N%<br>ber.: 77,10 9,35 5,00<br>gef.: 76,94 9,40 4,91<br>**Schmelzpunkt:** 88-92°C |

Tabelle 1 (Fortsetzung):

| Verbindung | Struktur | Elementaranalyse Massenspektrum Schmelzpunkt |
|---|---|---|
| 403 | 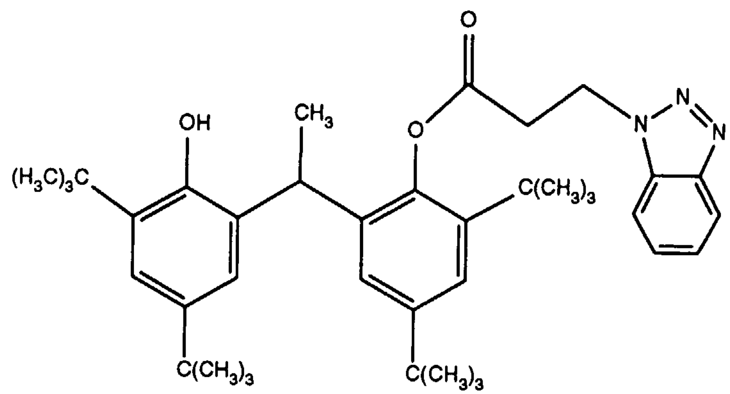 | Elementaranalyse:<br>C% H% N%<br>ber.: 76,56 8,73 6,87<br>gef.: 76,30 8,70 6,87<br>Schmelzpunkt: 174-178°C |
| 404 | 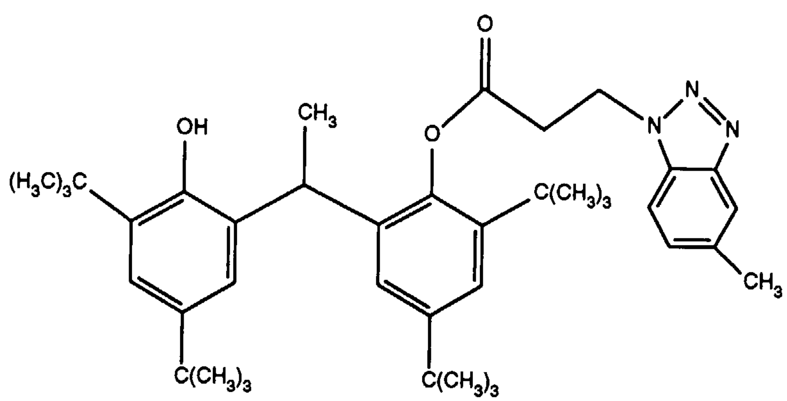 | Elementaranalyse:<br>C% H% N%<br>ber.: 76,76 8,86 6,71<br>gef.: 76,22 9,20 6,57<br>Schmelzpunkt: 81-84°C |

Tabelle 1 (Fortsetzung):

| Verbindung | Struktur | Elementaranalyse Massenspektrum Schmelzpunkt |
|---|---|---|
| 405 | 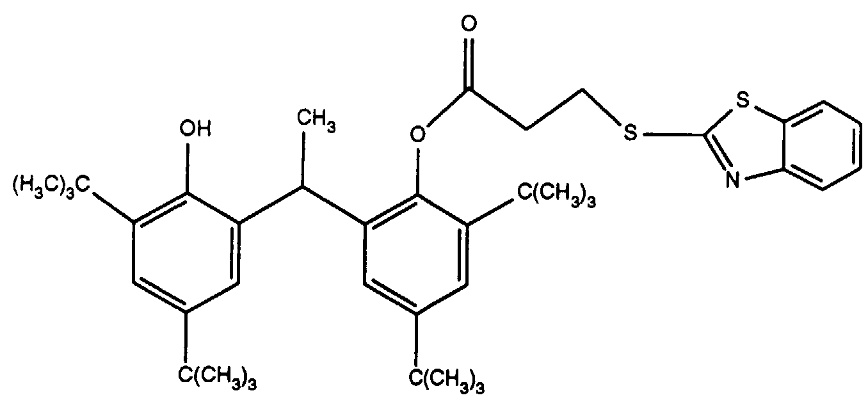 | Elementaranalyse:<br>C% H% N% S%<br>ber.: 72,80 8,09 2,12 9,72<br>gef.: 72,58 8,08 2,16 9,79<br>Schmelzpunkt: 123-128°C |

Die Verbindungen 113, 116, 117, 215 und 222 stellen besonders bevorzugte Ausführungsformen der Erfindung dar.

Die Verbindungen der Formel I eignen sich zum Stabilisieren von organischen Materialien gegen thermischen, oxidativen oder lichtinduzierten Abbau. Beispiele für derartige Materialien sind:

1. Polymere von Mono- und Diolefinen, beispielsweise Polypropylen, Polyisobutylen, Polybuten-1, Poly-4-methylpenten- 1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), verzweigtes Polyethylen niederer Dichte (VLDPE).
Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide, Polyetherimide, Polyesterimide, Polyhydantoine und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.

26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethern, Bisphenol-F-diglycidylethern, die mittels üblichen Härtern wie z.B. Anhydriden oder Aminen mit oder ohne Beschleunigern vernetzt werden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PBT/PC/ABS oder PBT/PET/PC.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Ein weiterer Gegenstand der Erfindung ist daher eine Zusammensetzung enthaltend ein gegen oxidativen, thermischen oder lichtinduzierten Abbau empfindliches organisches Material und mindestens eine Verbindung der Formel I.

Bei dem organischen Material handelt es sich vorzugsweise um synthetische Polymere, insbesondere solche aus den oben angegebenen Gruppen. Polyolefine und ein in Lösung polymerisierter Polybutadien-Kautschuk sind besonders bevorzugt. Ebenfalls besonders bevorzugt ist ein in Lösung polymerisiertes Styrol-Butadien-Copolymer oder Styrol-Butadien-Blockcopolymer, worin das Verhältnis von Styrol zu konjugiertem Butadien zum Beispiel 5:95 bis 95:5 beträgt. Bevorzugt liegt der Anteil von Polybutadien in diesen Copolymeren bei 5 bis 30%. Bevorzugt wird als organisches Material auch Acrylnitril-Butadien-Styrol eingesetzt.

Im allgemeinen werden die Verbindungen der Formel I dem zu stabilisierenden organischen Material in Mengen von 0,01 bis 10 %, vorzugsweise 0,01 bis 5 %, insbesondere 0,05 bis 0,5 %, bezogen auf das Gesamtgewicht des zu stabilisierenden Materials, zugesetzt.

Die Einarbeitung der erfindungsgemässen Verbindungen in das organische Material kann nach bekannten Methoden erfolgen, beispielsweise vor oder während der Formgebung oder durch Aufbringen der gelösten oder dispergierten Verbindungen auf das organische Material, gegebenenfalls unter nachträglichem Verdunsten des Lösungsmittels. Die erfindungsgemässen Verbindungen können als Pulver, Granulat oder auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die stabilisierten organischen Materialien der Erfindung können zusätzlich auch verschiedene herkömmliche Additive enthalten, wie beispielsweise:

1. Antioxidantien

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.

1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis-(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-bu-tyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1 ,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methylbenzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.

1.9. Hydroxybenzyl-Aromaten, z.B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiarnin, N,N'-Bis(3,5-di-tert-butyl-4-hydroxyphenylpropionyl)-trimethylendiamin, N, N'-Bis(3,5-di-tert-butyl-4-hydroxyphenylpropionyl)-hydrazin.

1.18. Ascorbinsäure (Vitamin C).

1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis (1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluolsulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylaminophenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N, N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300;

$$[R-CH_2CH_2-COO(CH_2)_3]_2-$$

mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäure-methylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiarnin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylarnino)-ethan, Kondensationsprodukt aus 2-Chlor-4,6-di-(4-n-butylamino- 1,2,2,6,6-pentamethylpiperidyl)-1,3,5-triazin und 1,2-Bis(3-aminopropylamino)-äthan, 8-Acetyl-3-dodecyl-7,7,9,9-tetramethyl- 1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)- 1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis-(2,4-dimethylphenyl)-1,3,5-triazin, 2-[2-Hydroxy-4-(2-hydroxy-3-butyloxy-propyloxy)-phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin.

3. Metalldesaktivatoren, wie z.B. N,N'-Diphenyloxalsäurediarnid, N-Salicylal-N'-salicyloylhydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz-[d,g]-1,3,2-dioxaphosphocin, Bis(2,4-di-tert-butyl-6-methylphenyl)-methylphosphit, Bis-(2,4-di-tert-butyl-6-methylphenyl)-ethylphosphit.

5. Hydroxylamine wie z.B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.

6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecylnitron, N-Hexadecyl-alpha-pentadecyl-nitron, N-Octadecyl-alpha-heptadecyl-nitron, N-Hexadecyl-alpha-heptadecyl-nitron, N-Ocatadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.

7. Thiosynergisten wie z.B. Thiodipropionsäure-di-laurylester oder Thiodipropionsäuredi-stearylester.

8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.

9. Polyamidstabilisatoren, wie z.B. Kupfersalze in Kombination mit Jodiden und/oder Phosphorverbindungen und Salze des zweiwertigen Mangans.

10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere").

12. Füllstoffe und Verstärkungsmittel, wie z.B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.

13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.

14. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Das Gewichtsverhältnis von erfindungsgemässen Verbindungen zu den herkömmlichen Additiven kann beispielsweise 1:0,5 bis 1:5 betragen.

Ein weiterer Gegenstand der Erfindung ist die Verwendung der Verbindungen der Formel I zum Stabilisieren von organischem Material gegen oxidativen, thermischen oder lichtinduzierten Abbau.

Die folgenden Beispiele erläutern die Erfindung weiter. Alle Mengenangaben entsprechen Gewichtsteilen sofern nichts anderes angegeben ist.

Beispiel 1: Herstellung der Verbindung 101 (Verfahren A).

7,2 g (18 mMol) 2,2'-Methylen-bis[6-tert-butyl-4-methylphenol]monoacrylat werden in 70 ml Dichlormethan gelöst. Zu dieser Lösung werden bei Raumtemperatur (RT) langsam 2,17 ml (20 mMol) Benzylamin getropft. Anschliessend wird die Mischung während 3 Stunden bei RT weitergerührt. Eindampfen des Lösungsmittels und Chromatographie des Rückstandes an Kieselgel (Laufmittel: Hexan) liefern 6,63 g (66 %) der Verbindung 101 als weisses Pulver. Der Schmelzpunkt beträgt 124-126°C.

Die Verbindungen 102-104 werden nach der in Beispiel 1 beschriebenen Methode hergestellt

Beispiel 2: Herstellung der Verbindung 105 (Verfahren A).

In einem Rundkolben mit Magnetrührer und Kühler werden unter Argon-Atmosphäre 16,57 g (42 mMol) 2,2'-Methylen-bis[6-tert-butyl-4-methylphenol]monoacrylat und 2,17 ml (20 mMol) Benzylamin in 150 ml Dichlorethan vorgelegt Das Reaktionsgemisch wird über Nacht am Rückfluss gekocht und anschliessend am Vakuumrotationsverdampfer

eingeengt. Die Chromatographie des Rückstandes an Kieselgel (Laufmittel: Hexan → Hex/EE 80:1; Hex = Hexan, EE= Essigsäureethylester) liefert 12,44 g (69 %) der Verbindung 105 als weisses Pulver. Der Schmelzpunkt beträgt 70-76 °C.

Die Verbindungen 106-110 werden nach der in Beispiel 2 beschriebenen Methode hergestellt

Beispiel 3: Herstellung der Verbindung 111 (Verfahren A).

Ein Gemisch von 4,6 g (8,3 mMol) 2,2'-Ethyliden-bis[4,6-di-tert-amylphenol]monoacrylat und 1 g (4,16 mMol) N, N'-Dibenzylethylendiamin wird während 4 Stunden bei 140°C gerührt. Nach Abkühlen auf RT und Chromatographie des erhaltenen Rohprodukts an Kieselgel (Laufmittel: Hexan) werden 5,15 g (93 %) der Verbindung 111 als amorphes Pulver erhalten. Der Schmelzpunkt beträgt 129-134°C.

Beispiel 4: Herstellung der Verbindung 112 (Verfahren C).

a) Herstellung von 3-Chlorpropionsäure-2,4-di-tert-butyl-6-[1-(3,5-di-tert-butyl-2-hydroxyphenyl)-ethyl]-phenylester.

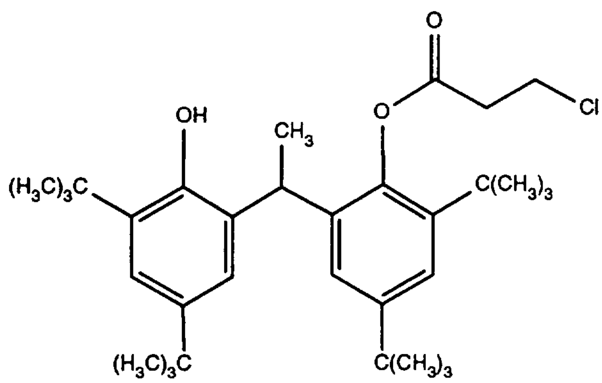

112a

In einem 250 ml Rundkolben mit Magnetrührer, Kühler und Gasblasenzähler werden 21,9 g (50 mMol) 2,2'-Ethyliden-bis[4,6-di-tert-butylphenol] und 48 ml (500 mMol) 3-Chlorpropionsäurechlorid in 100 ml Xylol vorgelegt Die Mischung wird während 15 Stunden am Rückfluss gekocht (HCl-Entwicklung). Der Überschuss an Säurechlorid sowie das Toluol werden abdestilliert. Die Kristallisation des Rückstandes in Acetonitril liefert 15,8 g (60 %) des gewünschten Monoesters 112 a als beiges Pulver. Der Schmelzpunkt beträgt 118-121°C.

| Elementaranalyse: | C% | H% | Cl% |
|---|---|---|---|
| berechnet: | 74,90 | 9,33 | 6,70 |
| gefunden: | 75,10 | 9,29 | 6,13 |

b) Zu einer unter Stickstoff-Atmosphäre gerührten Lösung von 4,5 g (8,5 mMol) der Verbindung 112a (Beispiel 4a) in 30 ml Toluol, werden bei RT 1,63 g (18,7 mMol) Morpholin getropft. Das Reaktionsgemisch wird während 1 Stunde bei 70°C gerührt, auf RT abgekühlt, über Hyflo filtriert und eingedampft. Die Kristallisation des Rückstandes in Hexan liefert 3,9 g (80 %) der Verbindung 112 als weisses Pulver. Der Schmelzpunkt beträgt 150-152°C.

Beispiel 5: Herstellung der Verbindung 113 (Verfahren A).

Ein Gemisch von 19,7 g (0,04 Mol) 2,2'-Ethyliden-bis[4,6-di-tert-butylphenol]monoacrylat (Herstellung siehe z.B EP-A-500 323) und 6,72 g (0,052 Mol) Octylamin wird auf 115°C erwärmt. Die erhaltene farblose Lösung wird während 30 Minuten bei dieser Temperatur gerührt. Anschliessend werden 50 ml Ethanol zugegeben. Beim Abkühlen auf RT kristallisiert das Produkt aus. Nach Filtration und Trocknung unter Vakuum werden 21,9 g (88 %) der Verbindung 113 als weisses Pulver erhalten. Der Schmelzpunkt beträgt 102-104°C.

Die Verbindungen 114, 115 und 116 werden analog zu dem im Beispiel 5 beschriebenen Verfahren hergestellt.

Beispiel 6: Herstellung der Verbindung 117 (Verfahren A).

In einem 200 ml Sulfierkolben mit Thermometer, Rührer, Rückflusskühler und Tropftrichter werden unter $N_2$-At-

mosphäre 6,41 g (13 mMol) 2,2'-Ethyliden-bis[4,6-di-tert-butylphenol]monoacrylat, 2 g (14,3 mMol) Glycin-ethylester. Hydrochlorid und 50 ml Ethylenchlorid vorgelegt. 2 ml (1,45 g, 14,3 mMol) Triethylamin werden zugetropft. Nach Ende der Zugabe wird das Gemisch auf 50°C erwärmt und während 5 Stunden bei dieser Temperatur gerührt. Die erhaltene Suspension wird mit 25 ml Hexan verdünnt und die Salze abfiltriert. Nach Eindampfen des Lösungsmittels am Vakuumrotationsverdampfer und Kristallisation des erhaltenen Rohproduktes aus Hexan werden 3,7 g (48 %) der Verbindung 117 als weisses Pulver erhalten. Der Schmelzpunkt beträgt 121-122°C.

Beispiel 7: Herstellung der Verbindung 118 (Verfahren A).

Ein Gemisch von 6,92 g (14 mMol) 2,2'-Ethyliden-bis[4,6-di-tert-butylphenol]monoacrylat und 0,89 g (7,7 mMol) 1,6-Diaminohexan in 40 ml Essigester wird während 30 Minuten am Rückfluss erhitzt. Anschliessend wird das Lösungsmittel abgedampft. Die Kristallisation des Rückstandes aus Acetonitril liefert 5 g (65 %) der Verbindung 118 als weisses Pulver. Der Schmelzpunkt beträgt 148°C.

Beispiel 8: Herstellung der Verbindung 121 (Verfahren B).

Ein Gemisch von 2,05 g (12 mMol) 3-[2-oxo-piperidino]-propionsäure (siehe z.B. Dado, G.P; Gellman, S.H.; J. Am. Chem. Soc., 116(3), 1054-62, 1994), 4,4 g (10 mMol) 2,2'-Ethyliden-bis[4,6-di-tert-butylphenol], 2,06 g (10 mMol) Dicyclohexylcarbodiimid und 50 mg Dimethylaminopyridin in 60 ml Dichlorethan wird während 15 Stunden am Rückfluss gekocht. Anschliessend wird der ausgefallene Harnstoff abfiltriert und das Lösungsmittel abgedampft. Die Kristallisation des Rückstandes aus einem Gemisch Hexan-Essigester 1:1 liefert 2,6 g (44 %) der Verbindung 121 als weisses Pulver. Der Schmelzpunkt beträgt 217-223°C.

Beispiel 9: Herstellung der Verbindung 122 (Verfahren A).

Ein Gemisch von 6,6 g (13 mMol) 2,2'-Ethyliden-bis[4,6-di-tert-butylphenol]monoacrylat, 4 g (23,5 mMol) O-Benzylhydroxyl-amin-hydrochlorid und 38 ml (27 mMol) Triethylamin in 70 ml Dichlorethan wird während 8 Stunden bei 65°C gerührt. Anschliessend wird das Reaktionsgemisch mit ca. 10 ml Hexan verdünnt, die Salze werden abfiltriert und die Lösungsmittel am Vakuumrotationsverdampfer abdestilliert. Die Kristallisation des Rückstandes aus Methanol liefert 4,6 g (55 %) der Verbindung 122 als weisses Pulver. Der Schmelzpunkt beträgt 123°C.

Beispiel 10: Herstellung der Verbindung 201 (Verfahren A).

In einem mit Stickstoff inertisierten 250 ml-Rundkolben werden 5,65 g (33 mMol) 4-Hydroxy-1,2,2,6,6-pentamethylpiperidin in 85 ml Tetrahydrofuran vorgelegt Die Lösung wird auf 0°C abgekühlt und 5,6 ml (9 mMol) einer 1,6 N Butyllithium Lösung in Hexan langsam zugetropft. Die erhaltene weisse Suspension wird während 30 Minuten nachgerührt und anschliessend eine Lösung von 11,84 g (30 mMol) 2,2'-Ethyliden-bis[4,6-di-tert-butylphenol]monoacrylat in 85 ml Tetrahydrofuran langsam zugetropft. Die erhaltene gelbe Lösung wird während 3 Stunden bei RT gerührt. Daraufhin wird das Reaktionsgemisch eingedampft, auf wässrige, gesättigte Ammoniumchlorid-Lösung gegossen und mit Essigester mehrmals extrahiert. Die organischen Phasen werden vereinigt, über Kaliumcarbonat getrocknet und am Vakuumrotationsverdampfer eingeengt. Die Chromatographie des Rückstandes ($SiO_2$:Hex/EE 9:1 $\rightarrow$ 1:1) liefert 11,06 g (65 %) der Verbindung 201 als weisses Pulver. Der Schmelzpunkt beträgt 104-105°C.

Die Verbindungen 202 und 203 werden in Analogie zu dem im Beispiel 4 beschriebenen Verfahren hergestellt.

Beispiel 11: Herstellung der Verbindung 204 (Verfahren B).

a) Herstellung von 3-Octyloxypropionsäure

Zu einer farblosen Lösung von 39,1 g (0,3 Mol) Octanol und 1,2 ml (2,9 Mol) Benzyltrimethylammoniumhydroxid (40%ige Lösung in Wasser) wird bei RT und unter $N_2$-Atmosphäre innerhalb 25 Minuten 21,7 ml (0,33 Mol) Acrylonitril getropft (exotherm: Ti 40°C). Nach Ende der Zugabe lässt man weitere 25 Minuten bei RT nachrühren. Der Überschuss von Acrylonitril wird destillativ entfernt (Vakuumrotationsverdampfer). Das erhaltene rohe 3-Octyloxypropionitril (hellgelbe Flüssigkeit; 97 % GC) wird in 150 ml konz. Salzsäure und 150 ml Essigsäure gelöst und die Lösung während 3 Stunden auf 85°C erwärmt. Das Gemisch wird abgekühlt, auf 100 ml Wasser gegossen und mit Essigester extrahiert. Die organische Pase wird abgetrennt, mit einer wässrigen, gesättigten NaCl-Lösung gewaschen, über $Na_2SO_4$ getrocknet und am Vakuumrotationsverdampfer eingeengt. Man erhält 60 g 3-Octyloxypropionsäure als farblose Flüssigkeit (85 % GC $\rightarrow$ 85 % Ausbeute).

| Elementaranalyse: | C% | H% |
|---|---|---|
| berechnet: | 65,31 | 10,96 |
| gefunden: | 64,94 | 11,09 |

b) In einem 100 ml-Rundkolben mit Magnetrührer und Kühler werden unter Argon-Atmosphäre 7,08 g (35m Mol) 3-Octyloxypropionsäure (siehe Beispiel 11a) in 50 ml Toluol vorgelegt. Zu dieser Lösung werden 7,6 ml (18,5 g, 105 mMol) Thionylchlorid gegeben. Das Gemisch wird langsam auf 90°C erwärmt (HCl und $SO_2$ Entwicklung) und während 2 Stunden bei dieser Temperatur gerührt. Nun wird unter Einleiten eines Argon-Stroms weitere 30 Minuten nachgerührt. Bei reduziertem Druck (20 mbar) wird der Ueberschuss an Thionylchlorid und das Toluol abdestilliert. Der Rückstand (7 g 3-Octyloxypropionsäurechlorid) wird in 50 ml Toluol gelöst und 10,97 g (25 mMol) 2,2'-Ethyliden-bis[4,6-di-tert-butylphenol] zugegeben. Zu dieser Lösung werden bei 10°C 4,9 ml (35 mMol) Triethylamin getropft. Nach ca. 10 Minuten wird das Gemisch auf RT erwärmt und während 1 Stunde 30 Minuten bei RT nachgerührt. Anschliesend wird das Reaktionsgemisch über Celite filtiert und das Filtrat am Vakuumrotationsverdampfer eingeengt. Der Rückstand wird in ca. 30 ml Acetonitril aufgenommen, wobei die Verbindung 204 auskristallisiert. Absaugen und Trocknung des Produktes liefern 14,05 g (90 %) der Verbindung 204 als weisses Pulver. Der Schmelzpunkt beträgt 114°C.

Die Herstellung der Verbindungen 205 bis 214 bis wird in Analogie zu Beispiel 11 durchgeführt.

<u>Beispiel 12:</u> Herstellung der Verbindung 204 (Verfahren B).

In einem 100 ml Sulfierkolben mit Thermometer, Rührer, Rückflusskühler und Tropftrichter werden unter $N_2$-Atmosphäre 6,6 g (15 mMol) 2,2'-Ethyliden-bis[4,6-di-tert-butylphenol], 3,8 g (15 mMol) 3-Octyloxypropionsäure und 6 ml Heptan vorgelegt. Zu dieser Suspension werden 4,4 ml (31,5 mMol) Triethylamin (die Suspension löst sich) und anschliessend 0,96ml (10,5 mMol) Phosphoroxychlorid getropft (exotherm: Ti 60°C). Die erhaltene Suspension wird während 1 Stunde auf 80°C erwärmt. Nach Abkühlen auf RT werden 10 ml Wasser zugegeben (Auflösen der Salze). Die organische Phase wird abgetrennt, mit Wasser gewaschen, über $Na_2SO_4$ getrocknet und am Vakuumrotationsverdampfer eingeengt. Die Kristallisation des Rückstandes (ca. 10 g) in 20 ml Acetonitril liefert 6,8 g (73 %) der Verbindung 204 als weisses Pulver. Der Schmelzpunkt beträgt 114-115°C.

<u>Beispiel 13:</u> Herstellung der Verbindung 204 (Verfahren B).

In einem 50 ml Rundkolben mit Magnetrührer und Kühler werden 12,18 g (0,05 Mol) 3-Octyloxypropionsäure (83 % GC), 4,7 ml (0,065 Mol) Thionylchlorid und 0,11 g (0,5 mmol) Benzyltriethylammoniumchlorid vorgelegt. Die farblose Lösung wird während 10 Minuten bei RT gerührt (HCl + $SO_2$ Entwicklung; endotherm: Ti 12°C). Nun wird unter leichtem $N_2$-Strom auf 65°C erwärmt und während 45 Minuten bei dieser Temperatur weitergerührt. Der Ueberschuss an Thionylchlorid wird abdestilliert (70-80°C/40 mbar; 30 Minuten). Zur erhaltenen farblosen Flüssigkeit (rohes Säurechlorid) werden 18 g (0,041 Mol) 2,2'-Ethylen-bis[4,6-di-tert-butylphenol] gegeben. Das Gemisch wird auf 140°C erwärmt (HCl Entwicklung) und während 30 Minuten bei dieser Temperatur gerührt. Anschliessend wird unter leichtem $N_2$-Strom während einer Stunde weiter gerührt. Die erhaltene braune Flüssigkeit wird auf ca. 100°C abgekühlt und 70 ml Ethanol werden zugegeben. Beim Abkühlen in einem Eisbad kristallisiert das Produkt aus. Nach Filtration und Trocknung am Hochvakuum werden 20 g (78 %) der Verbindung 204 als weisses Pulver erhalten. Der Schmelzpunkt beträgt 115°C.

<u>Beispiel 14:</u> Herstellung der Verbindung 215 (Verfahren B).

a) Herstellung von 3-(2-Butoxy-ethoxy)-propionsäure.

Zu einer Lösung von 3,8 g (32 mMol) 2-Butoxyethanol in 2 ml Tetrahydrofuran wird bei ca. 10°C unter $N_2$-Aunosphäre 2 ml (3,2 mMol, 10 Mol%) einer 1,6 N Butyllithiumlösung in Hexan getropft. Die Lösung wird während 5 Minuten bei 10°C gerührt. Anschliessend werden 2,5 ml (38 mMol) Acrylonitril zugegeben. Das Reaktionsgemisch wird auf 55°C erwärmt und während 2,5 Stunden bei dieser Temperatur gerührt. 0,25 ml (3,8 mMol) Acrylonitril werden nochmals zugegeben und das Gemisch eine weitere Stunde bei 55°C gerührt. Nach Abkühlen auf RT wird das Reaktionsgemisch auf Wasser gegossen, mit HCl 2N (2 ml) angesäuert und mit Toluol extrahiert. Die organische Phase wird mit einer wässrigen, gesättigten NaCl-Lösung gewaschen, über Magnesiumsulfat getrocknet und am Vakuumrotationsverdampfer eingedampft. Das erhaltene rohe 3-(2-Buthoxy-ethoxy)-propionitril (4,4 g) wird in 15 ml konzentrierte Salzsäure aufgenommen und die Lösung während 2,5 Stunden auf 80°C erwärmt. Anschliessend wird die erhaltene braune Lösung auf RT gekühlt, mit Wasser verdünnt und nochmals mit Essigester

extrahiert. Die organischen Phasen werden vereinigt, mit einer wässrigen gesättigten NaCl-Lösung gewaschen, über $MgSO_4$ getrocknet und am Vakuumrotationsverdampfer eingeengt. Nach Trocknung unter Hochvakuum werden 3,2 g (53 %) 3-(2-Butoxy-ethoxy)-propionsäure als hellbraune Flüssigkeit erhalten.

$^1$H-NMR (300 MHz; $CDCl_3$): $\delta$ (ppm) 0,91 (t, J = 7,3 Hz, 3 H); 1,36 (Sextet, J = 7,6 Hz, 2 H); 1,57 (Quintet, J = 7,8 Hz, 2 H); 2,65 (t, J = 6,3 Hz, 2 H); 3,47 (t, J = 6,6 Hz, 2 H); 3,56 - 3,65 (dxm, 4 H); 3,77 (t, J = 6,3 Hz, 2 H); 9,6 (s, breit, 1 H).

b) Zu einer Lösung von 6,6 g (15 mMol) 2,2'-Ethyliden-bis[4,6-di-tert-butylphenol], 3,76 g (18 mMol) 3-(2-Butoxy-ethoxy)-propionsäure und 0,1 g (0,8 mMol, 5 Mol%) Dimethylaminopyridin in 60 ml Ethylenchlorid wird bei RT und unter $N_2$-Atmosphäre eine Lösung von 3,1 g (15 mMol) Dicyclohexylcarbodiimid in 10 ml Ethylenchlorid getropft Das Reaktionsgemisch wird während 15 Stunden bei RT gerührt. Anschliessend wird der ausgefallene Harnstoff abfiltriert und das Lösungsmittel abgedampft. Die Kristallisation des Rückstandes aus Acetonitril liefert 5 g (54 %) der Verbindung 215 als weisses Pulver. Der Schmelzpunkt beträgt 88-91°C.

Beispiel 15: Herstellung der Verbindung 216 (Verfahren B).

a) In einem 250 ml Rundkolben werden unter Stickstoff-Atmosphäre 10,02 g (55 mMol) 3-[3-(2-Carboxy-ethoxy)-propoxy]-propansäure (siehe z.B. R.V. Christian und R.M. Hixon J.A.C.S. 70, 1333, (1948)) in 100 ml Toluol vorgelegt. Zu dieser Suspension werden 13,2 ml (180 mMol) Thionylchlorid gegeben, das Gemisch langsam (innerhalb von 40 Minuten) auf 90°C erwärmt (HCl + $SO_2$ Entwicklung) und während 1 Stunde bei dieser Temperatur gerührt. Anschliessend wird unter Einleiten eines Argon-Stromes weitere 45 Minuten bei 90°C nachgerührt. Destillation des Ueberschusses an Thionylchlorid sowie des Toluols liefert das rohe Bissäurechlorid welches direkt weiter eingesetzt wird.

b) In einem 350 ml Sulfierkolben werden unter Argon-Atmosphäre das in Beispiel 15a beschriebene Bissäurechlorid (55 mMol) und 43,87 g (0,1 Mol) 2,2'-Ethyliden-bis[4,6-di-tert-butylphenol] in 100 ml Toluol vorgelegt. Diese Lösung wird auf 10°C abgekühlt und 16,7 ml (120 mMol) Triethylamin langsam zugetropft. Nach Ende der Zugabe (ca. 50 Minuten) wird das Gemisch auf 70°C erwärmt und über Nacht bei dieser Temperatur weitergerührt.

Das ausgefallene Triethylaminhydrochorid wird abfiltriert und das Filtrat eingedampft. Chromatographie des Rückstandes an Kieselgel (Laufmittel: Hex/EE 40:1) liefert 21 g (40 %) der Verbindung 216 als amorphes Pulver. Der Schmelzpunkt beträgt 94°C.

Die Verbindungen 217 und 218 werden in Analogie zu der im Beispiel 15 beschriebenen Methode hergestellt.

Beispiel 16: Herstellung der Verbindung 219 (Verfahren B).

a) Herstellung von 3-(2,4-Di-tert-butylphenoxy)-propionsäure.

Zu einer Lösung von 9,7 g (47 mMol) 2,4-Di-tert-butylphenol und 0,6 ml (1,5 mMol) Benzyltrimethylammoniumhydroxid (40 %ige Lösung in Wasser) wird bei Raumtemperatur unter $N_2$-Atmosphäre innerhalb 20 Minuten 6,2 ml (94 mMol) Acrylonitril getropft. Nach Ende der Zugabe wird das Reaktionsgemisch auf 65°C erwärmt und während 2,5 Stunden bei dieser Temperatur gerührt. Anschliessend wird der Ueberschuss an Acrylonitril destillativ entfernt (Vakuumrotationsverdampfer). Das erhaltene rohe 3-(2,4-Di-tert-butylphenoxy)-propionitril wird in 40 ml konz. Salzsäure und 40 ml Essigsäure gelöst und die Lösung während 10 Stunden auf 100°C erwärmt. Das Gemisch wird abgekühlt, auf 60 ml Wasser gegossen und mit Hexan-Essigester 1:1 extrahiert. Die organische Phase wird abgetrennt, mit einer wässrigen gesättigten NaCl-Lösung gewaschen, über Natriumsulfat getrocknet und am Vakuumrotationsverdampfer eingeengt. Die Destillation des Rückstandes (60°C/0,1 mbar) liefert 10 g (77 %) der gewünschten Säure als weiss-beiges Pulver. Der Schmelzpunkt beträgt 105-106°C.

$^1$H-NMR (300 MHz) $CDCl_3$: $\delta$=2,89 (t, J = 6,2 Hz, 20 H; -$CH_2$-COOH-)' 4,27 (t, J = 6,2 Hz, 2 H, Ar-O-$CH_2$-).

b) In einem Rundkolben mit Kühler und Magnetrührer werden 10 g (36 mMol) 3-(2,4-Di-tert-butylphenoxy)-propionsäure, 13,1 g (30 mMol) 2,2'-Ethyliden-bis[4,6-di-tert-butylphenol], 0,2 g (1,6 mMol, 5 Mol%) Dimethylaminopyridin und 180 ml Dichlorethan vorgelegt. Zu dieser Lösung wird bei RT unter $N_2$-Aunosphäre eine Lösung von 6,2 g (30 mMol) Dicyclohexylcarbodiimid in 20 ml Dichlorethan getropft. Das Gemisch wird während 15 Stunden bei RT gerührt. Der ausgefallene Harnstoff wird abfiltriert und das Lösungsmittel abgedampft. Die Kristallisation des Rückstandes aus Hexan liefert 10 g (48 %) der Verbindung 219 als weisses Pulver. Der Schmelzpunkt beträgt

185°C.

Die Verbindung 220 wird aus der im Handel erhältlichen 3-Phenoxypropionsäure und 2,2'-Ethyliden-bis[4,6-di-tert-butylphenol] in Analogie zu dem im Beispiel 16b beschriebenen Verfahren hergestellt.

<u>Beispiel 17:</u> Herstellung der Verbindung 221 (Verfahren B).

a) Herstellung von 3-Cyclohexylidenaminoxypropionsäure.

Ein Gemisch von 32,8 g (0,29 Mol) Cyclohexanonoxim, 26 g (0,26 Mol) Ethylacrylat, 26 ml (0,052 Mol) einer 2 N KOH-Lösung in Ethanol und 150 ml Ethanol wird während 96 Stunden bei 60°C gerührt und anschliessend am Vakuumrotationsverdampfer eingeengt. Der Rückstand (oranges Oel) wird in 175 g (0,31 Mol) einer 10 %igen KOH Lösung in Ethanol gelöst und während 1 Stunde auf 60°C erwärmt. Das Lösungsmittel wird abdestilliert und der feste Rückstand in Wasser aufgenommen, mit ca. 15 ml konz. Salzsäure angesäuert und mit Essigester extrahiert. Die organische Phase wird mit Wasser gewaschen, über $Na_2SO_4$ getrocknet und am Vakuumrotationsverdampfer eingeengt. Die Destillation des Rückstandes liefert 11,8 g (25 %) der gewünschten Säure als orange Flüssigkeit. Der Siedepunkt beträgt 135°C/0,05 mbar.

| Elementaranalyse: | C % ber.: 58.36 | gef.: 58.47H % |
|---|---|---|
| | H % ber.: 8.16 | gef.: 8.30 |
| | N % ber.: 7.56 | gef.: 7.13 |

b) Verfährt man wie unter Beispiel 16b beschrieben, so erhält man aus 8,03 g (39 mMol) 3-Cyclohexylidenaminoxypropionsäure und 15,34 g (35 mMol) 2,2'-Ethyliden-bis[4,6-di-tert-butylphenol] 15,3 g (72 %) der Verbindung 221 als weisses Pulver. Der Schmelzpunkt beträgt 118-121°C (Methanol).

Die Herstellung der Verbindungen 222-224 erfolgt in Analogie zu der im Beispiel 13 (Verfahren B) beschriebenen Methode.

Die Herstellung der Verbindung 225 erfolgt in Analogie zu der im Beispiel 14b (Verfahren B) beschriebenen Methode.

<u>Beispiel 18:</u> Herstellung der Verbindung 301 (Oxidation des nach Verfahren A erhaltenen Thioethers).

a) Herstellung von 3-(Octylthio)-propansäure-2-tert-butyl-6-(3-tert-butyl-2-hydroxy-5-methylphenylmethyl)-4-methylphenylester.

In einem 200 ml Sulfierkolben werden 9,86 g (25 mMol) 2,2'-Methylen-bis[6-tert-butyl-4-methylphenol] monoacrylat, 4,02 g (27,5 mMol) Octanthiol in 50 ml Toluol gelöst. Bei RT werden 0,75 ml (1,25 mMol) einer 1,7 N Kalium-tert-pentylatlösung in Toluol zugetropft. Anschliessend wird das Reaktionsgemisch auf ca. 55°C erwärmt und während 1 Stunde bei dieser Temperatur gerührt. Das Gemisch wird auf RT abgekühlt, auf verdünnte Salzsäure (0,1 N) gegossen und mehrmals mit Essigester extrahiert. Die organischen Phasen werden vereinigt, über Natriumsulfat getrocknet und am Vakuumrotationsverdampfer eingeengt Nach Abtrennen des unreagierten Bisphenols (Kugelrohrdestillation 200°C/10 mbar), erhält man 11,35 g (84 %) der gewünschten Verbindung als hellgelbes Oel.

| Elementaranalyse: | | |
|---|---|---|
| C% ber.: 75.51<br>gef.: 75.31 | H % ber.: 9.69<br>gef.: 9.84 | S % ber.: 5.93<br>gef.: 6.05 |

b) In einem 250 ml Sulfierkolben werden unter Stickstoff-Atmosphäre 10,82 g (20 mMol) der Verbindung aus Beispiel 18a in 130 ml Dichlormethan gelöst. Zu dieser Lösung wird bei -15°C eine Lösung von 6,59 g (21 mMol) m-Chlorperbenzoesäure in 70 ml Dichlormethan getropft. Das Reaktionsgemisch wird während 30 Minuten bei -15°C gerührt, auf eine wässrige, gesättigte Natriumhydrogencarbonatlösung gegossen und mehrmals mit Dichlormethan extrahiert. Die organischen Phasen werden vereinigt, mit Wasser gewaschen, über Natriumsulfat getrocknet und im Vakuumrotationsverdampfer eingeengt. Nach Trocknung des Rückstandes im Hochvakuum werden 11,13 g (100 %) der Verbindung 301 als farbloses, hochviskoses Oel erhalten.

Beispiel 19: Herstellung der Verbindung 302 (Oxidation des durch Verfahren A erhaltenen Thioethers).

In einem 250 ml Sulfierkolben werden unter Stickstoff-Atmosphäre 10,82 g (20 mMol) der Verbindung aus Beispiel 18 in 80 ml Dichlormethan gelöst.

Zu dieser Lösung wird bei -15°C eine Lösung von 13,82 g (42 mMol) m-Chlorperbenzoesäure in 120 ml Dichlormethan getropft. Die dicke Suspension wird bis zum Aufwärmen auf RT weitergerührt und dann auf eine wässrige, gesättigte Natriumhydrogencarbonat-Lösung gegossen und mehrmals mit Dichlormethan extrahiert. Die organischen Phasen werden vereinigt, mit Wasser gewaschen, über Natriumsulfat getrocknet und am Vakuumrotationsverdampfer eingeengt. Die Kristallisation des Rückstandes aus Isopropanol liefert 8,05 g (70 %) der Verbindung 302 als hellbeiges Pulver. Der Schmelzpunkt beträgt 108-113°C.

Beispiel 20: Herstellung der Verbindung 401 (Verfahren A).

Ein Gemisch aus 12,3 g (0,025 Mol) 2,2'-Ethyliden-bis[4,6-di-tert-butylphenol]monoacrylat, 2,07 g (0,03 Mol) 1,2,4-Triazol und einigen Tropfen Pyridin wird während 3 Stunden bei 150°C gerührt. Anschliessend wird das Reaktionsgemisch auf ca. 100°C abgekühlt, mit ca. 30 ml Isopropanol versetzt und heiss filtriert. Beim Abkühlen auf Raumtemperatur kristallisiert die Verbindung 401 aus. Nach Absaugen und Trocknen werden 9,10 g (65 %) der Verbindung 401 als weisses Pulver erhalten. Der Schmelzpunkt bertägt 160-162°C.

Die Verbindung 402 wird in Analogie zu der im Beispiel 20 beschriebenen Methode hergestellt, wobei die Reaktion bei 170°C an Stelle von 150°C durchgeführt wird.

Beispiel 21: Herstellung der Verbindung 403 (Verfahren B).

Zu einer farblosen Lösung von 4,39 g (10 mMol) 2,2'-Ethyliden-bis[4,6-di-tert-butylphenol], 2,5 g (13 mMol) 3-Benzotriazol-1-yl-propionsäure (Wiley et al; J.A.C.S. 76, 4933 (1954)) und 2,02 g (20 mMol) Triethylamin in 40 ml Tetrahydrofuran wird bei 50°C unter $N_2$-Atmosphäre 0,68 ml (1,15 g; 7,5 mMol) Phosphorylchlorid getropft. Die erhaltene Suspension wird während 5 Stunden unter Rückfluss gerührt. Nach Abkühlen auf Raumtemperatur wird das Reaktionsgemisch von den Salzen abfiltriert und am Vakuumrotationsverdampfer eingeengt. Der Rückstand wird auf Wasser gegossen und mit Essigester zweimal extrahiert. Die organischen Phasen werden vereinigt, mit einer wässrigen, gesättigten NaCl Lösung gewaschen, über Natriumsulfat getrocknet und am Vakuumrotationsverdampfer eingeengt.

Die Kristallisation des Rückstandes aus Ethanol liefert 3,9 g (80 %) der Verbindung 403 als weisses Pulver. Der Schmelzpunkt beträgt 174-178°C.

Beispiel 22: Herstellung der Verbindung 404 (Verfahren A).

Ein Gemisch aus 14,78 g (0,03 Mol) 2,2'Ethyliden-bis[4,6-di-tert-butylphenol]monoacrylat, 5,02 g (0,03 Mol) 2-Mercapto-benzothiazol und einigen Tropfen Pyridin in 25 ml Dichlorethan wird während 15 Stunden unter Rückfluss gerührt. Das Reaktionsgemisch wird am Vakuumrotationsverdampfer eingedampft. Nach chromatographischer Reinigung ($SiO_2$; Hexan/Essigsäureethylester: 40:1) und Kristallisation aus Acetonitril werden 7,65 g (39 %) der Verbindung 404 als weisses Pulver erhalten. Der Schmelzpunkt beträgt 123-128°C.

Beispiel 22a: Herstellung der Verbindung 405.

Eine Lösung aus 14,78 g (0,03 Mol) 2,2'-Ethyliden-bis[4,6-di-tert-butylphenol]monoacrylat, 5,02 g 2-Mercaptobenzthiazol und einigen Tropfen Pyridin in 25 ml Dichlorethan wird während 15 Stunden bei 90°C gerührt. Anschliessend wird das Reaktionsgemisch am Vakuumrotationsverdampfer eingeengt. Nach chromatographischer Reinigung des Rückstandes ($SiO_2$; Hexan/Essigsäureethylester: 40:1) werden 7,7 g (39 %) der Verbindung 405 als amorphes Pulver erhalten. Der Schmelzpunkt beträgt 123-128°C nach Kristallisation in Acetonitril.

Beispiel 23: Stabilisierung von Polypropylen bei Mehrfachextrusion.

1,3 kg Polypropylenpulver (®Profax 6501), das mit 0,025 % 3-[3,5-di-tert-butyl-4-hydroxyphenyl]propionsäure-n-octadecylester vorstabilisiert wurde, (mit einem bei 230°C und mit 2,16 kg gemessenen Schmelzindex von 3,2) werden mit 0,05 % Pentaerythrityl-tetrakis-[3-(3,5-di-tert-butyl-4-hydroxyphenyl)-propionat]), 0,05 % Calciumstearat, 0,03 % Dihydrotalcit [$Mg_{4.5}Al_2(OH)_{13}CO_3 \cdot 3{,}5H_2O$] und 0,05 % einer Verbindung aus Tabelle 1 gemischt. Diese Mischung wird in einem Extruder mit einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260, 270, 280°C. Das Extrudat wird zur Kühlung durch ein Wasserbad gezogen und anschliessend granuliert. Dieses Granulat wird

wiederholt extrudiert. Nach 3 Extrusionen wird der Schmelzindex gemessen (bei 230°C mit 2,16 kg). Grosse Zunahme des Schmelzindex bedeutet starken Kettenabbau, also schlechte Stabilisierung. Die Resultate sind in Tabelle 2 zusammengefasst.

Tabelle 2:

| Verbindung aus Tabelle 1 | Schmelzindex nach 3 Extrusionen |
|---|---|
| - | 20,0 |
| 101 | 7,7 |
| 106 | 8,0 |
| 107 | 7,5 |
| 108 | 7,5 |
| 109 | 6,2 |
| 110 | 6,2 |
| 301 | 7,4 |

Beispiel 24: Stabilisierung von Elastomeren (Brabender-Test).

Ein Styrol-Butadien-Styrol Elastomer (®Finaprene 902) wird mit dem in Tabelle 3 angegebenen Stabilisator versetzt und in einem Brabender Plastographen bei 200°C mit 60 Umdrehungen/Minute geknetet. Der Knetwiderstand wird als Drehmoment kontinuierlich registriert. Infolge der Vernetzung des Polymeren erfolgt im Laufe der Knetzeit nach anfänglicher Konstanz eine rasche Zunahme des Drehmoments. Die Wirksamkeit der Stabilisatoren äussert sich in einer Verlängerung der Zeitspanne, in der das Drehmoment konstant bleibt. Die erhaltenen Werte sind in Tabelle 3 aufgeführt.

Tabelle 3:

| 0,25% Stabilisator aus Tabelle 1 | Zeit in Minuten bis zum Drehmomentanstieg |
|---|---|
| - | 24,2 |
| 113 | 63,3 |
| 114 | 60,0 |
| 115 | 53,5 |
| 116 | 56,6 |
| 118 | 68,4 |
| 119 | 54,0 |

Beispiel 25: Stabilisierung von Styrol-Butadienblock-Polymerisaten.

Bei thermisch oxidativer Schädigung von Styrol-Butadienblock-Polymeren erfolgt eine Vernetzung der Kautschukphase. Diese Vernetzung führt während der Verarbeitung in einem Extruder oder in einer Spritzgussmaschine zu einer Steigerung der Schmelzviskosität und daher des Extrusionsdrucks.

Die Prüfung der Verarbeitungsstabilität von Styrol-Butadienblock-Polymeren wird häufig in einem Kapillarrheometer durchgeführt. Dabei wird das Polymerisat, ähnlich wie bei der Extrusion, als Schmelze durch eine Düse gedrückt.

25 g Granulat von Styrol-Butadienblock-Polymerisat (®K-Resin KR-01 von ®Phillips Petroleum) enthaltend 0,2% n-Octadecyl-3-[3',5'-di-tert-butyl-4'-hydroxyphenyl]propionat und 0,6% Tris[nonylphenyl]phosphit werden in 250 ml Cyclohexan bei Raumtemperatur gelöst.

Die in Tabelle 4 angegebene Menge an Stabilisator wird in Toluol gelöst und der Polymerlösung beigemischt. Anschliessend wird das Cyclohexan bei 60°C und 0,013 bar abgedampft.

Das erhaltene Polymer wird bei 180°C zu 2 mm dicken Platten verpresst, aus denen kreisförmige Proben mit 8 mm Durchmesser gestanzt werden. Diese Proben werden in den Vorlagekanal eines Kapillarrheometers des Typs ®Keyeness Galaxy V eingefüllt und bei 250°C mit einer Schergeschwindigkeit von 14,594 $sec^{-1}$ vermessen. Nach einer Verlaufzeit von 6 Minuten wird die scheinbare Schergeschwindigkeit während 30 Minuten als Funktion der Zeit

registriert. Die Steigung dieser Kurve ($\Delta\eta/\Delta t$ in [Pa sec/min]) ist ein Mass für den Vernetzungsgrad des Polymers und steht daher in direktem Zusammenhang mit der Wirkung des Stabilisators. Je kleiner dieser Wert ist, desto wirksamer ist der Stabilisator.

Die Ergebnisse sind in Tabelle 4 aufgeführt.

Tabelle 4:

| 0,15% Stabilisator aus Tabelle 1 | $\frac{\Delta\eta\ (Pa\ sec)}{\Delta t\ (min)}$ |
|---|---|
| - | 23 |
| 113 | 2 |
| 114 | 8 |
| 116 | 5 |
| 117 | 5 |
| 214 bis | 6 |
| 215 | 2 |
| 216 | 6 |

**Patentansprüche**

**1.** Verbindung der Formel I,

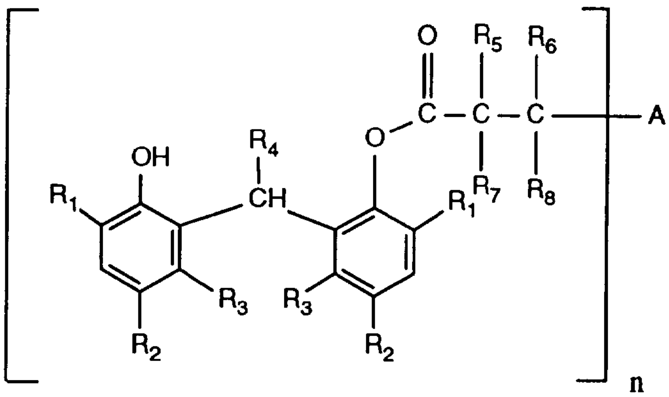

(I)

worin

die Reste $R_1$ unabhängig voneinander $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl oder $-CH_2$-S-$X_1$ bedeuten,

die Reste $R_2$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl, $-CH_2$-S-$X_1$, $-(CH_2)_p COO$-$X_2$ oder $-(CH_2)_q O$-$X_3$ sind,

die Reste $R_3$ unabhängig voneinander Wasserstoff oder $C_1$-$C_4$-Alkyl darstellen,

$R_4$ Wasserstoff oder $C_1$-$C_8$-Alkyl ist,

$R_5$ Wasserstoff, $C_1$-$C_{10}$-Alky, Phenyl, $-CH_2$-COO-$X_4$ oder CN bedeutet,

$R_6$ Wasserstoff, $C_1$-$C_4$-Alkyl, Phenyl, -COO-$X_5$, -CN oder -CON($X_6$)($X_7$) ist,

$R_7$ Wasserstoff oder $C_1$-$C_{10}$-Alkyl bedeutet,

$R_8$ Wasserstoff, $C_1$-$C_4$-Alkyl oder Phenyl ist,

$X_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$X_2$, $X_4$ und $X_5$ unabhängig voneinander $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl bedeuten,

$X_3$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$X_6$ und $X_7$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl oder $C_7$-$C_9$-Phenylalkyl sind,

$Y_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

$Y_2$ Wasserstoff oder $C_1$-$C_8$-Alkyl bedeutet,

p 0, 1 oder 2 ist,

q eine ganze Zahl von 0 bis 8 darstellt,

r 1 oder 2 bedeutet,

n eine ganze Zahl von 1 bis 4 ist und,

wenn n 1 ist, A eine Gruppe $-O-Z_1$, $-N(Z_2)(Z_3)$, $-NH(OZ_4)$, $-O-N=C(Z_5)(Z_6)$, $-S(O)_mZ_7$, $-NH-Z_8$ oder $-S-Z_8$ bedeutet, oder A ferner einen unsubstituierten oder durch $C_1$-$C_4$-Alkyl substituierten heterocyclischen Rest darstellt, der die freie Valenz an einem Stickstoffatom besitzt,

$Z_1$ Wasserstoff, $C_1$-$C_{25}$-Alkyl, durch Sauerstoff, Schwefel oder >N-$Y_2$ unterbrochenes $C_3$-$C_{25}$-Alkyl, $C_3$-$C_{24}$-Alkenyl, einen monocyclischen, gesättigten Kohlenwasserstoffrest mit 5 bis 20 Kohlenstoffatomen, einen bicyclischen, gesättigten Kohlenwasserstoffrest mit 7 bis 20 Kohlenstoffatomen, einen tricyclischen, gesättigten Kohlenwasserstoffrest mit 10 bis 20 Kohlenstoffatomen, $C_5$-$C_{12}$-Cycloalkenyl, durch $C_1$-$C_4$-Alkyl substituiertes $C_5$-$C_{12}$-Cycloalkenyl, $C_7$-$C_9$-Phenylalkyl, am Phenylring durch $C_1$-$C_4$-Alkyl substituiertes $C_7$-$C_9$-Phenylalkyl, Tetrahydrofurfuryl, Tetrahydroabietyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl, Furoyl oder eine Gruppe der Formel IIa oder IIb bedeutet,

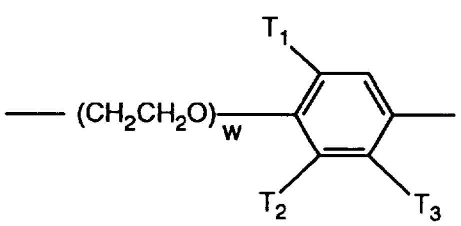
(IIa),

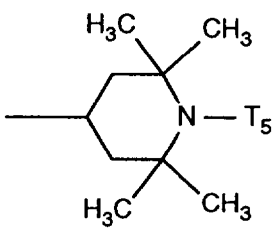
(IIb)

$Z_2$ Wasserstoff, $C_1$-$C_{25}$-Alkyl, durch OH substituiertes $C_2$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl, Furoyl, $-(CH_2)_pCOO-X_2$ oder einen Rest der Formel IIb bedeutet,

$Z_3$ Wasserstoff, $C_1$-$C_{25}$-Alkyl, durch OH substituiertes $C_2$-$C_{25}$-Alkyl, $C_3$-$C_{24}$-Alkenyl, $C_5$-$C_{12}$-Cycloalkyl, durch

$C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb ist oder

$Z_2$ und $Z_3$ zusammen $C_3$-$C_6$-Alkylen, $C_3$-$C_6$-Oxoalkylen oder durch Sauerstoff, Schwefel oder >N-$T_6$ unterbrochenes $C_3$-$C_6$-Alkylen bilden,

$Z_4$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

$Z_5$ und $Z_6$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl oder $C_7$-$C_9$-Phenylalkyl bedeuten oder

die Reste $Z_5$ und $Z_6$ zusammen mit dem Kohlenstoffatom, an das sie gebunden sind, einen unsubstituierten oder durch $C_1$-$C_4$-Alkyl substituierten $C_5$-$C_{12}$-Cycloalkylidenring bilden,

$Z_7$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$ und $T_2$ unabhängig voneinander Wasserstoff, $C_1$-$C_{25}$-Alkyl $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl oder $-CH_2-S-X_1$ bedeuten,

$T_3$ Wasserstoff oder $C_1$-$C_4$-Alkyl ist,

$T_4$ Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl, $-CH_2-S-X_1$, $-(CH_2)_pCOO-X_2$ oder $-(CH_2)_qO-X_3$ ist,

$T_5$ Wasserstoff, $C_1$-$C_8$-Alkyl, durch -OH substituiertes $C_2$-$C_4$-Alkyl, $O^{\cdot}$ , -OH, -NO, $-CH_2CN$, $C_1$-$C_{18}$-Alkyloxy, $C_5$-$C_{12}$-Cycloalkyloxy, $C_3$-$C_6$-Alkenyl, $C_7$-$C_9$-Phenylalkyl, am Phenylring durch $C_1$-$C_4$-Alkyl substituiertes $C_7$-$C_9$-Phenylalkyl, $C_1$-$C_8$-Alkanoyl, $C_3$-$C_8$-Alkenoyl oder Benzoyl bedeutet,

$T_6$ $C_1$-$C_{12}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

m 1 oder 2 bedeutet,

w 0 oder 1 bedeutet,

wenn n 2 ist, A eine Gruppe der Formel IIIa, IIIb, IIIc, IIId, IIIe oder IIIf ist,

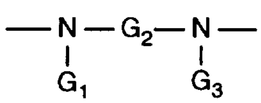 (IIIa), 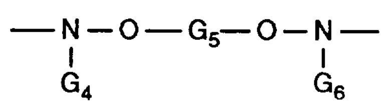 (IIIb),

$-O-G_7-O-$ (IIIc), 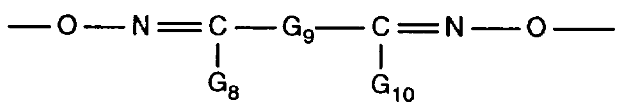 (IIId),

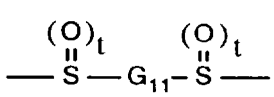 (IIIe), $>N-G_{12}$ (IIIf)

$G_1$ und $G_3$ unabhängig voneinander Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $-(CH_2)_pCOO-X_2$ oder einen Rest der Formel IIb bedeuten,

$G_2$ $C_2$-$C_{12}$-Alkylen, durch Sauerstoff, Schwefel oder >N-$Y_2$ unterbrochenes $C_4$-$C_{20}$-Alkylen, $C_4$-$C_{20}$-Alkenylen, $C_4$-$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen, Naphthylen, $C_2$-$C_{20}$-Alkandioyl, $C_4$-$C_{20}$-Alkendioyl oder Carboxybenzoyl bedeutet,

$G_4$ und $G_6$ unabhängig voneinander Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_5$ $C_2$-$C_{12}$-Alkylen, durch Sauerstoff, Schwefel oder >N-$Y_2$ unterbrochenes $C_4$-$C_{20}$-Alkylen, $C_4$-$C_{20}$-Alkenylen, $C_4$-$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen oder Naphthylen bedeutet,

$G_7$ $C_2$-$C_{20}$-Alkylen, durch Sauerstoff, Schwefel oder >N-$Y_2$ unterbrochenes $C_4$-$C_{20}$-Alkylen, $C_4$-$C_{20}$-Alkenylen, $C_4$-$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen, Naphthylen, $C_2$-$C_{20}$-Alkandioyl, $C_4$-$C_{20}$-Alkendioyl, Carboxybenzoyl oder eine Gruppe der Formel IVa, IVb oder IVc

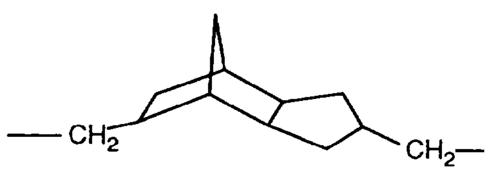

(IVa)

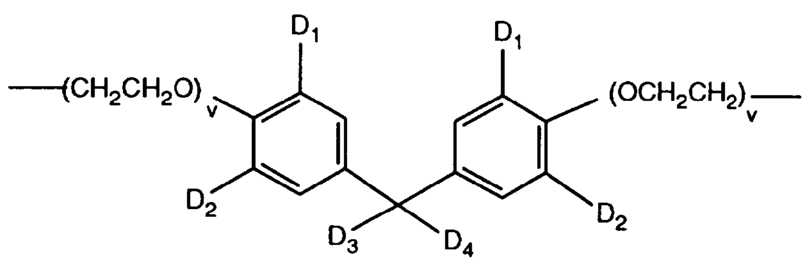

(IVb)

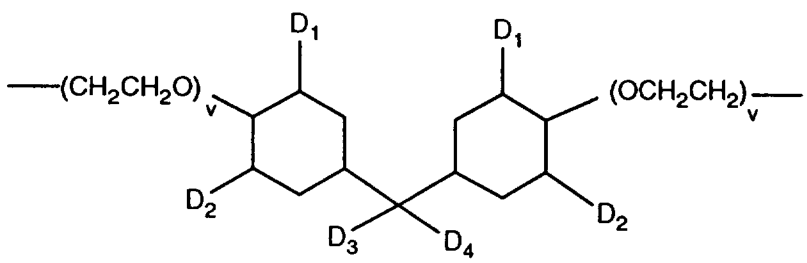

(IVc)

darstellt,

$G_8$ und $G_{10}$ unabhängig voneinander Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_9$ und $G_{11}$ $C_2$-$C_{12}$-Alkylen, durch Sauerstoff, Schwefel oder >N-$Y_2$ unterbrochenes $C_4$-$C_{20}$-Alkylen, $C_4$-$C_{20}$-Alkenylen, $C_4$-$C_{20}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, einen bicyclischen, gesättigten Kohlenwasserstoffrest mit zwei freien Valenzen und 7 bis 30 Kohlenstoffatomen, Phenylen, durch $C_1$-$C_4$-Alkyl substituiertes Phenylen oder Naphthylen ist,

$G_{12}$ Wasserstoff, $C_1$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb ist,

die Reste $D_1$ unabhängig voneinander Wasserstoff, $C_1$-$C_8$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl bedeuten,

die Reste $D_2$ unabhängig voneinander Wasserstoff, $C_1$-$C_8$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl sind,

$D_3$ und $D_4$ unabhängig voneinander Wasserstoff, $CF_3$, $C_1$-$C_{12}$-Alkyl oder Phenyl bedeuten oder

die Reste $D_3$ und $D_4$ zusammen mit dem Kohlenstoffatom, an das sie gebunden sind, einen unsubstituierten oder durch $C_1$-$C_4$-Alkyl substituierten $C_5$-$C_{12}$-Cycloalkylidenring bilden,

t 1 oder 2 bedeutet,

v 0 oder 1 bedeutet,

wenn n 3 ist, A eine Gruppe der Formel Va, Vb oder Vc bedeutet,

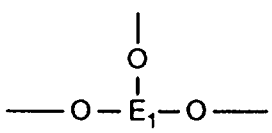 (Va), 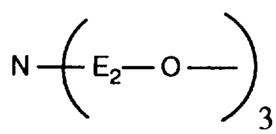 (Vb), 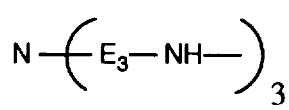 (Vc)

$E_1$ $C_3$-$C_7$-Alkantriyl darstellt,

$E_2$ und $E_3$ $C_2$-$C_8$-Alkylen sind,

wenn n 4 ist, A eine Gruppe der Formel VI bedeutet

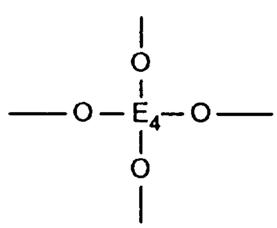 (VI)

und $E_4$ $C_4$-$C_{10}$-Alkantetrayl oder durch Sauerstoff unterbrochenes $C_4$-$C_{10}$-Alkantetrayl ist.

**2.** Verbindung gemäss Anspruch 1, worin

die Reste $R_1$ unabhängig voneinander $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl oder -$CH_2$-S-$X_1$ bedeuten,

die Reste $R_2$ unabhängig voneinander Wasserstoff, $C_1$-$C_{18}$-Alkyl, $C_2$-$C_{18}$-Alkenyl, $C_5$-$C_8$-Cycloalkyl, durch

$C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl, $-CH_2-S-X_1$, $-(CH_2)_pCOO-X_2$ oder $-(CH_2)_qO-X_3$ sind,

die Reste $R_3$ unabhängig voneinander Wasserstoff oder $C_1$-$C_4$-Alkyl darstellen,

$R_4$ Wasserstoff oder $C_1$-$C_8$-Alkyl ist,

$R_5$ Wasserstoff, $C_1$-$C_{10}$-Alkyl oder Phenyl bedeutet,

$R_6$ Wasserstoff, $C_1$-$C_4$-Alkyl oder Phenyl ist,

$R_7$ Wasserstoff oder $C_1$-$C_{10}$-Alkyl bedeutet,

$R_8$ Wasserstoff, $C_1$-$C_4$-Alkyl oder Phenyl ist,

$X_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$X_2$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl bedeutet,

$X_3$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $C_1$-$C_{18}$-Alkanoyl, $C_3$-$C_{18}$-Alkenoyl, durch Sauerstoff oder >N-$Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$Y_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist,

$Y_2$ Wasserstoff oder $C_1$-$C_8$-Alkyl bedeutet,

p 0, 1 oder 2 ist,

q eine ganze Zahl von 0 bis 8 darstellt,

r 1 oder 2 bedeutet,

n eine ganze Zahl von 1 bis 4 ist und,

wenn n 1 ist, A eine Gruppe $-O-Z_1$, $-N(Z_2)(Z_3)$, $-NH(OZ_4)$, $-O-N=C(Z_5)(Z_6)$, $-S(O)_mZ_7$, $-NH-Z_8$ oder $-S-Z_8$ bedeutet, oder A ferner ein unsubstituierter oder durch $C_1$-$C_4$-Alkyl substituierter heterocyclischer Rest ist, der die freie Valenz an einem Stickstoffatom besitzt,

$Z_1$ Wasserstoff, $C_1$-$C_{18}$-Alkyl, durch Sauerstoff oder >N-$Y_2$ unterbrochenes $C_3$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, $C_7$-$C_9$-Phenylalkyl, am Phenylring durch $C_1$-$C_4$-Alkyl substituiertes $C_7$-$C_9$-Phenylalkyl, Tetrahydrofurfuryl, Tetrahydroabietyl, $C_1$-$C_{18}$-Alkanoyl, $C_3$-$C_{18}$-Alkenoyl, durch Sauerstoff oder >N-$Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl, Furoyl oder eine Gruppe der Formel IIa oder IIb bedeutet,

$Z_2$ Wasserstoff, $C_1$-$C_{18}$-Alkyl, durch OH substituiertes $C_2$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $C_1$-$C_{18}$-Alkanoyl, $C_3$-$C_{18}$-Alkenoyl, durch Sauerstoff oder >N-$Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl, Furoyl, $-(CH_2)_pCOO-X_2$ oder einen Rest der Formel IIb bedeutet,

$Z_3$ Wasserstoff, $C_1$-$C_{18}$-Alkyl, durch OH substituiertes $C_2$-$C_{18}$-Alkyl, $C_3$-$C_{18}$-Alkenyl, $C_5$-$C_8$-Cycloalkyl, durch

$C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb ist oder

$Z_2$ und $Z_3$ zusammen $C_3$-$C_6$-Alkylen, $C_3$-$C_6$-Oxoalkylen oder durch Sauerstoff oder >N-$T_6$ unterbrochenes $C_3$-$C_6$-Alkylen bilden,

$Z_4$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist,

$Z_5$ und $Z_6$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl oder $C_7$-$C_9$-Phenylalkyl bedeuten oder

die Reste $Z_5$ und $Z_6$ zusammen mit dem Kohlenstoffatom, an das sie gebunden sind, einen unsubstituierten oder durch $C_1$-$C_4$-Alkyl substituierten $C_5$-$C_8$-Cycloalkylidenring bilden,

$Z_7$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$ und $T_2$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl oder $-CH_2-S-X_1$ bedeuten,

$T_3$ Wasserstoff oder $C_1$-$C_4$-Alkyl ist,

$T_4$ Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl, $-CH_2-S-X_1$, $-(CH_2)_pCOO-X_2$ oder $-(CH_2)_qO-X_3$ ist,

$T_5$ Wasserstoff, $C_1$-$C_4$-Alkyl, -OH, $C_6$-$C_{12}$-Alkyloxy, $C_5$-$C_8$-Cycloalkyloxy, Allyl, Benzyl oder Acetyl bedeutet,

$T_6$ $C_1$-$C_{12}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist,

m 1 oder 2 bedeutet,

w 0 oder 1 bedeutet,

wenn n 2 ist, A eine Gruppe der Formel IIIa, IIIb, IIIc, IIId, IIIe oder IIIf ist,

$G_1$ und $G_3$ unabhängig voneinander Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $-(CH_2)_pCOO-X_2$ oder einen Rest der Formel IIb bedeuten,

$G_2$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen, Naphthylen, $C_2$-$C_{18}$-Alkandioyl, $C_4$-$C_{18}$-Alkendioyl oder Carboxybenzoyl bedeutet,

$G_4$ und $G_6$ unabhängig voneinander Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_5$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen oder Naphthylen bedeutet,

$G_7$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen, Naphthylen, $C_2$-$C_{18}$-Alkandioyl, $C_4$-$C_{18}$-Alkendioyl oder Carboxybenzoyl ist,

$G_8$ und $G_{10}$ unabhängig voneinander Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb sind,

$G_9$ und $G_{11}$ $C_2$-$C_{12}$-Alkylen, durch Sauerstoff oder >N-$Y_2$ unterbrochenes $C_4$-$C_{12}$-Alkylen, $C_4$-$C_{12}$-Alkenylen, $C_4$-$C_{12}$-Alkinylen,
($C_1$-$C_4$-Alkylen)-phenylen-($C_1$-$C_4$-alkylen), einen monocyclischen, gesättigten Kohlenwasserstoffrest mit zwei freien Valenzen und 5 bis 12 Kohlenstoffatomen, Phenylen, durch $C_1$-$C_4$-Alkyl substituiertes Phenylen oder Naphthylen ist,

$G_{12}$ Wasserstoff, $C_1$-$C_{18}$-Alkyl, $C_3$-$C_{18}$-Alkenyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder eine Gruppe der Formel IIb ist und

t 1 oder 2 bedeutet.

**3.** Verbindung gemäss Anspruch 1, worin

die Reste $R_1$ unabhängig voneinander $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl oder -$CH_2$-S-$X_1$ bedeuten,

die Reste $R_2$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl, -$CH_2$-S-$X_1$, -$(CH_2)_p$COO-$X_2$ oder -$(CH_2)_q$O-$X_3$ sind,

die Reste $R_3$ Wasserstoff darstellen,

$R_4$ Wasserstoff oder $C_1$-$C_4$-Alkyl ist,

$R_5$ Wasserstoff, $C_1$-$C_4$-Alkyl oder Phenyl bedeutet,

$R_6$ Wasserstoff, $C_1$-$C_4$-Alkyl oder Phenyl ist,

$R_7$ Wasserstoff oder $C_1$-$C_4$-Alkyl bedeutet,

$R_8$ Wasserstoff, $C_1$-$C_4$-Alkyl oder Phenyl ist,

$X_1$ $C_1$-$C_{10}$-Alkyl, $C_5$-$C_8$-Cycloalkyl, Phenyl, $C_7$-$C_9$-Phenylalkyl oder -$(CH_2)_r$COO-$Y_1$ darstellt,

$X_2$ $C_1$-$C_{10}$-Alkyl, $C_5$-$C_8$-Cycloalkyl, Phenyl oder $C_7$-$C_9$-Phenylalkyl bedeutet,

$X_3$ $C_1$-$C_{10}$-Alkyl, $C_5$-$C_8$-Cycloalkyl, Phenyl, $C_7$-$C_9$-Phenylalkyl, $C_1$-$C_{10}$-Alkanoyl, $C_3$-$C_{18}$-Alkenoyl, durch Sauerstoff unterbrochenes $C_3$-$C_{18}$-Alkanoyl oder Benzoyl ist,

$Y_1$ $C_1$-$C_{10}$-Alkyl, $C_5$-$C_8$-Cycloalkyl, Phenyl oder $C_7$-$C_9$-Phenylalkyl ist,

p 0, 1 oder 2 ist,

q eine ganze Zahl von 0 bis 8 darstellt,

r 1 oder 2 bedeutet,

n eine ganze Zahl von 1 bis 4 ist und,

wenn n 1 ist, A eine Gruppe $-O-Z_1$, $-N(Z_2)(Z_3)$, $-NH(OZ_4)$, $-O-N=C(Z_5)(Z_6)$, $-S(O)_mZ_7$, $-NH-Z_8$ oder $-S-Z_8$ bedeutet, oder A ferner ein unsubstituierter oder durch $C_1$-$C_4$-Alkyl substituierter heterocyclischer Rest ist, der die freie Valenz an einem Stickstoffatom besitzt,

$Z_1$ Wasserstoff, $C_1$-$C_{18}$-Alkyl, durch Sauerstoff unterbrochenes $C_3$-$C_{18}$-Alkyl, $C_3$-$C_{18}$-Alkenyl, $C_5$-$C_8$-Cycloalkyl, $C_5$-$C_8$-Cycloalkenyl, $C_7$-$C_9$-Phenylalkyl, Tetrahydrofurfuryl, $C_1$-$C_{10}$-Alkanoyl, $C_3$-$C_{18}$-Alkenoyl, durch Sauerstoff unterbrochenes $C_3$-$C_{18}$-Alkanoyl, Benzoyl oder eine Gruppe der Formel IIa oder IIb bedeutet,

$Z_2$ Wasserstoff, $C_1$-$C_{18}$-Alkyl, durch OH substituiertes $C_2$-$C_{10}$-Alkyl, $C_3$-$C_{18}$-Alkenyl, $C_5$-$C_8$-Cycloalkyl, Phenyl, $C_7$-$C_9$-Phenylalkyl, $C_1$-$C_{10}$-Alkanoyl, $C_3$-$C_{18}$-Alkenoyl, durch Sauerstoff unterbrochenes $C_3$-$C_{18}$-Alkanoyl, Benzoyl, $-(CH_2)_pCOO-X_2$ oder einen Rest der Formel IIb bedeutet,

$Z_3$ Wasserstoff, $C_1$-$C_{18}$-Alkyl, durch OH substituiertes $C_2$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb ist oder

$Z_2$ und $Z_3$ zusammen $C_3$-$C_6$-Alkylen, $C_3$-$C_6$-Oxoalkylen oder durch Sauerstoff unterbrochenes $C_3$-$C_6$-Alkylen bilden,

$Z_4$ $C_1$-$C_{10}$-Alkyl, $C_5$-$C_8$-Cycloalkyl, Phenyl oder $C_7$-$C_9$-Phenylalkyl ist,

$Z_5$ und $Z_6$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl oder $C_7$-$C_9$-Phenylalkyl bedeuten oder die Reste $Z_5$ und $Z_6$ zusammen mit dem Kohlenstoffatom, an das sie gebunden sind, einen $C_5$-$C_8$-Cycloalkylidenring bilden,

$Z_7$ $C_1$-$C_{10}$-Alkyl, $C_5$-$C_8$-Cycloalkyl, Phenyl, $C_7$-$C_9$-Phenylalkyl oder $-(CH_2)_rCOO-Y_1$ darstellt,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$ und $T_2$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl oder $-CH_2-S-X_1$ bedeuten,

$T_3$ Wasserstoff oder $C_1$-$C_4$-Alkyl ist,

$T_4$ Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl, $-CH_2-S-X_1$, $-(CH_2)_pCOO-X_2$ oder $-(CH_2)_qO-X_3$ ist,

$T_5$ Wasserstoff, $C_1$-$C_4$-Alkyl, -OH, $C_6$-$C_{12}$-Alkyloxy, $C_5$-$C_8$-Cycloalkyloxy, Allyl, Benzyl oder Acetyl bedeutet,

m 1 oder 2 bedeutet,

w 0 oder 1 bedeutet,

wenn n 2 ist, A eine Gruppe der Formel IIIa, IIIb, IIIc, IIId, IIIe oder IIIf ist,

$G_1$ und $G_3$ unabhängig voneinander Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl, $-(CH_2)_pCOO-X_2$ oder einen Rest der Formel IIb bedeuten,

$G_2$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen, Phenylen, $C_2$-$C_{10}$-Alkandioyl, $C_4$-$C_{10}$-Alkendioyl oder Carboxybenzoyl bedeutet,

$G_4$ und $G_6$ unabhängig voneinander Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb sind,

$G_5$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen oder Phenylen bedeutet,

$G_7$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen, Phenylen, $C_2$-$C_{10}$-Alkandioyl, $C_4$-$C_{10}$-Alkendioyl oder Carboxybenzoyl ist,

$G_8$ und $G_{10}$ unabhängig voneinander Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb sind,

$G_9$ und $G_{11}$ $C_2$-$C_{10}$-Alkylen, durch Sauerstoff unterbrochenes $C_4$-$C_{12}$-Alkylen, $C_4$-$C_{10}$-Alkenylen, $C_4$-$C_{10}$-Alkinylen, ($C_1$-$C_4$-Alkylen)-phenylen-($C_1$-$C_4$-alkylen), einen monocyclischen, gesättigten Kohlenwasserstoffrest mit zwei freien Valenzen und 5 bis 10 Kohlenstoffatomen oder Phenylen ist,

$G_{12}$ Wasserstoff, $C_1$-$C_{10}$-Alkyl, $C_3$-$C_{18}$-Alkenyl, $C_5$-$C_8$-Cycloalkyl, Phenyl, $C_7$-$C_9$-Phenylalkyl oder eine Gruppe der Formel IIb ist und

t 1 oder 2 bedeutet.

**4.** Verbindung gemäss Anspruch 1, worin die Reste $R_5$, $R_6$, $R_7$ und $R_8$ Wasserstoff bedeuten.

**5.** Verbindung gemäss Anspruch 1, worin n 1 oder 2 ist

**6.** Verbindung gemäss Anspruch 1, worin

n 1 ist,

A eine Gruppe der Formel -N($Z_2$)($Z_3$) bedeutet,

$Z_2$ Wasserstoff ist und

$Z_3$ Wasserstoff, $C_1$-$C_{25}$-Alkyl, durch OH substituiertes $C_2$-$C_{25}$-Alkyl, $C_3$-$C_{24}$-Alkenyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder eine Gruppe der Formel IIb ist.

**7.** Verbindung gemäss Anspruch 1, worin

n 1 ist,

A eine Gruppe der Formel -O-$Z_1$ darstellt und

$Z_1$ durch Sauerstoff unterbrochenes $C_1$-$C_{25}$-Alkyl oder eine Gruppe der Formel IIa bedeutet und

w 1 ist.

**8.** Verbindung gemäss Anspruch 1, worin

die Reste $R_1$ gleich sind und $C_1$-$C_5$-Alkyl oder $C_5$-$C_8$-Cycloalkyl bedeuten,

die Reste $R_2$ gleich sind und $C_1$-$C_5$-Alkyl darstellen,

die Reste $R_3$ Wasserstoff bedeuten und

$R_4$ Wasserstoff oder $C_1$-$C_4$-Alkyl ist.

**9.** Verbindung gemäss Anspruch 1, worin

die Reste $R_1$ gleich sind und $C_1$-$C_5$-Alkyl oder $C_5$-$C_8$-Cycloalkyl bedeuten,

die Reste $R_2$ gleich sind und $C_1$-$C_5$-Alkyl darstellen,

die Reste $R_3$ Wasserstoff bedeuten,

$R_4$ Wasserstoff oder $C_1$-$C_4$-Alkyl ist,

$R_5$, $R_6$, $R_7$ und $R_8$ Wasserstoff sind,

n 1 oder 2 ist und,

wenn n 1 ist, A eine Gruppe -O-$Z_1$, -N($Z_2$)($Z_3$), -NH(O$Z_4$), -O-N=C($Z_5$)($Z_6$), -S(O)$_m$$Z_7$, -NH-$Z_8$ oder -S-$Z_8$ bedeutet, oder A ferner ein unsubstituierter oder durch $C_1$-$C_4$-Alkyl substituierter heterocyclischer Rest ist, der die freie Valenz an einem Stickstoffatom besitzt,

$Z_1$ $C_1$-$C_{18}$-Alkyl, durch Sauerstoff unterbrochenes $C_3$-$C_{10}$-Alkyl, $C_5$-$C_8$-Cycloalkyl, Tetrahydrofurfuryl oder eine Gruppe der Formel IIa oder II b ist,

$Z_2$ $C_1$-$C_{18}$-Alkyl, durch -OH substituiertes $C_2$-$C_4$-Alkyl, $C_7$-$C_9$-Phenylalkyl, -$(CH_2)_p$COO-$X_2$ oder einen Rest der Formel IIb darstellt,

$Z_3$ Wasserstoff, $C_1$-$C_{18}$-Alkyl, durch -OH substituiertes $C_2$-$C_4$-Alkyl, $C_7$-$C_9$-Phenylalkyl oder einen Rest der Formel IIb bedeutet oder

$Z_2$ und $Z_3$ zusammen $C_3$-$C_6$-Oxoalkylen oder durch Sauerstoff unterbrochenes $C_3$-$C_6$-Alkylen bilden,

$Z_4$ $C_7$-$C_9$-Phenylalkyl bedeutet,

die Reste $Z_5$ und $Z_6$ zusammen mit dem Kohlenstoffatom, an das sie gebunden sind, einen $C_5$-$C_8$-Cycloalkylidenring bilden,

$Z_7$ $C_1C_{10}$-Alkyl bedeutet,

$Z_8$ unsubstituiertes oder durch $C_1$-$C_4$-Alkyl substituiertes 2-Benzoxazolyl oder unsubstituiertes oder durch $C_1$-$C_4$-Alkyl substituiertes 2-Benzothiazolyl ist,

$T_1$, $T_2$, $T_3$ und $T_4$ unabhängig voneinander Wasserstoff oder $C_1$-$C_4$-Alkyl sind,

$T_5$ Wasserstoff oder $C_1$-$C_4$-Alkyl ist,

$X_2$ $C_1$-$C_{10}$-Alkyl darstellt,

m 1 oder 2 bdeutet,

p 1 ist,

w 0 oder 1 darstellt und

wenn n 2 ist, A eine Gruppe der Formel IIIa, IIIc oder IIIf bedeutet,

$G_1$ und $G_3$ unabhängig voneinander Wasserstoff, $C_7$-$C_9$-Phenylalkyl oder einen Rest der Formel IIb bedeuten,

$G_2$ $C_2$-$C_8$-Alkylen oder einen monocyclischen, gesättigten Kohlenwasserstoffrest mit zwei freien Valenzen und 10 Kohlenstoffatomen ist,

$G_7$ $C_2$-$C_8$-Alkylen oder durch Sauerstoff unterbrochenes $C_4$-$C_{12}$-Alkylen darstellt und

$G_{12}$ $C_7$-$C_9$-Phenylalkyl oder eine Gruppe der Formel IIb ist.

**10.** Zusammensetzung enthaltend ein gegen oxidativen, thermischen oder lichtinduzierten Abbau empfindliches organisches Material und mindestens eine Verbindung der Formel I gemäss Anspruch 1.

**11.** Zusammensetzung gemäss Anspruch 10, worin das organische Material ein synthetisches Polymer ist.

**12.** Zusammensetzung gemäss Anspruch 10, worin das organische Material ein Polyolefin ist

**13.** Zusammensetzung gemäss Anspruch 10, worin das organische Material ein in Lösung polymerisierter Polybutadien-Kautschuk ist.

**14.** Zusammensetzung gemäss Anspruch 10, worin das organische Material ein in Lösung polymerisiertes Styrol-Butadien-Copolymer oder Styrol-Butadien-Blockcopolymer ist.

**15.** Zusammensetzung gemäss Anspruch 10, worin das organische Material Acrylnitril-Butadien-Styrol ist.

**16.** Verwendung einer Verbindung der Formel I gemäss Anspruch 1 zum Stabilisieren eines organischen Materials gegen oxidativen, thermischen oder lichtinduzierten Abbau.